(19) **Europäisches Patentamt / European Patent Office / Office européen des brevets**

(11) **EP 4 786 498 A1**

(12) # EUROPEAN PATENT APPLICATION
published in accordance with Art. 153(4) EPC

(43) Date of publication:
05.08.2026 Bulletin 2026/32

(21) Application number: 24870870.3

(22) Date of filing: 26.09.2024

(51) International Patent Classification (IPC):
C07K 16/46 (2006.01)     C07K 16/28 (2006.01)
C12N 15/13 (2006.01)     C07K 19/00 (2006.01)
A61K 39/395 (2006.01)     A61P 35/00 (2006.01)

(52) Cooperative Patent Classification (CPC):
A61K 39/395; A61P 35/00; C07K 16/00;
C07K 16/28; C07K 16/46; C07K 19/00

(86) International application number:
PCT/CN2024/121415

(87) International publication number:
WO 2025/067330 (03.04.2025 Gazette 2025/14)

(84) Designated Contracting States:
AL AT BE BG CH CY CZ DE DK EE ES FI FR GB
GR HR HU IE IS IT LI LT LU LV MC ME MK MT NL
NO PL PT RO RS SE SI SK SM TR
Designated Extension States:
BA
Designated Validation States:
GE KH MA MD TN

(30) Priority: 27.09.2023 PCT/CN2023/122262
20.05.2024 PCT/CN2024/094223

(71) Applicant: Nanjing Leads Biolabs Co., Ltd.
Jiangbei New Area
Nanjing
Jiangsu 210031 (CN)

(72) Inventors:
• CAI, Shengli
Nanjing, Jiangsu 210019 (CN)
• KANG, Xiaoqiang
Nanjing, Jiangsu 210019 (CN)

(74) Representative: Bianchetti & Minoja with
Trevisan & Cuonzo IPS SRL
Via Plinio, 63
20129 Milano (IT)

Remarks:
The complete document including Reference
Table(s) and the Sequence Listing(s) can be
downloaded from the EPO website

(54) **ANTI-PD-L1 AND ANTI-4-1BB BISPECIFIC ANTIBODY FOR TREATING TUMORS**

(57)     Provided is an anti-4-1BB and anti-PD-L1 bispecific antibody and use of same in combination with a chemotherapeutic agent in the preparation of a drug for treating tumors.

FIG. 1

EP 4 786 498 A1

**Description**

**TECHNICAL FIELD**

The present disclosure relates to use of an anti-4-1BB and anti-PD-L1 bispecific antibody in the treatment of a tumor, and specifically further relates to use of an anti-PD-L1 and anti-4-1BB antibody and a combination thereof with a chemotherapeutic agent in the preparation of a drug for treating a tumor.

**BACKGROUND**

Over the past decade, tumor immunotherapy has significantly advanced and has become an effective therapy widely used in clinical practice following surgery, radiotherapy, chemotherapy, and targeted therapy. Tumor immunotherapy can trigger a tumor immune response in a subject. By blocking immunosuppressive pathways activated by cancer cells, it activates the subject's own immune system to kill tumors. This therapy has the advantages of strong specificity, long-lasting effects, small side effects, etc.

Programmed cell death protein 1 (PD-1) is an immunosuppressive receptor mainly expressed on the surface of T cells, B cells, monocytes, and natural killer cells. Its related ligands are programmed death ligand 1 (PD-L1) and programmed death ligand 2 (PD-L2). PD-L1 is widely expressed on various tumor cells and immune cells. Within the tumor microenvironment, its expression level can be upregulated by a variety of cytokines such as interferon (IFN)-$\gamma$. The binding of PD-L1 on the surface of tumor cells and antigen-presenting cells to PD-1 leads to sustained activation of the PD-1/PD-L1 pathway, which can inhibit the activation of tumor antigen-specific T cells, weakening the anti-tumor activity of T cells. PD-1/PD-L1 antibodies interfere with the binding of PD-1 to PD-L1 to remove the immunosuppressive effect of the pathway and restore anti-tumor immune responses of T cells. Immune checkpoint inhibitors are currently a hot topic in the field of tumor immunotherapy. Several anti-PD-(L)1 antibodies have been approved in China and other countries. PD-(L)1 inhibitors show response rates of 40-70% in tumors such as melanoma, Merkel cell carcinoma, Hodgkin lymphoma, and microsatellite instability-high (MSI-H) tumors. However, their response rates are only 10-25% in tumors for which many immune checkpoint inhibitors are approved, such as advanced small cell lung cancer and gastric cancer (according to the instructions of products on the market). In addition, existing immune checkpoint inhibitors may have problems such as primary and acquired drug resistance or may trigger immune-related adverse events. Therefore, it is necessary to find other immune checkpoints within the tumor microenvironment to reduce the development of drug resistance and improve anti-tumor efficacy.

4-1BB (CD137) is one of the potential targets in tumor immunotherapy. 4-1BB belongs to the tumor necrosis factor (TNF) receptor family and is mainly expressed in activated T cells, natural killer cells, regulatory T cells, dendritic cells, mast cells, etc. Its ligand 4-1BBL is mainly expressed in activated antigen-presenting cells (APCs), including dendritic cells, macrophages, and B cells. 4-1BB is a T cell-specific co-stimulatory molecule that plays a key role in fully activating T cells and other immune cells in immunotherapy. The main physiological function of 4-1BB on T cells is as follows: Upon binding to 4-1BBL, 4-1BB recruits TNF receptor-associated factors 1 and 2 (TRAF1 and TRAF2) to form a heterotrimer, thereby activating downstream signaling pathways dominated by nuclear factor $\kappa$-light-chain-enhancer of activated B cells (NF-$\kappa$B), c-Jun N-terminal kinase (JNK), and p38 mitogen-activated protein kinase (MAPK). These activated signaling pathways produce co-stimulatory signals, promote the production and secretion of cytokines (e.g., interleukin [IL]-2, IL-4, and IFN-$\gamma$), and promote the activation of CD8+ and CD4+ T lymphocytes. In particular, NF-$\kappa$B activation improves the survival rate of CD8+ T lymphocytes by increasing the expression of the anti-apoptotic genes bcl-XL and bfl-l. In addition to activating the immune system through T cells, 4-1BB can also activate or regulate the immune system through natural killer cells, macrophages, and B cells to achieve the purpose of inhibiting tumor proliferation.

In addition, combination therapies of anti-4-1BB antibodies and other antibodies (e.g., anti-PD-1 antibodies) are currently undergoing clinical trials. For example, there is a need for bispecific antibodies that can simultaneously target PD-L1 and 4-1BB to block the PD-1/PD-L1 pathway and provide co-stimulatory signals through 4-1BB upon PD-L1 cross-linking on PD-L1-expressing tumor cells. However, the bispecific antibodies described in patent US_2017_0198050_A1 can activate or induce 4-1BB signaling without cross-linking with target cells, which has raised concerns about the potential toxicity induced by 4-1BB at high concentrations. Several PD-L1/4-1BB-targeting bispecific antibodies have been in the clinical research and development stage in China and other countries. GEN1046 (jointly developed by BioNTech and Genmab), FS222 (developed by F-star Therapeutics, UK), and PM1003 (developed by Biotheus Biotechnology (Zhuhai) Co., Ltd.) are currently in phase I/II clinical trials. To date, no bispecific antibodies targeting the PD-L1/4-1BB signaling pathway have been approved in China and other countries.

**SUMMARY**

The applicant has developed a bispecific antibody specifically binding to 4-1BB and PD-L1 on the basis of its 4-1BB antibody platform "X-Body". The present disclosure provides use of the bispecific antibody in the treatment of a tumor.

Thus, in a first aspect, the present disclosure relates to use of a bispecific antibody in the preparation of a drug for treating a malignant tumor, wherein the bispecific antibody comprises a first antigen-binding region specifically binding to 4-1BB and a second antigen-binding region specifically binding to PD-L1.

In one embodiment, in the use to which the present disclosure relates, the first antigen-binding region comprises a first heavy chain variable region and/or a first light chain variable region, wherein:

the first heavy chain variable region:

(i) comprises an HCDR1, an HCDR2, and an HCDR3 derived from a heavy chain variable region consisting of the sequence set forth in SEQ ID NO: 7 or SEQ ID NO: 9;

(ii) comprises an HCDR1, an HCDR2, and an HCDR3 comprising or consisting of the amino acid sequences of SEQ ID NO: 1, SEQ ID NO: 2, and SEQ ID NO: 3, respectively, or comprising or consisting of amino acid sequences having one, two, or three modifications (preferably amino acid substitutions, and preferably conservative substitutions) compared to the amino acid sequences of SEQ ID NO: 1, SEQ ID NO: 2, and SEQ ID NO: 3, respectively; or

(iii) comprises or consists of the amino acid sequence of SEQ ID NO: 7 or SEQ ID NO: 9, or comprises or consists of an amino acid sequence having at least 80%, 81%, 82%, 83%, 84%, 85%, 86%, 87%, 88%, 89%, 90%, 91%, 92%, 93%, 94%, 95%, 96%, 97%, 98%, or 99% identity to the amino acid sequence of SEQ ID NO: 7 or SEQ ID NO: 9, or comprises or consists of an amino acid sequence having one or more (preferably no more than 10, and more preferably no more than 5) amino acid modifications (preferably amino acid substitutions, and more preferably conservative substitutions) compared to the amino acid sequence of SEQ ID NO: 7 or SEQ ID NO: 9, wherein preferably, the amino acid modifications do not occur in a CDR; more preferably, the amino acid modifications occur in an FR, for example, in an FR1, an FR2, an FR3, or an FR4;

the first light chain variable region:

(i) comprises an LCDR1, an LCDR2, and an LCDR3 derived from a light chain variable region consisting of the sequence set forth in SEQ ID NO: 8 or SEQ ID NO: 10;

(ii) comprises an LCDR1, an LCDR2, and an LCDR3 comprising or consisting of the sequences set forth in SEQ ID NO: 4, SEQ ID NO: 5, and SEQ ID NO: 6, respectively, or comprising or consisting of amino acid sequences having one, two, or three modifications (preferably amino acid substitutions, and preferably conservative substitutions) compared to the amino acid sequences of SEQ ID NO: 4, SEQ ID NO: 5, and SEQ ID NO: 6, respectively; or

(iii) comprises or consists of the amino acid sequence of SEQ ID NO: 8 or SEQ ID NO: 10, or comprises or consists of an amino acid sequence having at least 80%, 81%, 82%, 83%, 84%, 85%, 86%, 87%, 88%, 89%, 90%, 91%, 92%, 93%, 94%, 95%, 96%, 97%, 98%, or 99% identity to the amino acid sequence of SEQ ID NO: 8 or SEQ ID NO: 10, or comprises or consists of an amino acid sequence having one or more (preferably no more than 10, and more preferably no more than 5) amino acid modifications (preferably amino acid substitutions, and more preferably conservative substitutions) compared to the amino acid sequence of SEQ ID NO: 8 or SEQ ID NO: 10, wherein preferably, the amino acid modifications do not occur in a CDR; more preferably, the amino acid modifications occur in an FR, for example, in an FR1, an FR2, an FR3, or an FR4;

in SEQ ID NO: 8, X = S or G.

In one embodiment, in the use to which the present disclosure relates, the first antigen-binding region further comprises a first heavy chain constant region and/or a first light chain constant region, wherein the first heavy chain constant region comprises or consists of the amino acid sequence set forth in SEQ ID NO: 19 or 20, and the first light chain constant region comprises or consists of the amino acid sequence set forth in SEQ ID NO: 21 or 22.

In one embodiment, in the use to which the present disclosure relates, the first antigen-binding region activates 4-1BB signaling and is from an anti-4-1BB monoclonal antibody. In some embodiments, the first antigen-binding region is an scFv of an anti-4-1BB antibody.

In one embodiment, in the use to which the present disclosure relates, the second antigen-binding region specifically binding to PD-L1 comprises a second heavy chain variable region and a second light chain variable region, wherein the second heavy chain variable region comprises an HCDR1, an HCDR2, and an HCDR3 consisting of the sequences set forth in SEQ ID NO: 11, SEQ ID NO: 12, and SEQ ID NO: 13, respectively, and the second light chain variable region comprises an LCDR1, an LCDR2, and an LCDR3 consisting of the sequences set forth in SEQ ID NO: 14, SEQ ID NO: 15, and SEQ ID NO: 16, respectively.

In one embodiment, in the use to which the present disclosure relates, the second antigen-binding region further comprises a second heavy chain constant region and/or a second light chain constant region, wherein the second heavy chain constant region comprises or consists of the amino acid sequence set forth in SEQ ID NO: 19 or 20, and the second light chain constant region comprises or consists of the amino acid sequence set forth in SEQ ID NO: 21 or 22.

In one embodiment, in the use to which the present disclosure relates, the bispecific antibody comprises: (1) a chain 1: the heavy chain of the second antigen-binding region that is linked to an antigen-binding portion in the first antigen-binding region at the C-terminus by a linker or by no linker; and

(2) a chain 2: the light chain of the second antigen-binding region.

In one embodiment, in the use to which the present disclosure relates, the linker comprises an amino acid sequence of (Gly4Ser)n or (GlySer4)n, wherein n is a positive integer from 1 to 7.

In one embodiment, in the use to which the present disclosure relates, the bispecific antibody consists of two chains 1 and two chains 2.

In one embodiment, in the use to which the present disclosure relates, the antigen-binding portion is selected from: (i) a Fab fragment; (ii) a F(ab')2 fragment; (iii) an Fd fragment; (iv) an Fv fragment; (v) a dAb fragment; (vi) a nanobody; and (vii) a single-chain Fv (scFv); the scFv comprises the first heavy chain variable region and the first light chain variable region (e.g., linked by a linker).

In one embodiment, in the use to which the present disclosure relates, the first antigen-binding region and/or the second antigen-binding region is from a mouse antibody, a human antibody, a chimeric antibody, or a humanized antibody.

In one embodiment, in the use to which the present disclosure relates, the first antigen-binding region and/or the second antigen-binding region is of the IgG1, IgG2, or IgG4 isotype.

In one embodiment, in the use to which the present disclosure relates, the chain 1 of the bispecific antibody comprises or consists of an amino acid sequence having at least 90%, 91%, 92%, 93%, 94%, 95%, 96%, 97%, 98%, or 99% identity to the amino acid sequence set forth in SEQ ID NO: 25, and the chain 2 of the bispecific antibody comprises or consists of an amino acid sequence having at least 90%, 91%, 92%, 93%, 94%, 95%, 96%, 97%, 98%, or 99% identity to the amino acid sequence set forth in SEQ ID NO: 26.

In one embodiment, in the use to which the present disclosure relates, the malignant tumor is bladder cancer, breast cancer, endometrioid carcinoma, cervical cancer, an advanced solid tumor, renal cell carcinoma, a neuroendocrine neoplasm, gastric cancer, transitional cell carcinoma, urothelial carcinoma, Hodgkin lymphoma, non-Hodgkin lymphoma, colorectal cancer, non-small cell lung cancer, or hepatocellular carcinoma. In a preferred embodiment, in the use to which the present disclosure relates, the neuroendocrine neoplasm is a neuroendocrine carcinoma or neuroendocrine tumor. In a relatively preferred embodiment, in the use to which the present disclosure relates, the neuroendocrine neoplasm is an extrapulmonary neuroendocrine neoplasm. In a more preferred embodiment, in the use to which the present disclosure relates, the neuroendocrine neoplasm is an advanced neuroendocrine neoplasm.

In one embodiment, in the use to which the present disclosure relates, the neuroendocrine carcinoma is an advanced neuroendocrine carcinoma. In a preferred embodiment, in the use to which the present disclosure relates, the neuroendocrine carcinoma is an extrapulmonary neuroendocrine carcinoma, e.g., an advanced extrapulmonary neuroendocrine carcinoma, or small cell lung cancer, e.g., extensive-stage small cell lung cancer. In a more preferred embodiment, in the use to which the present disclosure relates, the neuroendocrine carcinoma is an unresectable, locally advanced or metastatic neuroendocrine carcinoma.

In one embodiment, in the use to which the present disclosure relates, the drug is administered to a subject having the malignant tumor, and the subject is a subject who has not received systemic therapy or a subject who has experienced progression after receiving at least a prior chemotherapy first-line treatment. In an exemplary embodiment, the subject includes, but is not limited to, a subject who has experienced progression after receiving prior second-line or higher-line chemotherapy, e.g., a subject who has experienced progression after receiving prior second-line chemotherapy, a subject who has experienced progression after receiving prior third-line chemotherapy, or a subject who has experienced progression after receiving prior fourth-line chemotherapy.

In a specific embodiment, the subject may be a subject with small cell lung cancer who has not previously received systemic therapy, or a subject with an advanced extrapulmonary neuroendocrine carcinoma who has experienced progression after receiving at least a prior chemotherapy first-line treatment, including but not limited to a subject with an advanced extrapulmonary neuroendocrine carcinoma who has experienced progression after receiving prior second-line or higher-line chemotherapy, e.g., a subject with an advanced extrapulmonary neuroendocrine carcinoma who has experienced progression after receiving prior second-line chemotherapy, a subject with an advanced extrapulmonary neuroendocrine carcinoma who has experienced progression after receiving prior third-line chemotherapy, or a subject with an advanced extrapulmonary neuroendocrine carcinoma who has experienced progression after receiving prior fourth-line chemotherapy.

In a more specific embodiment, the chemotherapy treatment that the subject has received may be a platinum-containing chemotherapy treatment, e.g., a platinum-containing chemotherapy first-line treatment, a platinum-containing chemotherapy second-line treatment, or a platinum-containing chemotherapy third-line treatment.

In one embodiment, the use to which the present disclosure relates comprises administering to the subject the bispecific antibody at 0.2 mg/kg-200 mg/kg in each administration cycle or each time. In a preferred embodiment, the use to which the present disclosure relates comprises administering to the subject the bispecific antibody at 0.8 mg/kg-25 mg/kg in each administration cycle or each time. In a more preferred embodiment, the use to which the present disclosure relates comprises administering to the subject the bispecific antibody at 3.2 mg/kg-15 mg/kg in each administration cycle or each time. In a yet more preferred embodiment, the use to which the present disclosure relates comprises administering to the subject the bispecific antibody at 6 mg/kg-15 mg/kg in each administration cycle or each time. In a most preferred embodiment, the use to which the present disclosure relates comprises administering to the subject the bispecific antibody at 10 mg/kg-15 mg/kg in each administration cycle or each time.

In one embodiment, the use to which the present disclosure relates comprises administering to the subject 50 mg-5000 mg of the bispecific antibody in each administration cycle or each time. In a preferred embodiment, the use to which the present disclosure relates comprises administering to the subject 60 mg-4000 mg of the bispecific antibody in each administration cycle or each time. In a more preferred embodiment, the use to which the present disclosure relates comprises administering to the subject 80 mg-3750 mg of the bispecific antibody in each administration cycle or each time. In a most preferred embodiment, the use to which the present disclosure relates comprises administering to the subject 100 mg-3500 mg of the bispecific antibody in each administration cycle or each time. In some specific embodiments, the use to which the present disclosure relates comprises administering to the subject 200 mg, 400 mg, 600 mg, 800 mg, 1000 mg, 1250 mg, 1500 mg, 1750 mg, 2000 mg, 2250 mg, 2500 mg, 2750 mg, 3000 mg, 3250 mg, or 3500 mg of the bispecific antibody in each administration cycle or each time.

In one embodiment, in the use to which the present disclosure relates, the administration cycle of the bispecific antibody is as follows: the bispecific antibody is administered once every 12 weeks, once every 9 weeks, once every 6 weeks, once every 5 weeks, once every 4 weeks, once every 3 weeks, once every 2 weeks, once weekly, twice weekly, or three times weekly.

In one embodiment, in the use to which the present disclosure relates, the bispecific antibody is formulated as a formulation for intravenous infusion or subcutaneous injection.

In one embodiment, in the use to which the present disclosure relates, the bispecific antibody is further administered in combination with one or more chemotherapeutic agents. In a preferred embodiment, in the use to which the present disclosure relates, the chemotherapeutic agents are platinum-based agents and/or podophyllotoxin derivatives. In a more preferred embodiment, in the use to which the present disclosure relates, the platinum-based agents are carboplatin or cisplatin. In a most preferred embodiment, in the use to which the present disclosure relates, the podophyllotoxin derivatives are etoposide, etoposide glucuronide, or etoposide phosphate.

In one embodiment, in the use to which the present disclosure relates, an administration cycle of the chemotherapeutic agents is as follows: the chemotherapeutic agents are administered once every 5 weeks, once every 4 weeks, once every 3 weeks, once every 2 weeks, once weekly, twice weekly, or three times weekly.

In one embodiment, in the use to which the present disclosure relates, the chemotherapeutic agents are formulated for intravenous infusion or oral formulation administration.

In one embodiment, the use to which the present disclosure relates comprises, after administering the bispecific antibody to the subject in each administration cycle or each time after administering the bispecific antibody to the subject, administering the chemotherapeutic agents.

In one embodiment, the use to which the present disclosure relates comprises, after administering the bispecific antibody in each administration cycle or each time after administering the bispecific antibody, administering the podophyllotoxin derivatives for 1-5 days, e.g., 3 days or 1 day, starting from the day the bispecific antibody is administered. In a preferred embodiment, the use to which the present disclosure relates comprises administering the podophyllotoxin derivatives at 100 mg/m$^2$ or less daily.

In one embodiment, the use to which the present disclosure relates comprises, after administering the bispecific antibody in each administration cycle or each time after administering the bispecific antibody, administering the platinum-based agents for 1-5 days, e.g., 3 days or 1 day, starting from the day the bispecific antibody is administered. In a preferred embodiment, the use to which the present disclosure relates comprises administering the platinum-based agents at 75 mg/m$^2$ or less, or at AUC = 5 mg/mL/min or less, daily.

In a second aspect, the present disclosure relates to a drug for treating a malignant tumor, and the drug comprises the bispecific antibody defined in the first aspect.

In a third aspect, the present disclosure relates to a pharmaceutical combination comprising the bispecific antibody defined in the first aspect and one or more chemotherapeutic agents. In a preferred embodiment, the chemotherapeutic agents are platinum-based agents and/or podophyllotoxin derivatives. In a more preferred embodiment, the platinum-based agents are carboplatin or cisplatin. In a most preferred embodiment, the podophyllotoxin derivatives are etoposide, etoposide glucuronide, or etoposide phosphate. In a preferred embodiment, the pharmaceutical combination is used for treating a malignant tumor.

In one embodiment, the present disclosure relates to the drug or the pharmaceutical combination for treating a malignant tumor, wherein the malignant tumor is bladder cancer, breast cancer, endometrioid carcinoma, cervical cancer, an advanced solid tumor, renal cell carcinoma, a neuroendocrine neoplasm, gastric cancer, transitional cell carcinoma, urothelial carcinoma, Hodgkin lymphoma, non-Hodgkin lymphoma, colorectal cancer, non-small cell lung cancer, or hepatocellular carcinoma. In a preferred embodiment, the neuroendocrine neoplasm is a neuroendocrine carcinoma or neuroendocrine tumor. In a relatively preferred embodiment, the neuroendocrine neoplasm is an extrapulmonary neuroendocrine neoplasm. In a more preferred embodiment, the neuroendocrine neoplasm is an advanced neuroendocrine neoplasm.

In one embodiment, the neuroendocrine carcinoma is an advanced neuroendocrine carcinoma. In a preferred embodiment, the neuroendocrine carcinoma is an extrapulmonary neuroendocrine carcinoma, e.g., an advanced extrapulmonary neuroendocrine carcinoma, or small cell lung cancer, e.g., extensive-stage small cell lung cancer. In a more preferred embodiment, the neuroendocrine carcinoma is an unresectable, locally advanced or metastatic neuroendocrine carcinoma.

In one embodiment, the drug is administered to a subject having the malignant tumor, and the subject is a subject who has not received systemic therapy or a subject who has experienced progression after receiving at least a prior platinum-containing chemotherapy first-line treatment.

In an exemplary embodiment, the subject includes, but is not limited to, a subject who has experienced progression after receiving prior second-line or higher-line chemotherapy, e.g., a subject who has experienced progression after receiving prior second-line chemotherapy, a subject who has experienced progression after receiving prior third-line chemotherapy, or a subject who has experienced progression after receiving prior fourth-line chemotherapy.

In a specific embodiment, the subject may be a subject with small cell lung cancer who has not previously received systemic therapy, or a subject with an advanced extrapulmonary neuroendocrine carcinoma who has experienced progression after receiving at least a prior chemotherapy first-line treatment, including but not limited to a subject with an advanced extrapulmonary neuroendocrine carcinoma who has experienced progression after receiving prior second-line or higher-line chemotherapy, e.g., a subject with an advanced extrapulmonary neuroendocrine carcinoma who has experienced progression after receiving prior second-line chemotherapy, a subject with an advanced extrapulmonary neuroendocrine carcinoma who has experienced progression after receiving prior third-line chemotherapy, or a subject with an advanced extrapulmonary neuroendocrine carcinoma who has experienced progression after receiving prior fourth-line chemotherapy.

In a more specific embodiment, the chemotherapy treatment that the subject has received may be a platinum-containing chemotherapy treatment, e.g., a platinum-containing chemotherapy first-line treatment, a platinum-containing chemotherapy second-line treatment, or a platinum-containing chemotherapy third-line treatment.

In a fourth aspect, the present disclosure relates to a method for treating a malignant tumor, and the method comprises administering to the subject a bispecific antibody comprising a first antigen-binding region specifically binding to 4-1BB and a second antigen-binding region specifically binding to PD-L1.

In one embodiment, in the method to which the present disclosure relates, the first antigen-binding region comprises a first heavy chain variable region and/or a first light chain variable region, wherein:

the first heavy chain variable region:

(i) comprises an HCDR1, an HCDR2, and an HCDR3 derived from a heavy chain variable region consisting of the sequence set forth in SEQ ID NO: 7 or SEQ ID NO: 9;
(ii) comprises an HCDR1, an HCDR2, and an HCDR3 comprising or consisting of the amino acid sequences of SEQ ID NO: 1, SEQ ID NO: 2, and SEQ ID NO: 3, respectively, or comprising or consisting of amino acid sequences having one, two, or three modifications (preferably amino acid substitutions, and preferably conservative substitutions) compared to the amino acid sequences of SEQ ID NO: 1, SEQ ID NO: 2, and SEQ ID NO: 3, respectively; or
(iii) comprises or consists of the amino acid sequence of SEQ ID NO: 7 or SEQ ID NO: 9, or comprises or consists of an amino acid sequence having at least 80%, 81%, 82%, 83%, 84%, 85%, 86%, 87%, 88%, 89%, 90%, 91%, 92%, 93%, 94%, 95%, 96%, 97%, 98%, or 99% identity to the amino acid sequence of SEQ ID NO: 7 or SEQ ID NO: 9, or comprises or consists of an amino acid sequence having one or more (preferably no more than 10, and more preferably no more than 5) amino acid modifications (preferably amino acid substitutions, and more preferably conservative substitutions) compared to the amino acid sequence of SEQ ID NO: 7 or SEQ ID NO: 9, wherein preferably, the amino acid modifications do not occur in a CDR; more preferably, the amino acid modifications occur in an FR, for example, in an FR1, an FR2, an FR3, or an FR4;

the first light chain variable region:

(i) comprises an LCDR1, an LCDR2, and an LCDR3 derived from a light chain variable region consisting of the sequence set forth in SEQ ID NO: 8 or SEQ ID NO: 10;

(ii) comprises an LCDR1, an LCDR2, and an LCDR3 comprising or consisting of the sequences set forth in SEQ ID NO: 4, SEQ ID NO: 5, and SEQ ID NO: 6, respectively, or comprising or consisting of amino acid sequences having one, two, or three modifications (preferably amino acid substitutions, and preferably conservative substitutions) compared to the amino acid sequences of SEQ ID NO: 4, SEQ ID NO: 5, and SEQ ID NO: 6, respectively; or

(iii) comprises or consists of the amino acid sequence of SEQ ID NO: 8 or SEQ ID NO: 10, or comprises or consists of an amino acid sequence having at least 80%, 81%, 82%, 83%, 84%, 85%, 86%, 87%, 88%, 89%, 90%, 91%, 92%, 93%, 94%, 95%, 96%, 97%, 98%, or 99% identity to the amino acid sequence of SEQ ID NO: 8 or SEQ ID NO: 10, or comprises or consists of an amino acid sequence having one or more (preferably no more than 10, and more preferably no more than 5) amino acid modifications (preferably amino acid substitutions, and more preferably conservative substitutions) compared to the amino acid sequence of SEQ ID NO: 8 or SEQ ID NO: 10, wherein preferably, the amino acid modifications do not occur in a CDR; more preferably, the amino acid modifications occur in an FR, for example, in an FR1, an FR2, an FR3, or an FR4;

in SEQ ID NO: 8, X = S or G.

In one embodiment, in the method to which the present disclosure relates, the first antigen-binding region further comprises a first heavy chain constant region and/or a first light chain constant region, wherein the first heavy chain constant region comprises or consists of the amino acid sequence set forth in SEQ ID NO: 19 or 20, and the first light chain constant region comprises or consists of the amino acid sequence set forth in SEQ ID NO: 21 or 22.

In one embodiment, in the method to which the present disclosure relates, the first antigen-binding region activates 4-1BB signaling and is a monoclonal antibody.

In one embodiment, in the method to which the present disclosure relates, the second antigen-binding region specifically binding to PD-L1 comprises a second heavy chain variable region and a second light chain variable region, wherein the second heavy chain variable region comprises an HCDR1, an HCDR2, and an HCDR3 consisting of the sequences set forth in SEQ ID NO: 11, SEQ ID NO: 12, and SEQ ID NO: 13, respectively, and the second light chain variable region comprises an LCDR1, an LCDR2, and an LCDR3 consisting of the sequences set forth in SEQ ID NO: 14, SEQ ID NO: 15, and SEQ ID NO: 16, respectively.

In one embodiment, in the method to which the present disclosure relates, the second antigen-binding region further comprises a second heavy chain constant region and/or a second light chain constant region, wherein the second heavy chain constant region comprises or consists of the amino acid sequence set forth in SEQ ID NO: 19 or 20, and the second light chain constant region comprises or consists of the amino acid sequence set forth in SEQ ID NO: 21 or 22.

In one embodiment, in the method to which the present disclosure relates, the bispecific antibody comprises: (1) a chain 1: the heavy chain of the second antigen-binding region that is linked to an antigen-binding portion in the first antigen-binding region at the C-terminus by a linker or by no linker; and

(2) a chain 2: the light chain of the second antigen-binding region.

In one embodiment, in the method to which the present disclosure relates, the linker comprises an amino acid sequence of (Gly4Ser)n or (GlySer4)n, wherein n is a positive integer from 1 to 7.

In one embodiment, in the method to which the present disclosure relates, the bispecific antibody consists of two chains 1 and two chains 2.

In one embodiment, in the method to which the present disclosure relates, the antigen-binding portion is selected from: (i) a Fab fragment; (ii) a F(ab')2 fragment; (iii) an Fd fragment; (iv) an Fv fragment; (v) a dAb fragment; (vi) a nanobody; and (vii) a single-chain Fv (scFv); the scFv comprises the first heavy chain variable region and the first light chain variable region.

In one embodiment, in the method to which the present disclosure relates, the first antigen-binding region and/or the second antigen-binding region is a mouse antibody, a human antibody, a chimeric antibody, or a humanized antibody.

In one embodiment, in the method to which the present disclosure relates, the first antigen-binding region and/or the second antigen-binding region is of the IgG1, IgG2, or IgG4 isotype.

In one embodiment, in the method to which the present disclosure relates, the chain 1 of the bispecific antibody comprises or consists of an amino acid sequence having at least 90%, 91%, 92%, 93%, 94%, 95%, 96%, 97%, 98%, or 99% identity to the amino acid sequence set forth in SEQ ID NO: 25, and the chain 2 of the bispecific antibody comprises or consists of an amino acid sequence having at least 90%, 91%, 92%, 93%, 94%, 95%, 96%, 97%, 98%, or 99% identity to the amino acid sequence set forth in SEQ ID NO: 26.

In one embodiment, in the method to which the present disclosure relates, the malignant tumor is bladder cancer, breast cancer, endometrioid carcinoma, cervical cancer, an advanced solid tumor, renal cell carcinoma, a neuroendocrine neoplasm, gastric cancer, transitional cell carcinoma, urothelial carcinoma, Hodgkin lymphoma, non-Hodgkin lymphoma, colorectal cancer, non-small cell lung cancer, or hepatocellular carcinoma. In a preferred embodiment, in the method to which the present disclosure relates, the neuroendocrine neoplasm is a neuroendocrine carcinoma or neuroendocrine tumor. In a relatively preferred embodiment, in the method to which the present disclosure relates, the neuroendocrine neoplasm is an extrapulmonary neuroendocrine neoplasm. In a more preferred embodiment, in the method to which the present disclosure relates, the neuroendocrine neoplasm is an advanced neuroendocrine neoplasm.

In one embodiment, in the method to which the present disclosure relates, the neuroendocrine carcinoma is an advanced neuroendocrine carcinoma. In another embodiment, in the method to which the present disclosure relates, the neuroendocrine carcinoma is an extrapulmonary neuroendocrine carcinoma or small cell lung cancer. In one specific embodiment, in the method to which the present disclosure relates, the neuroendocrine carcinoma is an unresectable, locally advanced or metastatic neuroendocrine carcinoma.

In one embodiment, the method to which the present disclosure relates comprises administering the bispecific antibody to a subject having the malignant tumor, and the subject is a subject who has not received systemic therapy or a subject who has experienced progression after receiving at least a prior platinum-containing chemotherapy first-line treatment.

In an exemplary embodiment, the subject includes, but is not limited to, a subject who has experienced progression after receiving prior second-line or higher-line chemotherapy, e.g., a subject who has experienced progression after receiving prior second-line chemotherapy, a subject who has experienced progression after receiving prior third-line chemotherapy, or a subject who has experienced progression after receiving prior fourth-line chemotherapy.

In a specific embodiment, the subject may be a subject with small cell lung cancer who has not previously received systemic therapy, or a subject with an advanced extrapulmonary neuroendocrine carcinoma who has experienced progression after receiving at least a prior chemotherapy first-line treatment, including but not limited to a subject with an advanced extrapulmonary neuroendocrine carcinoma who has experienced progression after receiving prior second-line or higher-line chemotherapy, e.g., a subject with an advanced extrapulmonary neuroendocrine carcinoma who has experienced progression after receiving prior second-line chemotherapy, a subject with an advanced extrapulmonary neuroendocrine carcinoma who has experienced progression after receiving prior third-line chemotherapy, or a subject with an advanced extrapulmonary neuroendocrine carcinoma who has experienced progression after receiving prior fourth-line chemotherapy.

In a more specific embodiment, the chemotherapy treatment that the subject has received may be a platinum-containing chemotherapy treatment, e.g., a platinum-containing chemotherapy first-line treatment, a platinum-containing chemotherapy second-line treatment, or a platinum-containing chemotherapy third-line treatment.

In one embodiment, the method to which the present disclosure relates comprises administering to the subject the bispecific antibody at 0.2 mg/kg-200 mg/kg in each administration cycle or each time. In a preferred embodiment, the method to which the present disclosure relates comprises administering to the subject the bispecific antibody at 0.8 mg/kg-25 mg/kg in each administration cycle or each time. In a more preferred embodiment, the method to which the present disclosure relates comprises administering to the subject the bispecific antibody at 3.2 mg/kg-20 mg/kg in each administration cycle or each time. In a yet more preferred embodiment, the method to which the present disclosure relates comprises administering to the subject the bispecific antibody at 6.0 mg/kg-15 mg/kg in each administration cycle or each time. In a most preferred embodiment, the method to which the present disclosure relates comprises administering to the subject the bispecific antibody at 10 mg/kg-15 mg/kg in each administration cycle or each time.

In one embodiment, the method to which the present disclosure relates comprises administering to the subject 50 mg-5000 mg of the bispecific antibody in each administration cycle or each time. In a preferred embodiment, the method to which the present disclosure relates comprises administering to the subject 60 mg-4000 mg of the bispecific antibody in each administration cycle or each time. In a more preferred embodiment, the method to which the present disclosure relates comprises administering to the subject 80 mg-3750 mg of the bispecific antibody in each administration cycle or each time. In a most preferred embodiment, the method to which the present disclosure relates comprises administering to the subject 100 mg-3500 mg of the bispecific antibody in each administration cycle or each time. In some specific embodiments, the method to which the present disclosure relates comprises administering to the subject 200 mg, 400 mg, 600 mg, 800 mg, 1000 mg, 1250 mg, 1500 mg, 1750 mg, 2000 mg, 2250 mg, 2500 mg, 2750 mg, 3000 mg, 3250 mg, or 3500 mg of the bispecific antibody in each administration cycle or each time.

In one embodiment, in the method to which the present disclosure relates, the administration cycle of the bispecific antibody is as follows: the bispecific antibody is administered once every 12 weeks, once every 9 weeks, once every 6 weeks, once every 5 weeks, once every 4 weeks, once every 3 weeks, once every 2 weeks, once weekly, twice weekly, or three times weekly.

In one embodiment, in the method to which the present disclosure relates, the bispecific antibody is formulated as a formulation for intravenous infusion or subcutaneous injection.

In one embodiment, the method to which the present disclosure relates further comprises administering to the subject one or more chemotherapeutic agents. In a preferred embodiment, in the method to which the present disclosure relates, the chemotherapeutic agents are platinum-based agents and/or podophyllotoxin derivatives. In a more preferred embodiment, in the method to which the present disclosure relates, the platinum-based agents are carboplatin or cisplatin. In a most preferred embodiment, in the method to which the present disclosure relates, the podophyllotoxin derivatives are etoposide, etoposide glucuronide, or etoposide phosphate.

In one embodiment, in the method to which the present disclosure relates, an administration cycle of the chemotherapeutic agents is as follows: the chemotherapeutic agents are administered once every 5 weeks, once every 4 weeks, once every 3 weeks, once every 2 weeks, once weekly, twice weekly, or three times weekly.

In one embodiment, in the method to which the present disclosure relates, the chemotherapeutic agents are formulated for intravenous infusion or oral formulation administration.

In one embodiment, the method to which the present disclosure relates comprises, after administering the bispecific antibody to the subject in each administration cycle or each time after administering the bispecific antibody to the subject, administering the chemotherapeutic agents.

In one embodiment, the method to which the present disclosure relates comprises, after administering the bispecific antibody in each administration cycle or each time after administering the bispecific antibody, administering the podophyllotoxin derivatives for 1-5 days, e.g., 3 days or 1 day, starting from the day the bispecific antibody is administered. In a preferred embodiment, the method to which the present disclosure relates comprises administering the podophyllotoxin derivatives at 100 mg/m$^2$ or less daily.

In one embodiment, the method to which the present disclosure relates comprises, after administering the bispecific antibody in each administration cycle or each time after administering the bispecific antibody, administering the platinum-based agents for 1-5 days, e.g., 3 days or 1 day, starting from the day the bispecific antibody is administered. In a preferred embodiment, the method to which the present disclosure relates comprises administering the platinum-based agents at 75 mg/m$^2$ or less, or at AUC = 5 mg/mL/min or less, daily.

## BRIEF DESCRIPTION OF THE DRAWINGS

FIG. 1 shows the structure of bispecific antibody P4B-3.

FIG. 2 shows curves of the serum concentration of antibody P4B-3 changing over time in different administration cycles, wherein FIG. 2A shows curves of the serum concentration changing over time in the first cycle, and FIG. 2B shows curves of the serum concentration changing over time in the fifth cycle.

FIG. 3 shows release levels of the peripheral cytokine IFN-$\gamma$ after the administration of a single dose of antibody P4B-3.

FIG. 4 shows an analysis of the correlation of antibody P4B-3 and PD-L1 expression with efficacy.

## DETAILED DESCRIPTION

To aid in understanding the present disclosure, certain terms are defined first. Additional definitions are set forth throughout the detailed description.

The term "4-1BB" refers to tumor necrosis factor receptor superfamily member 9. The term "4-1BB" includes variants, isotypes, homologs, orthologs, and paralogs. For example, an antibody specific to the human 4-1BB protein may, in certain cases, cross-react with a 4-1BB protein from a species other than humans (such as monkeys). In other embodiments, an antibody specific to the human 4-1BB protein may be fully specific to the human 4-1BB protein and exhibit no cross-reactivity with other species or other types, or may cross-react with 4-1BB from some other species, but not all others.

The term "human 4-1BB" refers to a 4-1BB protein having an amino acid sequence from humans, such as the amino acid sequence of human 4-1BB with Genbank Accession No. NP_001552.2. In one embodiment, human 4-1BB comprises the amino acid sequence set forth in SEQ ID NO: 48. The terms "monkey or rhesus monkey 4-1BB" and "mouse 4-1BB" refer to monkey and mouse 4-1BB sequences, respectively, e.g., monkey and mouse 4-1BB sequences having amino acid sequences with Genbank Accession Nos. NP_001253057.1 and NP_033430.1, respectively.

The term "antibody" as referred to herein includes intact antibodies and any antigen-binding fragment (i.e., "antigen-binding portion") or single chain thereof. In addition, the term "antibody" as referred to herein also encompasses multispecific antibodies, such as bispecific antibodies or trispecific antibodies. An intact antibody is a glycoprotein comprising at least two heavy (H) chains and two light (L) chains interlinked by disulfide bonds. Each heavy chain comprises a heavy chain variable region (abbreviated herein as $V_H$) and a heavy chain constant region (abbreviated herein as $C_H$). The heavy chain constant region comprises three domains: CH1, CH2, and CH3. Each light chain comprises a light chain variable region (abbreviated herein as $V_L$) and a light chain constant region (abbreviated herein as $C_L$). The light chain constant region comprises one domain: CL. The VH and $V_L$ regions can be further subdivided into hypervariable regions termed complementarity determining regions (CDR). The hypervariable regions alternate with regions that are more conserved, termed framework regions (FR). Each $V_H$ and $V_L$ is composed of three CDRs and four FRs arranged from the amino-terminus to the carboxyl-terminus in the following order: FR1, CDR1, FR2, CDR2, FR3, CDR3, FR4. The variable regions of the heavy and light chains comprise binding domains that interact with antigens. The constant regions of an antibody can mediate the binding of immunoglobulins to host tissues or factors, including various cells of the immune system (e.g., effector cells) and the first component (C1q) of the classical complement system.

The term "antigen-binding portion" of an antibody (or simply "antibody portion") as used herein refers to one or more fragments of the antibody that retain the ability to specifically bind to an antigen (e.g., a 4-1BB protein). It has been demonstrated that the antigen-binding function of an antibody can be fulfilled by fragments of the full-length antibody. Examples of binding fragments encompassed within the term "antigen-binding portion" of an antibody include (i) a Fab fragment, i.e., a monovalent fragment consisting of a $V_L$ domain, a $V_H$ domain, a $C_L$ domain, and a $C_{H1}$ domain; (ii) a F(ab') 2 fragment, i.e., a bivalent fragment comprising two Fab fragments linked by a disulfide bridge in the hinge region; (iii) an Fd fragment consisting of a VH domain and a CH1 domain; (iv) an Fv fragment consisting of the VL and VH domains of a single arm; (v) a dAb fragment consisting of a VH domain (Ward et al. (1989) Nature 341:544-546); (vi) isolated complementarity determining regions (CDRs); and (vii) a nanobody, i.e., a heavy chain variable region comprising a single variable domain and two constant domains. In addition, although the two domains of the Fv fragment, VL and VH, are encoded by separate genes, they can be joined, using a recombinant method, by a synthetic linker that enables them to be made into a single protein chain in which the VL and VH regions are paired to form a monovalent molecule (referred to as single-chain Fv (scFv); see, e.g., Bird et al. (1988) Science 242:423-426; and Huston et al. (1988) Proc. Natl. Acad. Sci. USA 85:5879-5883). These single-chain antibodies are also intended to be encompassed within the term "antigen-binding portion" of an antibody. These antibody fragments are obtained using conventional techniques known to those skilled in the art, and the fragments are screened for utility in the same manner as intact antibodies.

The term "bispecific" means that the antibody is capable of specifically binding to at least two different antigenic determinants. Typically, a bispecific antibody comprises two antigen-binding regions, wherein each of the antigen-binding regions is specific to a different antigenic determinant. In certain embodiments, the bispecific antibody is capable of simultaneously binding two antigenic determinants, particularly two antigenic determinants expressed on two different cells. The term "antigenic determinant" as used herein is synonymous with "antigen" and "epitope", and refers to a site (e.g., a contiguous stretch of amino acids or a conformational configuration consisting of different regions of non-contiguous amino acids) on a polypeptide macromolecule to which an antigen-binding portion binds to form an antigen-binding region-antigen complex. Useful antigenic determinants may be present, for example, on the surface of tumor cells, on the surface of virus-infected cells, on the surface of other diseased cells, on the surface of immune cells, in free substances in serum, and/or in the extracellular matrix (ECM).

The term "antigen-binding region" as used herein refers to a polypeptide molecule that specifically binds to an antigenic determinant. In one embodiment, the antigen-binding region is capable of directing the entity to which it is linked (e.g., the second antigen-binding region) to a target site, e.g., to a specific type of tumor cell bearing the antigenic determinant. In another embodiment, the antigen-binding region (e.g., the first antigen-binding region) is capable of activating signaling through a target antigen thereof, e.g., a T cell receptor antigen. Antigen-binding regions include antibodies or antigen-binding fragments thereof as defined herein. Particular antigen-binding regions include an antigen-binding fragment of an antibody, wherein the antibody comprises an antibody heavy chain variable region and an antibody light chain variable region. In certain embodiments, the antigen-binding region may comprise an antibody constant region as defined herein and known in the art.

"Specific binding" means that the binding is selective for antigens and can be distinguished from unwanted or non-specific interactions. The ability of an antigen-binding region to bind to a particular antigenic determinant can be determined by enzyme-linked immunosorbent assay (ELISA) or other techniques familiar to those skilled in the art, such as the surface plasmon resonance (SPR) technique (e.g., analysis on a BIAcore instrument).

Unless otherwise specified, the term "binding affinity" as used herein refers to intrinsic binding affinity that reflects a 1:1 interaction between the members of a binding pair (e.g., a monoclonal antibody and an antigen, an antigen-binding region and an antigen, or a receptor and a ligand thereof). Affinity can be determined by methods well known in the art, including those described herein.

The term "isolated antibody" as used herein is intended to refer to an antibody that is substantially free of other antibodies having different antigenic specificities (for example, an isolated antibody that specifically binds to a 4-1BB protein is substantially free of antibodies that specifically bind to antigens other than the 4-1BB protein). However, an isolated antibody that specifically binds to the human 4-1BB protein may exhibit cross-reactivity with other antigens, such as 4-1BB proteins from other species. In addition, an isolated antibody may be substantially free of other cellular materials and/or chemical substances.

The term "monoclonal antibody" or "monoclonal antibody composition" as used herein refers to a formulation of an antibody molecule having a single molecular composition. A monoclonal antibody composition exhibits a single binding specificity and affinity for a particular epitope.

The term "human antibody" as used herein is intended to include antibodies having variable regions in which both the framework regions and the CDRs are derived from human germline immunoglobulin sequences. In addition, if the antibodies comprise constant regions, the constant regions are also derived from human germline immunoglobulin sequences. The human antibody of the present disclosure may include amino acid residues not encoded by human germline immunoglobulin sequences (e.g., mutations introduced by random or site-specific mutagenesis in vitro or by somatic cell mutations in vivo). However, the term "human antibody" as used herein is not intended to include antibodies in which CDR sequences derived from the germline of another mammalian species have been grafted onto human framework sequences.

The term "agonistic 4-1BB antibody" or "agonistic anti-4-1BB antibody" refers to an anti-4-1BB antibody that binds to 4-1BB and activates or induces 4-1BB signaling to promote the activation and/or proliferation of immune cells (such as T cells).

The term "isotype" refers to the class of antibodies encoded by heavy chain constant region genes (e.g., IgM or IgG1).

The phrases "an antibody that recognizes an antigen" and "an antibody that is specific to an antigen" are used interchangeably herein with the term "an antibody that specifically binds to an antigen".

An antibody that "specifically binds to 4-1BB" as used herein means an antibody that binds to a 4-1BB protein (including 4-1BB proteins from humans and possibly from one or more non-human species) but does not substantially bind to non-4-1BB proteins. Preferably, the antibody binds to the human 4-1BB protein with a KD of $1.0 \times 10^{-6}$ M or less, preferably $5.0 \times 10^{-7}$ M or less, and more preferably $1.0 \times 10^{-7}$ M or less.

The term "not substantially bind to" a protein or cell as used herein refers to not specifically binding or not binding with high affinity to the protein or cell, i.e., binding to the protein or cell with a KD of $1.0 \times 10^{-6}$ M or greater, preferably $1.0 \times 10^{-5}$ M or greater, preferably $1.0 \times 10^{-4}$ M or greater, more preferably $1.0 \times 10^{-3}$ M or greater, and even more preferably $1.0 \times 10^{-2}$ M or greater.

For an IgG antibody or an antigen-binding region thereof, the term "specifically binds to a second antigen (PD-L1) with high affinity" means that the antibody or the antigen-binding region has a KD of $1.0 \times 10^{-8}$ M or less, preferably $5.0 \times 10^{-9}$ M or less, and more preferably $1.0 \times 10^{-9}$ M or less.

The term "K association" or "Ka" as used herein is intended to refer to the association rate of a particular antibody-antigen interaction, and the term "K dissociation" or "Kd" as used herein is intended to refer to the dissociation rate of a particular antibody-antigen interaction. The term "KD" as used herein is intended to refer to the dissociation constant, which is obtained from the ratio of Kd to Ka (i.e., Kd/Ka) and is expressed as a molar concentration (M). The KD value of an antibody can be determined using methods well known in the art. A preferred method for determining the KD of an antibody is by using surface plasmon resonance, preferably using a biosensor system, such as a Biacore™ system.

The term "EC50", also known as half maximal effective concentration, refers to an antibody concentration that elicits a response halfway between the baseline and the maximum after a specified exposure time.

The term "IC50", also known as half maximal inhibitory concentration, refers to an antibody concentration that inhibits a specific biological or biochemical function by 50% relative to the case where the antibody is absent.

The term "subject" includes any human or non-human animal. The term "non-human animal" includes all vertebrates, e.g., mammals and non-mammals, such as non-human primates, sheep, dogs, cats, cattle, horses, chickens, amphibians, and reptiles, although mammals are preferred, such as non-human primates, sheep, dogs, cats, cattle, and horses.

The term "therapeutically effective amount" refers to an amount of the antibody of the present disclosure that is sufficient to prevent or ameliorate a symptom associated with a disease or condition (such as cancer) and/or reduce the severity of the disease or condition. A therapeutically effective amount is understood to relate to the condition being treated, wherein the actual effective amount is readily discerned by those skilled in the art.

The term "therapeutic agent" as described herein encompasses any substance effective in preventing or treating tumors (such as cancer) or infections or autoimmune diseases, including chemotherapeutic agents, cytotoxic agents, vaccines, other antibodies (e.g., antibodies against immune checkpoint molecules), anti-infective agents, immunomodulatory agents, and small molecule drugs.

The term "chemotherapeutic agent" includes compounds for treating cancer.

The term "anti-infective agent" includes any molecule that, at the administration concentration and interval of administration, specifically inhibits or eliminates the growth of microorganisms (e.g., viruses, bacteria, fungi, or protozoa, such as parasites) but is not lethal to the host.

As used herein, the term anti-infective agent includes antibiotics, antibacterials, antivirals, antifungals, and antiprotozoals. In one specific aspect, the anti-infective agent is non-toxic to the host at the administration concentration and interval of administration.

Immunomodulatory agents include immune checkpoint molecule inhibitors and co-stimulatory molecule activators.

The term "small molecule drug" refers to a low-molecular-weight organic compound capable of regulating biological processes. "Small molecule" is defined as a molecule that generally has a molecular weight of less than 2 kD, preferably less than 1 kD, and more preferably about 0.5 kD or less. Small molecules include, but are not limited to, inorganic molecules, organic molecules, organic molecules containing inorganic components, molecules containing radioactive atoms, synthetic molecules, peptide mimetics, and antibody mimetics.

The terms "cancer" and "cancerous" refer to or describe a physiological disease in mammals that is typically characterized by unregulated cell growth.

The term "tumor" refers to all neoplastic cell growth and proliferation (whether malignant or benign) and all pre-cancerous and cancerous cells and tissues. The terms "cancer", "cancerous", "cell proliferative disorder", "proliferative disorder", and "tumor" are not mutually exclusive when referred to herein.

The term "neuroendocrine neoplasm" (NEN) refers to a class of neoplasms that originate from peptidergic neurons and neuroendocrine cells, have neuroendocrine differentiation, and express neuroendocrine markers. NENs may occur in various parts of the body and are highly heterogeneous, with pulmonary and gastroenteropancreatic NENs being the most common. According to the degree of differentiation, NENs are classified into neuroendocrine tumors (NETs), which are well differentiated and grow slowly, neuroendocrine carcinomas (NECs), which are poorly differentiated and are highly malignant, and mixed neuroendocrine-non-neuroendocrine neoplasms (MiNENs). NECs include small cell types (SCNECs) and large cell types (LCNECs); NECs are also classified into pulmonary NECs [including small cell lung cancer (SCLC) and pulmonary large cell NECs (p-LCNECs)] and extrapulmonary NECs (EP-NECs). Most NEC cases are diagnosed at relatively advanced stages or with distant metastases, resulting in a poor prognosis. Data analysis based on 162,983 neuroendocrine carcinoma patients from the U.S. SEER database showed that the 5-year survival rates for patients with pulmonary NECs, patients with gastrointestinal NECs, and patients with NECs in other parts were 5.6%, 13.1%, and 26.0%, respectively.

The term "non-small cell lung cancer" (NSCLC) has its general meaning in the art. NSCLC is the most common lung cancer and includes three major types: squamous cell carcinoma, large cell carcinoma, and adenocarcinoma.

The term "hepatocellular carcinoma" (HCC) has its general meaning in the art. HCC is a malignant tumor of hepatocytes and is one of the common types of primary liver cancer.

The term "infection" refers to a disease caused by a pathogen, including, e.g., viral infections, bacterial infections, fungal infections, or protozoal infections such as parasitic infections.

The term "tumor immune escape" means that the tumor evades immune recognition and clearance. Thus, as a concept of treatment, when the escape is weakened, tumor immunity is "treated", and the tumor is recognized and attacked by the immune system. Examples of tumor recognition include tumor binding, tumor shrinkage, and tumor clearance.

The term "pharmaceutical combination" or "pharmaceutical combination product" refers to a non-fixed combination product or a fixed combination product. The term "non-fixed combination" means that the active ingredients (e.g., (i) the antibody of the present disclosure, and platinum-based agents and/or podophyllotoxin derivatives) are administered as separate entities to a patient, either simultaneously without a specific time limitation or sequentially at identical or different time intervals, wherein such administration provides prophylactically or therapeutically effective levels of the two active agents in the patient. An exemplary non-fixed combination product is a kit. In some embodiments, the antibody of the present disclosure and the platinum-based agents and/or podophyllotoxin derivatives used in the pharmaceutical combination are administered at levels that do not exceed those when they are used alone. The term "fixed combination" means that two active agents are simultaneously administered in the form of a single entity to a patient. The dose and/or time intervals of the two active agents are preferably selected such that the combined use of the ingredients can result in a therapeutic effect on the disease or disorder greater than that achieved by the use of any one of the ingredients alone. Each ingredient may take a separate formulation form, and their formulation forms may be the same or different. The ingredients may also be used as pharmaceutical compositions.

The term "pharmaceutical composition" refers to a mixture consisting of one or more active ingredients and a pharmaceutically acceptable excipient.

The term "combination therapy" refers to the administration of two or more therapeutic agents or modalities of treatment (e.g., radiotherapy or surgery) to treat the diseases as described herein. Such administration includes co-administration of these therapeutic agents in a substantially simultaneous manner, for example, in a single capsule with a fixed proportion of active ingredients. Alternatively, such administration includes co-administration of the active ingredients in a variety of or separate containers (e.g., tablets, capsules, powders, and liquids). The powder and/or liquid may be reconstituted or diluted to a desired dose before administration. In addition, such administration further includes using each type of therapeutic agent at approximately the same time or in a sequential manner at different times. In any case, the therapeutic regimen will provide the beneficial effect of the pharmaceutical combination in the treatment of disorders or symptoms described herein.

"Subject/patient/individual sample" refers to a collection of cells or fluids obtained from a patient or a subject. The source of tissue or cell samples can be solid tissues, e.g., from fresh, frozen, and/or preserved organ or tissue samples or biopsy samples or puncture samples; blood or any blood component; body fluids such as tears, vitreous humors, cerebrospinal fluids, amniotic fluids, peritoneal fluids, or interstitial fluids; and cells from a subject at any time during pregnancy or development. In some embodiments, the tissue sample is a tumor tissue. The tissue sample may comprise compounds that are not naturally mixed with tissues, such as preservatives, anticoagulants, buffers, fixatives, nutrients, and antibiotics.

The term "cycle" refers to a specific period of time expressed in days or weeks that is repeated on a regular schedule. For example, each treatment cycle (or prevention cycle) of administering the pharmaceutical combination of the present disclosure is 14 to 30 days, e.g., 14 to 28 days; preferably, each cycle is two weeks (i.e., 14 days), three weeks (i.e., 21 days), or four weeks (i.e., 28 days). The components of the pharmaceutical combination of the present disclosure may be administered on the same day or different days of a cycle; that is, the (i) and (ii) of the pharmaceutical combination of the present disclosure are administered separately, simultaneously, or sequentially within the cycle. The term "administer", "administration", "administering", or "administered" refers to physically introducing the active ingredient(s) of the drug or pharmaceutical combination of the present disclosure into an individual using any one of a variety of methods and delivery systems known to those skilled in the art. Routes of administration for the active ingredient(s) of the drug or pharmaceutical combination of the present disclosure include oral, intravenous (e.g., infusion (also known as drip infusion) or injection), intramuscular, subcutaneous, intraperitoneal, spinal, topical, or other parenteral routes of administration. The phrase "parenteral administration" as used herein refers to modes of administration other than gastrointestinal administration and topical administration, typically by intravenous injection and infusion, including but not limited to intramuscular, intraarterial, intrathecal, intralymphatic, intralesional, intracapsular, intraorbital, intracardiac, intradermal, intraperitoneal, transtracheal, subcutaneous, subcuticular, intraarticular, subcapsular, subarachnoid, intraspinal, epidural, and intrasternal injection and infusion, as well as in vivo electroporation. Accordingly, the active ingredient(s) of the drug or pharmaceutical combination of the present disclosure may be formulated as capsules, tablets, injections (including infusion or injection solutions), syrups, sprays, lozenges, liposomes, suppositories, or the like.

The term "continuous administration" refers to daily administration. In the case of continuous administration, the drug may be administered once or multiple times daily; for example, the drug is administered once daily, twice daily, or three times daily, preferably once daily.

The term "dose" is an amount of the drug that elicits a therapeutic effect. Unless otherwise specified, the dose is related to the amount of the drug in free form. If the drug is in the form of a pharmaceutically acceptable salt, the amount of the drug is increased proportionally compared to the amount of the drug in free form. For example, the dose would be declared on a product package or in a product information leaflet.

The term "pharmaceutically acceptable salt" includes, but is not limited to, acid addition salts and base addition salts, for example: acid addition salts formed from a compound of formula (I) with inorganic acids, such as hydrochloride, hydrobromide, carbonate, bicarbonate, phosphate, sulfate, sulfite, and nitrate; and acid addition salts formed from a compound of formula (I) with organic acids, such as formate, acetate, malate, maleate, fumarate, tartrate, succinate, citrate, lactate, methanesulfonate, p-toluenesulfonate, 2-hydroxyethanesulfonate, benzoate, salicylate, stearate, and salts formed with alkanedicarboxylic acids of the formula $HOOC\text{-}(CH_2)_n\text{-}COOH$ (wherein n is 0-4). "Pharmaceutically acceptable salt" also includes base addition salts formed from a compound of formula (I) having an acidic group with pharmaceutically acceptable cations such as sodium, potassium, calcium, aluminum, lithium, and ammonium.

The term "pharmaceutically acceptable" refers to those compounds, materials, compositions, and/or dosage forms that are suitable for use in contact with human and animal tissues without excessive toxicity, irritation, allergic responses, or other problems or complications, commensurate with a reasonable benefit/risk ratio.

The term "adverse event" (AE) is any unfavorable and generally unintended or undesirable sign (including an abnormal laboratory finding), symptom, or disease associated with the use of a medical treatment. For example, an adverse event may be associated with activation of the immune system in response to a treatment or expansion of immune system cells (e.g., T cells) in response to a treatment. A medical treatment may have one or more related AEs, and each AE may have the same level of severity or a different level of severity.

The term "overall survival" or "OS" is the time from a patient's first dose of the study drug to death from any cause.

The term "progression-free survival" or "PFS" is the time from a patient's first dose of the study drug to disease progression or death from any cause.

All numerical value ranges herein are to be understood to disclose each numerical value and subset of numerical values within the ranges, regardless of whether they are specifically disclosed otherwise or not. For example, when any numerical value range is mentioned, it should be considered that every numerical value within the numerical value range, e.g., every integer within the numerical value range, is mentioned. The present disclosure involves all values falling within these ranges, all smaller ranges, and the upper or lower limits of numerical value ranges.

The term "therapeutically effective amount" is an amount that is sufficient to provide a therapeutic benefit in the treatment or management of cancer or to delay or minimize one or more symptoms associated with cancer. A therapeutically effective amount of a compound refers to an amount of a therapeutic agent, alone or in combination with other therapeutic agents, which provides a therapeutic benefit in the treatment or management of cancer. The term "therapeutically effective amount" may encompass an amount that improves overall therapy for cancer, palliates or avoids symptoms or causes of cancer, or enhances the therapeutic efficacy of another therapeutic agent. An example of an "effective amount" is an amount that is sufficient to contribute to the treatment, prevention, or palliation of one or more symptoms of a disease, which could also be referred to as a "therapeutically effective amount". "Palliation" of a symptom(s) means a reduction in the severity or frequency of one or more symptoms, or elimination of one or more symptoms. The exact amount of a composition (including a "therapeutically effective amount") will depend on the purpose of the treatment, and can be determined by those skilled in the art using known techniques (see, e.g., Lieberman, Pharmaceutical Dosage Forms (volumes 1-3, 1992); Lloyd, The Art, Science and Technology of Pharmaceutical Compounding (1999); Pickar, Dosage Calculations (1999); and Remington: The Science and Practice of Pharmacy, 20th Edition, 2003, Gennaro Ed., Lippincott, Williams & Wilkins).

The term "AUC" (area under the curve) refers to the total amount of a drug absorbed by or exposed to a subject, and may also refer to partial AUC at particular time intervals. AUC may be obtained based on a curve of the concentration of a drug in a subject changing over time.

The term "first-line treatment" refers to the first treatment given for a certain disorder or disease, which is usually part of a set of standard treatment methods, such as chemotherapy, radiotherapy, and/or immunotherapy after surgery, and is also known as the initial treatment, etc. Generally, surgical resection of a malignant tumor is not counted as a line of treatment. The use of a first-line treatment alone is recognized as the best therapy for the disorder or disease. If it cannot cure the disorder or disease (e.g., the treatment fails or stops working) or causes serious side effects, other treatment methods can be added or used.

Accordingly, the term "second-line treatment" refers to a treatment given when the first-line treatment is unsuccessful, the term "third-line treatment" refers to a treatment or treatment regimen given when the first-line treatment and the subsequent second-line treatment are both unsuccessful, and the term "multi-line treatment" refers to a second-line or higher-line treatment.

Various aspects of the present disclosure are described in more detail in the following subsections.

## II. Antibody

The applicant has developed a bispecific antibody specifically binding to 4-1BB and PD-L1 on the basis of its 4-1BB antibody platform "X-Body". The 4-1BB antibody platform "X-Body" and antibodies suitable for the present disclosure developed based on the platform are disclosed as in PCT/CN2020/119388.

## Bispecific Antibody

An exemplary bispecific antibody of the present disclosure comprises a first antigen-binding region specifically binding to 4-1BB and a second antigen-binding region specifically binding to PD-L1. Bispecific antibodies may have several structural forms (see, e.g., Aran F. Labrijn et al., Bispecific antibodies: a mechanistic review of the pipeline, Nature Reviews Drug Discovery, volume 18, pages 585-608 (2019)). For example, bispecific antibodies may be IgG-like bispecific antibodies, i.e., full-length bispecific antibodies, or non-IgG-like bispecific antibodies, which are not full-length antibody constructs.

The bispecific antibody of the present disclosure may comprise one or two or more first antigen-binding regions and/or one or two or more second antigen-binding regions.

In one embodiment, the first antigen-binding region is from or derived from an anti-4-1BB antibody or antigen-binding fragment, e.g., from or derived from an anti-4-1BB antibody or antigen-binding fragment in PCT/CN2020/119388.

In one aspect, the first antigen-binding region comprises a first heavy chain variable region and/or a first light chain variable region.

In another embodiment, the first antigen-binding region may bind to human 4-1BB. In one specific embodiment, the first antigen-binding region comprises the $V_H$ and/or $V_L$ of the anti-4-1BB antibody or antigen-binding fragment described above. In one embodiment, the first heavy chain variable region in the first antigen-binding region comprises the $V_H$ of the anti-4-1BB antibody or antigen-binding fragment described above or consists of the $V_H$, and/or the first light chain variable region in the first antigen-binding region comprises the $V_L$ of the anti-4-1BB antibody or antigen-binding fragment described above or consists of the $V_L$. In another embodiment, the first antigen-binding region further comprises a constant region. In another embodiment, the constant region in the first antigen-binding region comprises a first heavy chain constant region and/or a first light chain constant region. In another embodiment, the first antigen-binding region further comprises a constant region of the anti-4-1BB antibody or antibody-binding fragment described above, e.g., the heavy chain constant region and/or the light chain constant region of the anti-4-1BB antibody or antibody-binding fragment described above. In one embodiment, the first antigen-binding region comprises the scFv of the anti-4-1BB antibody described above.

In one aspect, the first heavy chain variable region in the first antigen-binding region comprises three heavy chain variable regions (HCDRs): an HCDR1, an HCDR2, and/or an HCDR3. In one embodiment, the three heavy chain variable regions (HCDRs), the HCDR1, the HCDR2, and the HCDR3, are or comprise the three heavy chain variable regions (HCDRs), the HCDR1, the HCDR2, and the HCDR3, of the anti-4-1BB antibody or antigen-binding fragment described above, respectively.

In another aspect, the first light chain variable region in the first antigen-binding region comprises three light chain variable regions (LCDRs): an LCDR1, an LCDR2, and/or an LCDR3. In one embodiment, the three light chain variable regions (LCDRs), the LCDR1, the LCDR2, and the LCDR3, are or comprise the three light chain variable regions (LCDRs), the LCDR1, the LCDR2, and the LCDR3, of the anti-4-1BB antibody or antigen-binding fragment described above, respectively.

In yet another aspect, the first heavy chain variable region in the first antigen-binding region comprises the three heavy chain variable regions, the HCDR1, the HCDR2, and/or the HCDR3, of the anti-4-1BB antibody or antigen-binding fragment described above, and the first light chain variable region in the first antigen-binding region comprises the three light chain variable regions, the LCDR1, the LCDR2, and/or the LCDR3, of the anti-4-1BB antibody or antigen-binding fragment described above.

In one specific embodiment, the first heavy chain variable region in the first antigen-binding region

(i) comprises or consists of an amino acid sequence having at least 80%, 81%, 82%, 83%, 84%, 85%, 86%, 87%, 88%, 89%, 90%, 91%, 92%, 93%, 94%, 95%, 96%, 97%, 98%, or 99% identity to the amino acid sequence of SEQ ID NO: 7 or SEQ ID NO: 9; or

(ii) comprises or consists of the amino acid sequence of SEQ ID NO: 7 or SEQ ID NO: 9; or

(iii) comprises or consists of an amino acid sequence having one or more (preferably no more than 10, and more preferably no more than 5) amino acid modifications (preferably amino acid substitutions, and more preferably conservative substitutions) compared to the amino acid sequence of SEQ ID NO: 7 or SEQ ID NO: 9, wherein preferably, the amino acid modifications do not occur in a CDR; more preferably, the amino acid modifications occur in an FR, for example, in an FR1, an FR2, an FR3, or an FR4.

In one specific embodiment, the HCDR1, the HCDR2, and the HCDR3 of the first heavy chain variable region in the first antigen-binding region are:

(i) an HCDR1, an HCDR2, and an HCDR3 derived from a heavy chain variable region consisting of the sequence set forth in SEQ ID NO: 7 or SEQ ID NO: 9; or

(ii) the HCDR1, the HCDR2, and the HCDR3 of (i), which further comprise at least one and no more than 5 amino acid modifications (preferably amino acid substitutions, and preferably conservative substitutions) in total compared to the three HCDRs of (i).

In one specific embodiment, the HCDR1 of the first heavy chain variable region in the first antigen-binding region comprises or consists of the amino acid sequence of SEQ ID NO: 1, or the HCDR1 comprises or consists of an amino acid sequence having one, two, or three modifications (preferably amino acid substitutions, and preferably conservative substitutions) compared to the amino acid sequence of SEQ ID NO: 1.

In one specific embodiment, the HCDR2 of the first heavy chain variable region in the first antigen-binding region comprises or consists of the amino acid sequence of SEQ ID NO: 2, or the HCDR2 comprises or consists of an amino acid sequence having one, two, or three modifications (preferably amino acid substitutions, and preferably conservative substitutions) compared to the amino acid sequence of SEQ ID NO: 2.

In one specific embodiment, the HCDR3 of the first heavy chain variable region in the first antigen-binding region comprises or consists of the amino acid sequence of SEQ ID NO: 3, or the HCDR3 comprises or consists of an amino acid sequence having one, two, or three modifications (preferably amino acid substitutions, and preferably conservative substitutions) compared to the amino acid sequence of SEQ ID NO: 3.

In one embodiment, the HCDR1, the HCDR2, and the HCDR3 of the first heavy chain variable region in the first antigen-binding region are:

(i) an HCDR1, an HCDR2, and an HCDR3 consisting of the sequences set forth in SEQ ID NO: 1, SEQ ID NO: 2, and SEQ ID NO: 3, respectively, or

(ii) the HCDR1, the HCDR2, and the HCDR3 of (i), which further comprise at least one and no more than 5 amino acid modifications (preferably amino acid substitutions, and preferably conservative substitutions) in total compared to the three HCDRs of (i).

In another embodiment, the first light chain variable region in the first antigen-binding region

(i) comprises or consists of an amino acid sequence having at least 80%, 81%, 82%, 83%, 84%, 85%, 86%, 87%, 88%, 89%, 90%, 91%, 92%, 93%, 94%, 95%, 96%, 97%, 98%, or 99% identity to the amino acid sequence of SEQ ID NO: 8 or SEQ ID NO: 10; or

(ii) comprises or consists of the amino acid sequence of SEQ ID NO: 8 or SEQ ID NO: 10; or
(iii) comprises or consists of an amino acid sequence having one or more (preferably no more than 10, and more preferably no more than 5) amino acid modifications (preferably amino acid substitutions, and more preferably conservative substitutions) compared to the amino acid sequence of SEQ ID NO: 8 or SEQ ID NO: 10, wherein preferably, the amino acid modifications do not occur in a CDR; more preferably, the amino acid modifications occur in an FR, for example, in an FR1, an FR2, an FR3, or an FR4;

in SEQ ID NO: 8, X = S or G.

In one embodiment, the LCDR1, the LCDR2, and the LCDR3 of the first light chain variable region in the first antigen-binding region are:

(i) an LCDR1, an LCDR2, and an LCDR3 derived from a light chain variable region consisting of the sequence set forth in SEQ ID NO: 8 or SEQ ID NO: 10, or
(ii) the LCDR1, the LCDR2, and the LCDR3 of (i), which further comprise at least one and no more than 5 amino acid modifications (preferably amino acid substitutions, and preferably conservative substitutions) in total compared to the three LCDRs of (i);

in SEQ ID NO: 8, X = S or G.

In one specific embodiment, the LCDR1 of the first light chain variable region in the first antigen-binding region comprises or consists of the amino acid sequence of SEQ ID NO: 4, or the LCDR1 comprises or consists of an amino acid sequence having one, two, or three modifications (preferably amino acid substitutions, and preferably conservative substitutions) compared to the amino acid sequence of SEQ ID NO: 4.

In one specific embodiment, the LCDR2 of the first light chain variable region in the first antigen-binding region comprises or consists of the amino acid sequence of SEQ ID NO: 5, or the LCDR2 comprises or consists of an amino acid sequence having one, two, or three modifications (preferably amino acid substitutions, and preferably conservative substitutions) compared to the amino acid sequence of SEQ ID NO: 5.

In one specific embodiment, the LCDR3 of the first light chain variable region in the first antigen-binding region comprises or consists of the amino acid sequence of SEQ ID NO: 6, or the LCDR3 comprises or consists of an amino acid sequence having one, two, or three modifications (preferably amino acid substitutions, and preferably conservative substitutions) compared to the amino acid sequence of SEQ ID NO: 6.

In one embodiment, the LCDR1, the LCDR2, and the LCDR3 of the first light chain variable region in the first antigen-binding region are:

(i) an LCDR1, an LCDR2, and an LCDR3 consisting of the sequences set forth in SEQ ID NO: 4, SEQ ID NO: 5, and SEQ ID NO: 6, respectively, or
(ii) the LCDR1, the LCDR2, and the LCDR3 of (i), which further comprise at least one and no more than 5 amino acid modifications (preferably amino acid substitutions, and preferably conservative substitutions) in total compared to the three LCDRs of (i).

In another embodiment, the first antigen-binding region comprises: a first heavy chain variable region comprising an HCDR1, an HCDR2, and an HCDR3 consisting of the sequences set forth in SEQ ID NO: 1, SEQ ID NO: 2, and SEQ ID NO: 3, and a first light chain variable region comprising an HCDR1, an HCDR2, and an HCDR3 consisting of the sequences set forth in SEQ ID NO: 4, SEQ ID NO: 5, and SEQ ID NO: 6.

In another embodiment, the first antigen-binding region comprises:

(i) a first heavy chain variable region comprising or consisting of the amino acid sequence set forth in SEQ ID NO: 7, and a first light chain variable region comprising or consisting of the amino acid sequence set forth in SEQ ID NO: 8 (wherein X = S or G); or
(ii) a first heavy chain variable region comprising or consisting of the amino acid sequence set forth in SEQ ID NO: 9, and a first light chain variable region comprising or consisting of the amino acid sequence set forth in SEQ ID NO: 10.

In yet another aspect, the first antigen-binding region optionally further comprises a constant region, and the constant region in the first antigen-binding region comprises a first heavy chain constant region and/or a first light chain constant region.

In one embodiment, the first heavy chain constant region is or is derived from a human IgG constant region, e.g., an IgG1, IgG2, IgG3, or IgG4 constant region, preferably an IgG1 constant region, an IgG2 constant region, or an IgG4 constant region.

In another embodiment, the first heavy chain constant region

(i) comprises or consists of an amino acid sequence having at least 80%, 85%, 90%, 91%, 92%, 93%, 94%, 95%, 96%, 97%, 98%, or 99% identity to the amino acid sequence set forth in SEQ ID NO: 19 or SEQ ID NO: 20; or
(ii) comprises or consists of the amino acid sequence set forth in SEQ ID NO: 19 or SEQ ID NO: 20; or
(iii) comprises or consists of an amino acid sequence having one or more (preferably no more than 10, and more preferably no more than 5) amino acid modifications (preferably amino acid substitutions, and more preferably conservative substitutions) compared to the amino acid sequence set forth in SEQ ID NO: 19 or SEQ ID NO: 20.

In one embodiment, the first light chain constant region is or is derived from a $\kappa$ or $\lambda$ light chain constant region, e.g., a human $\kappa$ or $\lambda$ light chain constant region, e.g., a human $\lambda$ light chain constant region.

In another embodiment, the first light chain constant region

(i) comprises or consists of an amino acid sequence having at least 80%, 85%, 90%, 91%, 92%, 93%, 94%, 95%, 96%, 97%, 98%, or 99% identity to the amino acid sequence set forth in SEQ ID NO: 21 or SEQ ID NO: 22; or
(ii) comprises or consists of the amino acid sequence set forth in SEQ ID NO: 21 or SEQ ID NO: 22; or
(iii) comprises or consists of an amino acid sequence having one or more (preferably no more than 10, and more preferably no more than 5) amino acid modifications (preferably amino acid substitutions, and more preferably conservative substitutions) compared to the amino acid sequence set forth in SEQ ID NO: 21 or SEQ ID NO: 22.

In one embodiment, the constant regions of the antibody may be mutated to improve the preparation and purification of the antibody. For example, the IgG4 constant region may have a mutation of S228P (EU numbering). The IgG1 constant region may have mutations of L234A, L235A, D265A, and P329A (EU numbering).

In one embodiment, the first antigen-binding region is an scFv fragment. In another embodiment, the scFv fragment comprises the HCDR1, the HCDR2, and/or the HCDR3 of the heavy chain variable region and/or the LCDR1, the LCDR2, and/or the LCDR3 of the light chain variable region of the anti-4-1BB antibody or antibody-binding fragment described above in the present disclosure. For example, the scFv fragment comprises a first light chain variable region and a first heavy chain variable region that are linked by a linker (or optionally without a linker).

In one embodiment, the linker is a flexible linker, such as a linker having a separate glycine residue and/or a separate serine residue or a combination thereof. In one embodiment, the linker comprises an amino acid sequence of (Gly4Ser)n or (GlySer4)n, wherein n is a positive integer equal to or greater than 1; for example, n is a positive integer from 1 to 7; for example, n is 2, 3, 4, 5, or 6. In one embodiment, n is 1, 2, 3, or 4. In one specific embodiment, the linker comprises or consists of the amino acid sequence set forth in SEQ ID NO: 47 or SEQ ID NO: 24 or SEQ ID NO: 23 ((GGGGS)n, wherein n is 1, 2, 3, 4, 5, or 6).

In another embodiment, the second antigen-binding region is from or derived from an anti-PD-L1 antibody or an antigen-binding fragment thereof, e.g., an anti-PD-L1 antibody or an antigen-binding fragment thereof as disclosed in CN109021107A.

In another aspect, the second antigen-binding region comprises a second heavy chain variable region and/or a second light chain variable region.

In another embodiment, the second antigen-binding region may bind to human PD-L1. In one specific embodiment, the second antigen-binding region comprises the $V_H$ and/or $V_L$ of the anti-PD-L1 antibody or antigen-binding fragment described above. In one embodiment, the second heavy chain variable region in the second antigen-binding region comprises or consists of the $V_H$ of the anti-PD-L1 antibody or antigen-binding fragment described above, and/or the second light chain variable region in the second antigen-binding region comprises or consists of the $V_L$ of the anti-PD-L1 antibody or antigen-binding fragment described above. In another embodiment, the second antigen-binding region further comprises a constant region. In another embodiment, the constant region in the second antigen-binding region comprises a second heavy chain constant region and/or a second light chain constant region. In another embodiment, the second antigen-binding region further comprises a constant region of the anti-PD-L1 antibody or antibody-binding fragment described above, e.g., the heavy chain constant region or the light chain constant region of the anti-PD-L1 antibody or antibody-binding fragment described above.

In yet another aspect, the second heavy chain variable region in the second antigen-binding region comprises three heavy chain variable regions (HCDRs): an HCDR1, an HCDR2, and/or an HCDR3. In one embodiment, the three heavy chain variable regions (HCDRs), the HCDR1, the HCDR2, and the HCDR3, are or comprise the three heavy chain variable regions (HCDRs), the HCDR1, the HCDR2, and/or the HCDR3, of the anti-PD-L1 antibody or antigen-binding fragment described above, respectively.

In another aspect, the second light chain variable region in the second antigen-binding region comprises three light chain variable regions (LCDRs): an LCDR1, an LCDR2, and/or an LCDR3. In one embodiment, the three light chain variable regions (LCDRs), the LCDR1, the LCDR2, and the LCDR3, are or comprise the three light chain variable regions (LCDRs), the LCDR1, the LCDR2, and the LCDR3, of the anti-PD-L1 antibody or antigen-binding fragment described above, respectively.

In yet another aspect, the second heavy chain variable region in the second antigen-binding region comprises the three heavy chain variable regions, the HCDR1, the HCDR2, and/or the HCDR3, of the anti-PD-L1 antibody or antigen-binding fragment described above, and the second light chain variable region in the second antigen-binding region comprises the three light chain variable regions, the LCDR1, the LCDR2, and/or the LCDR3, of the anti-PD-L1 antibody or antigen-binding fragment described above.

In one specific embodiment, the second heavy chain variable region in the second antigen-binding region

(i) comprises or consists of an amino acid sequence having at least 90%, 91%, 92%, 93%, 94%, 95%, 96%, 97%, 98%, or 99% identity to the amino acid sequence of SEQ ID NO: 17; or
(ii) comprises or consists of the amino acid sequence of SEQ ID NO: 17; or
(iii) comprises or consists of an amino acid sequence having one or more (preferably no more than 10, and more preferably no more than 5) amino acid modifications (preferably amino acid substitutions, and more preferably conservative substitutions) compared to the amino acid sequence of SEQ ID NO: 17, wherein preferably, the amino acid modifications do not occur in a CDR; more preferably, the amino acid modifications occur in an FR, for example, in an FR1, an FR2, an FR3, or an FR4.

In one specific embodiment, the HCDR1, the HCDR2, and the HCDR3 of the second heavy chain variable region in the second antigen-binding region are:

(i) an HCDR1, an HCDR2, and an HCDR3 derived from a heavy chain variable region comprising or consisting of the sequence set forth in SEQ ID NO: 17, or
(ii) the HCDR1, the HCDR2, and the HCDR3 of (i), which further comprise at least one and no more than 5 amino acid modifications (preferably amino acid substitutions, and preferably conservative substitutions) in total compared to the three HCDRs of (i).

In one specific embodiment, the HCDR1 of the second heavy chain variable region in the second antigen-binding region comprises or consists of the amino acid sequence of SEQ ID NO: 11, or the HCDR1 comprises or consists of an amino acid sequence having one, two, or three modifications (preferably amino acid substitutions, and preferably conservative substitutions) compared to the amino acid sequence of SEQ ID NO: 11.

In one specific embodiment, the HCDR2 of the second heavy chain variable region in the second antigen-binding region comprises or consists of the amino acid sequence of SEQ ID NO: 12, or the HCDR2 comprises or consists of an amino acid sequence having one, two, or three modifications (preferably amino acid substitutions, and preferably conservative substitutions) compared to the amino acid sequence of SEQ ID NO: 12.

In one specific embodiment, the HCDR3 of the second heavy chain variable region in the second antigen-binding region comprises or consists of the amino acid sequence of SEQ ID NO: 13, or the HCDR3 comprises or consists of an amino acid sequence having one, two, or three modifications (preferably amino acid substitutions, and preferably conservative substitutions) compared to the amino acid sequence of SEQ ID NO: 13.

In one embodiment, the HCDR1, the HCDR2, and the HCDR3 of the second heavy chain variable region in the second antigen-binding region are:

(i) an HCDR1, an HCDR2, and an HCDR3 consisting of the sequences set forth in SEQ ID NO: 11, SEQ ID NO: 12, and SEQ ID NO: 13, respectively, or
(ii) the HCDR1, the HCDR2, and the HCDR3 of (i), which further comprise at least one and no more than 5 amino acid modifications (preferably amino acid substitutions, and preferably conservative substitutions) in total compared to the three HCDRs of (i).

In one specific embodiment, the second light chain variable region in the second antigen-binding region

(i) comprises or consists of an amino acid sequence having at least 90%, 91%, 92%, 93%, 94%, 95%, 96%, 97%, 98%, or 99% identity to the amino acid sequence of SEQ ID NO: 18; or
(ii) comprises or consists of the amino acid sequence of SEQ ID NO: 18; or
(iii) comprises or consists of an amino acid sequence having one or more (preferably no more than 10, and more preferably no more than 5) amino acid modifications (preferably amino acid substitutions, and more preferably conservative substitutions) compared to the amino acid sequence of SEQ ID NO: 18, wherein preferably, the amino acid modifications do not occur in a CDR; more preferably, the amino acid modifications occur in an FR, for example, in an FR1, an FR2, an FR3, or an FR4.

In one specific embodiment, the LCDR1, the LCDR2, and the LCDR3 of the second light chain variable region in the second antigen-binding region are:

(i) an LCDR1, an LCDR2, and an LCDR3 derived from a light chain variable region comprising or consisting of the sequence set forth in SEQ ID NO: 18, or

(ii) the LCDR1, the LCDR2, and the LCDR3 of (i), which further comprise at least one and no more than 5 amino acid modifications (preferably amino acid substitutions, and preferably conservative substitutions) in total compared to the three LCDRs of (i).

In one specific embodiment, the LCDR1 of the second light chain variable region in the second antigen-binding region comprises or consists of the amino acid sequence of SEQ ID NO: 14, or the LCDR1 comprises or consists of an amino acid sequence having one, two, or three modifications (preferably amino acid substitutions, and preferably conservative substitutions) compared to the amino acid sequence of SEQ ID NO: 14.

In one specific embodiment, the LCDR2 of the second light chain variable region in the second antigen-binding region comprises or consists of the amino acid sequence of SEQ ID NO: 15, or the LCDR2 comprises or consists of an amino acid sequence having one, two, or three modifications (preferably amino acid substitutions, and preferably conservative substitutions) compared to the amino acid sequence of SEQ ID NO: 15.

In one specific embodiment, the LCDR3 of the second light chain variable region in the second antigen-binding region comprises or consists of the amino acid sequence of SEQ ID NO: 16, or the LCDR3 comprises or consists of an amino acid sequence having one, two, or three modifications (preferably amino acid substitutions, and preferably conservative substitutions) compared to the amino acid sequence of SEQ ID NO: 16.

In one embodiment, the three light chain variable regions, the LCDR1, the LCDR2, and the LCDR3, of the second light chain variable region in the second antigen-binding region are:

(i) an LCDR1, an LCDR2, and an LCDR3 consisting of the sequences set forth in SEQ ID NO: 14, SEQ ID NO: 15, and SEQ ID NO: 16, respectively, or

(ii) the LCDR1, the LCDR2, and the LCDR3 of (i), which further comprise at least one and no more than 5 amino acid modifications (preferably amino acid substitutions, and preferably conservative substitutions) in total compared to the three LCDRs of (i).

In another embodiment, the second antigen-binding region comprises: a second heavy chain variable region comprising an HCDR1, an HCDR2, and an HCDR3 consisting of the sequences set forth in SEQ ID NO: 11, SEQ ID NO: 12, and SEQ ID NO: 13, and a second light chain variable region comprising an HCDR1, an HCDR2, and an HCDR3 consisting of the sequences set forth in SEQ ID NO: 14, SEQ ID NO: 15, and SEQ ID NO: 16.

In another embodiment, the second antigen-binding region comprises: a second heavy chain variable region comprising or consisting of the amino acid sequence set forth in SEQ ID NO: 17, and a second light chain variable region comprising or consisting of the amino acid sequence set forth in SEQ ID NO: 18.

In yet another aspect, the second antigen-binding region optionally further comprises a constant region, and the constant region in the second antigen-binding region comprises a second heavy chain constant region and/or a second light chain constant region.

In one embodiment, the second heavy chain constant region is or is derived from a human IgG constant region, e.g., an IgG1, IgG2, IgG3, or IgG4 constant region, preferably an IgG1 constant region, an IgG2 constant region, or an IgG4 constant region.

In another embodiment, the second heavy chain constant region

(i) comprises or consists of an amino acid sequence having at least 80%, 85%, 90%, 91%, 92%, 93%, 94%, 95%, 96%, 97%, 98%, or 99% identity to the amino acid sequence set forth in SEQ ID NO: 19 or SEQ ID NO: 20; or

(ii) comprises or consists of the amino acid sequence set forth in SEQ ID NO: 19 or SEQ ID NO: 20; or

(iii) comprises or consists of an amino acid sequence having one or more (preferably no more than 10, and more preferably no more than 5) amino acid modifications (preferably amino acid substitutions, and more preferably conservative substitutions) compared to the amino acid sequence set forth in SEQ ID NO: 19 or SEQ ID NO: 20.

In one embodiment, the second light chain constant region is or is derived from a κ or λ light chain constant region, e.g., a human κ or λ light chain constant region, preferably a human κ light chain constant region.

In another embodiment, the second light chain constant region

(i) comprises or consists of an amino acid sequence having at least 80%, 85%, 90%, 91%, 92%, 93%, 94%, 95%, 96%, 97%, 98%, or 99% identity to the amino acid sequence set forth in SEQ ID NO: 21 or SEQ ID NO: 22; or

(ii) comprises or consists of the amino acid sequence set forth in SEQ ID NO: 21 or SEQ ID NO: 22; or

(iii) comprises or consists of an amino acid sequence having one or more (preferably no more than 10, and more preferably no more than 5) amino acid modifications (preferably amino acid substitutions, and more preferably conservative substitutions) compared to the amino acid sequence set forth in SEQ ID NO: 21 or SEQ ID NO: 22.

It is known that the $V_H$ and/or the $V_L$ of the bispecific antibody may differ in one or several amino acids from the $V_H$ and/or the $V_L$ of the monoclonal antibody from which they are derived for the construction of the bispecific antibody. For example, the derived $V_H$ and $V_L$ may have one or more mutations to make the scFv composed thereof more stable. In one embodiment, the $V_H$ and/or the $V_L$ may have a mutation(s), so that a disulfide bond is formed therebetween, thereby making the antibody or an antigen-binding portion thereof more stable. For example, a mutation(s) may be made to form a disulfide bond between the first heavy chain variable region and the first light chain variable region. For example, the mutation in the first heavy chain variable region is G44C (Eu numbering), and/or the mutation in the first light chain variable region is T104C (Eu numbering).

In one specific embodiment, the bispecific antibody comprises the following chains:

(1) a chain 1: the heavy chain of the second antigen-binding region that is linked to an antigen-binding portion in the first antigen-binding region at the C-terminus by a linker or by no linker; and
(2) a chain 2: the light chain of the second antigen-binding region.

In one specific embodiment, the bispecific antibody comprises two chains 1 and two chains 2 that are linked by disulfide bonds.

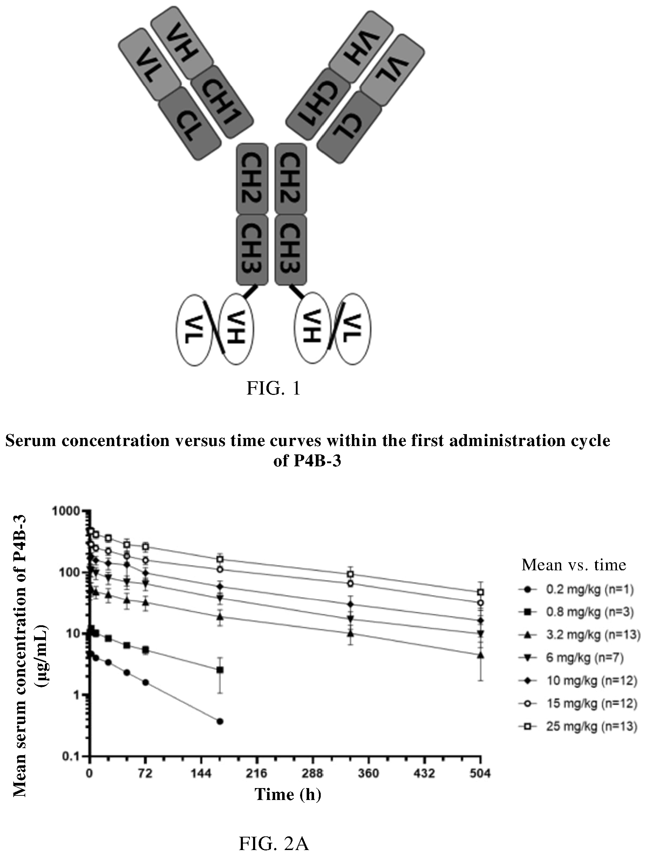

In another specific embodiment, the structure of the bispecific antibody is shown in FIG. 1.

In one embodiment, the antigen-binding portion in the first antigen-binding region is selected from: (i) a Fab fragment; (ii) a F(ab')2 fragment; (iii) an Fd fragment; (iv) an Fv fragment; (v) a dAb fragment; (vi) a nanobody; and (vii) a single-chain Fv (scFv); the scFv comprises the first heavy chain variable region and the first light chain variable region.

In one embodiment, in the method to which the present disclosure relates, the first antigen-binding region and/or the second antigen-binding region is a mouse antibody, a human antibody, a chimeric antibody, or a humanized antibody.

In one specific embodiment, the heavy chain of the second antigen-binding region comprises the second heavy chain variable region. In another embodiment, the heavy chain of the second antigen-binding region comprises the HCDR1, the HCDR2, and/or the HCDR3 of the second heavy chain variable region. In another specific embodiment, the light chain of the second antigen-binding region comprises the second light chain variable region. In another embodiment, the light chain of the second antigen-binding region comprises the LCDR1, the LCDR2, and/or the LCDR3 of the second heavy chain variable region.

In one specific embodiment, the heavy chain of the second antigen-binding region further comprises the second heavy chain constant region.

In one specific embodiment, the light chain of the second antigen-binding region further comprises the second light chain constant region.

In one embodiment, the linker in the bispecific antibody of the present disclosure is a flexible linker, such as a linker having a separate glycine residue and/or a separate serine residue or a combination thereof. In one embodiment, the linker comprises an amino acid sequence of (Gly4Ser)n or (GlySer4)n, wherein n is a positive integer equal to or greater than 1; for example, n is a positive integer from 1 to 7; for example, n is 2, 3, 4, 5, or 6. In one embodiment, n is 1, 2, 3, or 4, preferably 1 or 3. In one specific embodiment, the linker of the bispecific antibody comprises or consists of the amino acid sequence set forth in SEQ ID NO: 47, SEQ ID NO: 24, or SEQ ID NO: 23. In one preferred embodiment, the linker of the bispecific antibody comprises or consists of the amino acid sequence set forth in SEQ ID NO: 23, wherein n = 1 or 3.

In one embodiment, the chain 1 of the bispecific antibody

(i) comprises or consists of an amino acid sequence having at least 85%, 86%, 87%, 88%, 89%, 90%, 91%, 92%, 93%, 94%, 95%, 96%, 97%, 98%, or 99% identity to the amino acid sequence set forth in SEQ ID NO: 25; or
(ii) comprises or consists of the amino acid sequence set forth in SEQ ID NO: 25; or
(iii) comprises or consists of an amino acid sequence having one or more (preferably no more than 20, and more preferably no more than 10 or 5) amino acid modifications (preferably amino acid substitutions, and more preferably conservative substitutions) compared to the amino acid sequence set forth in SEQ ID NO: 25.

In one embodiment, the chain 2 of the bispecific antibody

(i) comprises or consists of an amino acid sequence having at least 90%, 91%, 92%, 93%, 94%, 95%, 96%, 97%, 98%, or 99% identity to the amino acid sequence set forth in SEQ ID NO: 26; or
(ii) comprises or consists of the amino acid sequence set forth in SEQ ID NO: 26; or
(iii) comprises or consists of an amino acid sequence having one or more (preferably no more than 20, and more preferably no more than 10 or 5) amino acid modifications (preferably amino acid substitutions, and more preferably conservative substitutions) compared to the amino acid sequence set forth in SEQ ID NO: 26.

### _Conservative Modification_

In another embodiment, the bispecific antibody of the present disclosure comprises a first antigen-binding region and a second antigen-binding region that differ from those first antigen-binding regions and second antigen-binding regions described above by having one or more conservative modifications. It is understood in the art that certain conservative sequence modifications can be made, and the modifications do not remove antigen binding. See, e.g., Brummell et al. (1993) Biochem 32:1180-8; de Wildt et al. (1997) Prot. Eng. 10:835-41; Komissarov et al. (1997) J. Biol. Chem. 272:26864-26870; Hall et al. (1992) J. Immunol. 149:1605-12; Kelley and O'Connell (1993) Biochem. 32:6862-35; Adib-Conquy et al. (1998) Int. Immunol. 10:341-6; and Beers et al. (2000) Clin. Can. Res. 6:2835-43.

Thus, in one embodiment, the bispecific antibody of the present disclosure comprises a first heavy chain variable region sequence and/or a first light chain variable region sequence that differ(s) from those first heavy chain variable region sequences and/or first light chain variable region sequences described above by having one or more conservative modifications, wherein preferably, the modifications do not occur in a CDR; preferably, the modifications occur in an FR.

In some embodiments, a modification(s) may be introduced into the heavy chain variable region and/or the light chain variable region, so that a disulfide bond is formed therebetween, thereby improving the stability of the scFv from which a bispecific antibody can be constructed.

In some embodiments, the modification(s) is/are a substitution(s), an addition(s), and/or a deletion(s).

In some embodiments, the substitution(s) is/are a conservative substitution(s). A conservative substitution refers to the substitution of an amino acid with another amino acid of the same class, for example, the substitution of an acidic amino acid with another acidic amino acid, the substitution of a basic amino acid with another basic amino acid, or the substitution of a neutral amino acid with another neutral amino acid. Exemplary substitutions are shown in the table below:

| Original residue | Conservative substitution(s) | Preferred conservative substitution |
|---|---|---|
| Ala(A) | Val, Leu, Ile | Val |
| Arg(R) | Lys, Gln, Asn | Lys |
| Asn(N) | Gln, His, Asp, Lys, Arg | Gln |
| Asp(D) | Glu, Asn | Glu |
| Cys(C) | Ser, Ala | Ser |
| Gln(Q) | Asn, Glu | Asn |
| Glu(E) | Asp, Gln | Asp |
| Gly(G) | Ala | Ala |
| His(H) | Asn, Gln, Lys, Arg | Arg |
| Ile(I) | Leu, Val, Met, Ala, Phe, norleucine | Leu |
| Leu(L) | Norleucine, Ile, Val, Met, Ala, Phe | Ile |
| Lys(K) | Arg, Gln, Asn | Arg |
| Met(M) | Leu, Phe, Ile | Leu |
| Phe(F) | Trp, Leu, Val, Ile, Ala, Tyr | Tyr |
| Pro(P) | Ala | Ala |
| Ser(S) | Thr | Thr |
| Thr(T) | Val, Ser | Ser |
| Trp(W) | Tyr, Phe | Tyr |
| Tyr(Y) | Trp, Phe, Thr, Ser | Phe |
| Val(V) | Ile, Leu, Met, Phe, Ala, norleucine | Leu |

In various embodiments, the antibody may be, for example, a mouse antibody, a human antibody, a humanized antibody, or a chimeric antibody.

The term "conservative sequence modification" as used herein is intended to refer to an amino acid modification that does not significantly affect or alter the binding properties of an antibody comprising the amino acid sequence. Such conservative modifications include amino acid substitutions, additions, and deletions. Modifications can be introduced into the antibody of the present disclosure by standard techniques known in the art, such as site-directed mutagenesis and PCR-mediated mutagenesis. A conservative amino acid substitution is an amino acid substitution in which the amino acid residue is substituted with an amino acid residue having a similar side chain. Families of amino acid residues having similar side chains have been defined in the art. These families include amino acids having basic side chains (e.g., lysine, arginine, and histidine), amino acids having acidic side chains (e.g., aspartic acid and glutamic acid), amino acids having uncharged polar side chains (e.g., glycine, asparagine, glutamine, serine, threonine, tyrosine, cysteine, and tryptophan), amino acids having nonpolar side chains (e.g., alanine, valine, leucine, isoleucine, proline, phenylalanine, and methionine), amino acids having β-branched side chains (e.g., threonine, valine, and isoleucine), and amino acids having aromatic side chains (e.g., tyrosine, phenylalanine, tryptophan, and histidine). Thus, one or more amino acid residues within the CDRs of the antibody of the present disclosure may be substituted with other amino acid residues from the same side chain family, and the retained functions (i.e., the functions described above) of the altered antibody can be tested using the functional assays described herein.

Engineered and Modified Antibody

The modified antibody may be engineered using an antibody having one or more of the $V_H$ sequences/$V_L$ sequences of the first antigen-binding region and the second antigen-binding region in the bispecific antibody of the present disclosure as a starting material to prepare the antibody of the present disclosure. The antibody may be engineered by modifying one or more residues within one or two variable regions (i.e., $V_H$ and/or $V_L$), for example, within one or more CDRs and/or within one or more framework regions. Additionally or optionally, the antibody may be engineered by modifying residues within one or more constant regions, for example, to alter one or more effector functions of the antibody.

In certain embodiments, CDR grafting may be used to engineer the variable regions of the antibody. Antibodies interact with target antigens mainly through amino acid residues located in heavy and light chain complementarity determining regions (CDRs).

For this reason, amino acid sequences within CDRs are more diverse between individual antibodies than sequences outside CDRs. Since CDR sequences are responsible for most antibody-antigen interactions, it is possible to express recombinant antibodies that mimic the properties of specific naturally occurring antibodies by constructing expression vectors that include CDR sequences from the specific naturally occurring antibody grafted onto framework sequences from a different antibody with different properties (see, e.g., Riechmann et al. (1998) Nature 332:323-327; Jones et al. (1986) Nature 321:522-525; Queen et al. (1989) Proc. Natl. Acad. See. U.S.A. 86:10029-10033; and U.S. Patent Nos. 5,225,539; 5,530,101; 5,585,089; 5,693,762; and 6,180,370).

Thus, another embodiment of the present disclosure relates to an isolated monoclonal antibody or an antigen-binding portion thereof, wherein the antibody or the antigen-binding portion thereof comprises a heavy chain variable region and/or a light chain variable region; the heavy chain variable region comprises an HCDR1 sequence, an HCDR2 sequence, and an HCDR3 sequence that have the sequences of the present disclosure described above, and the light chain variable region comprises an LCDR1 sequence, an LCDR2 sequence, and an LCDR3 sequence that have the sequences of the present disclosure described above. Although these antibodies comprise the $V_H$ and $V_L$ CDR sequences of the monoclonal antibodies of the present disclosure, they may comprise different framework sequences.

Such framework sequences can be obtained from public DNA databases or published references that include germline antibody gene sequences. For example, germline DNA sequences for human heavy and light chain variable region genes can be found in the "VBase" human germline sequence database (available on the Internet at www.mrc-cpe.cam.ac.uk/vbase), as well as in Kabat et al. (1991) (cited above); Tomlinson et al. (1992), J. Mol. Biol. 227:776-798; and Cox et al. (1994), Eur. J. Immunol. 24:827-836, the contents of each of which are expressly incorporated herein by reference. As another example, germline DNA sequences for human heavy and light chain variable region genes can be found in gene bank databases. For example, the following heavy chain germline sequences found in HCo7 HuMAb mice are available under the attached gene bank accession numbers: 1-69 (NG--0010109, NT--024637, and BC070333), 3-33 (NG--0010109 and NT--024637), and 3-7 (NG--0010109 and NT--024637). As another example, the following heavy chain germline sequences found in HCo12 HuMAb mice are available under the attached gene bank accession numbers: 1-69 (NG--0010109, NT--024637, and BC070333), 5-51 (NG--0010109 and NT--024637), 4-34 (NG--0010109 and NT--024637), 3-30.3 (CAJ556644), and 3-23 (AJ406678).

Antibody protein sequences are compared with a compiled protein sequence database using one of the sequence similarity search methods well known to those skilled in the art as Gapped BLAST (Altschul et al. (1997) (supra)).

Preferred framework sequences for use in the antibodies of the present disclosure are those framework sequences that are structurally similar to the framework sequences used in the antibodies of the present disclosure.

The HCDR1 sequence, the HCDR2 sequence, and the HCDR3 sequence of a $V_H$ may be grafted onto framework regions having sequences identical to those that are found in the germline immunoglobulin gene from which the framework sequences are derived, or the CDR sequences may be grafted onto framework regions comprising one or more mutations compared to the germline sequences. For example, it has been found that in certain cases, it is beneficial to mutate residues within the framework regions to maintain or enhance the antigen-binding ability of the antibody (see, e.g., U.S. Patent Nos. 5,530,101; 5,585,089; 5,693,762; and 6,180,370).

Another type of variable region modification is to mutate amino acid residues within the CDR1, the CDR2, and/or the CDR3 of the $V_H$ and/or the $V_L$ to improve one or more binding properties (e.g., affinity) of the antibody of interest. Site-directed mutagenesis or PCR-mediated mutagenesis can be performed to introduce one or more mutations, and their effects on antibody binding or other functional properties of interest can be evaluated in in vitro or in vivo assays described herein and provided in examples. Preferably, a conservative modification(s) (as known in the art) is/are introduced. The mutations may be amino acid substitutions, additions, or deletions, but are preferably substitutions. In addition, generally, no more than 1, 2, 3, 4, or 5 residues within CDRs are altered.

Thus, in another embodiment, the present disclosure provides an isolated anti-PD-L1 monoclonal antibody or an antigen-binding portion thereof, comprising a heavy chain variable region comprising: (a) a $V_H$ HCDR1 comprising the sequence of the present disclosure or an amino acid sequence having 1, 2, 3, 4, or 5 amino acid substitutions, deletions, or additions compared to the sequence of the present disclosure; (b) a $V_H$ HCDR2 comprising the sequence of the present disclosure or an amino acid sequence having 1, 2, 3, 4, or 5 amino acid substitutions, deletions, or additions compared to the sequence of the present disclosure; (c) a $V_H$ HCDR3 comprising the sequence of the present disclosure or an amino acid sequence having 1, 2, 3, 4, or 5 amino acid substitutions, deletions, or additions compared to the sequence of the present disclosure; (d) a $V_L$ LCDR1 comprising the sequence of the present disclosure or an amino acid sequence having 1, 2, 3, 4, or 5 amino acid substitutions, deletions, or additions compared to the sequence of the present disclosure; (e) a $V_L$ LCDR2 comprising the sequence of the present disclosure or an amino acid sequence having 1, 2, 3, 4, or 5 amino acid substitutions, deletions, or additions compared to the sequence of the present disclosure; and (f) a $V_L$ LCDR3 comprising the sequence of the present disclosure or an amino acid sequence having 1, 2, 3, 4, or 5 amino acid substitutions, deletions, or additions compared to the sequence of the present disclosure.

Engineered antibodies of the present disclosure include those antibodies in which modifications have been made to framework residues within the $V_H$ and/or the $V_L$, for example, to improve the properties of the antibody. Generally, such framework modifications are made to reduce the immunogenicity of the antibody. For example, one approach is to "back-mutate" one or more framework residues to the corresponding germline sequence. More specifically, an antibody that has undergone somatic mutation may comprise framework residues that differ from the germline sequence from which the antibody is derived. Such residues can be identified by comparing the antibody framework sequences with the germline sequence from which the antibody is derived.

Another type of framework modification involves mutating one or more residues within the framework regions or even within one or more CDRs to remove T cell epitopes, thereby reducing the potential immunogenicity of the antibody. This approach is also referred to as "deimmunization" and is described in more detail in U.S. Patent Publication No. 20030153043.

In addition to or as an alternative to modifying within framework regions or CDRs, the antibody of the present disclosure may be engineered to include a modification(s) within the Fc region, typically to alter one or more functional properties of the antibody, such as serum half-life, complement fixation, Fc receptor binding, and/or antigen-dependent cellular cytotoxicity. In addition, the antibody of the present disclosure may be chemically modified (for example, one or more chemical moieties may be attached to the antibody) or be modified to alter its glycosylation, thereby altering one or more functional properties of the antibody again.

In one embodiment, the hinge region of CH1 is modified such that the number of cysteine residues in the hinge region is altered, e.g., increased or decreased. This approach is further described in U.S. Patent No. 5,677,425. The number of cysteine residues in the hinge region of CH1 is altered, for example, to facilitate assembly of the light and heavy chains or to increase or decrease the stability of the antibody.

In another embodiment, the Fc hinge region of the antibody is mutated to reduce the biological half-life of the antibody. More specifically, one or more amino acid mutations are introduced into the CH2-CH3 domain interface region of the Fc hinge fragment such that the antibody has impaired Staphylococcal protein A (SpA) binding relative to native Fc hinge domain SpA binding. This approach is described in more detail in U.S. Patent No. 6,165,745.

In yet another embodiment, the glycosylation of the antibody is modified. For example, an aglycosylated antibody may be prepared (i.e., the antibody lacks glycosylation). Glycosylation may be altered, for example, to increase the affinity of the antibody for the antigen. Such carbohydrate modifications may be achieved by, for example, altering one or more glycosylation sites within the antibody sequence. For example, one or more amino acid substitutions may be made to eliminate one or more variable region framework glycosylation sites, thereby eliminating glycosylation at the site(s). Such aglycosylation may increase the affinity of the antibody for the antigen. See, e.g., U.S. Patent Nos. 5,714,350 and 6,350,861.

Additionally or optionally, an antibody having an altered type of glycosylation may be prepared, such as a low-fucosylated antibody having reduced amounts of fucosyl residues or an antibody having an increased proportion of a bisecting GlcNac structure. Such an altered glycosylation profile has been shown to increase the ADCC ability of the antibody. Such carbohydrate modifications may be achieved by, for example, expressing the antibody in a host cell with an altered glycosylation mechanism. Cells with an altered glycosylation mechanism have been described in the art and can be used as host cells for expressing the recombinant antibody of the present disclosure, thereby producing an antibody with altered glycosylation. For example, as cell lines Ms704, Ms705, and Ms709 lack the fucosyltransferase gene FUT8 ($\alpha$(1,6)-fucosyltransferase), antibodies expressed in the Ms704, Ms705, and Ms709 cell lines lack fucose on their carbohydrates. The Ms704, Ms705, and Ms709 FUT8-/- cell lines are produced by the targeted disruption of the FUT8 gene in CHO/DG44 cells using two substitution-type vectors (see U.S. Patent Publication No. 20040110704 and Yamane-Ohnuki et al. (2004) Biotechnol Bioeng 87:614-22). As another example, EP 1,176,195 describes a cell line with a functionally disrupted FUT8 gene (encoding a fucosyltransferase); thus, by reducing or eliminating $\alpha$-1,6 bond-related enzymes, antibodies expressed in such a cell line exhibit low fucosylation. EP 1,176,195 also describes cell lines with low or no enzymatic activity for adding fucose to the N-acetylglucosamine that binds to the Fc region of the antibody, such as the rat myeloma cell line YB2/0 (ATCC CRL 1662). PCT Publication WO 03/035835 describes Lec13 cells, a variant CHO cell line with a reduced ability to link fucose to Asn(297)-linked carbohydrates, which also causes antibodies expressed in the host cell to exhibit low fucosylation (see also Shields et al. (2002) J. Biol. Chem. 277:26733-26740). Antibodies with modified glycosylation profiles can also be produced in chicken eggs, as described in PCT Publication No. WO 06/089231. Optionally, antibodies with modified glycosylation profiles can be produced in plant cells such as duckweed. Methods for producing antibodies in a plant system are disclosed in a U.S. patent application corresponding to Alston & Bird LLP Attorney Docket No. 040989/314911, filed on August 11, 2006. PCT Publication No. WO 99/54342 describes cell lines engineered to express glycoprotein-modifying glycosyltransferases (e.g., $\beta$(1,4)-N-acetylglucosa-minyltransferase III (GnTIII)); thus, antibodies expressed in the engineered cell lines exhibit an increased proportion of a bisecting GlcNac structure, which increases the ADCC activity of the antibodies (see also Umana et al. (1999) Nat. Biotech. 17:176-180). Optionally, fucosidase can be used to cleave fucosyl residues from the antibody; for example, the fucosidase $\alpha$-L-fucosidase removes fucosyl residues from the antibody (Tarentino et al. (1975) Biochem. 14:5516-23).

Another modification contemplated by the present disclosure for the antibody herein is PEGylation. The antibody may be PEGylated to, for example, extend the biological (e.g., serum) half-life of the antibody. To PEGylate an antibody, the antibody or a fragment thereof is typically reacted with polyethylene glycol (PEG), such as a reactive ester or aldehyde derivative of PEG, under conditions in which one or more PEG groups are linked to the antibody or the antibody fragment. Preferably, PEGylation is performed via an acylation reaction or an alkylation reaction with a reactive PEG molecule (or an analogous reactive water-soluble polymer). The term "polyethylene glycol" as used herein is intended to encompass any one of the forms of PEG that have been used to derivatize other proteins, such as mono(C1-C10)alkoxy polyethylene glycol, aryloxy polyethylene glycol, or polyethylene glycol-maleimide. In certain embodiments, the antibody to be PEGylated is an aglycosylated antibody. Methods for PEGylating proteins are known in the art and can be applied to the antibody of the present disclosure. See, e.g., EPO 154 316 and EP 0 401 384.

## II. Physical Properties of Antibody

The antibodies of the present disclosure can be characterized by their various physical properties to detect and/or distinguish their different classes.

For example, an antibody may comprise one or more glycosylation sites in the light chain variable region or the heavy chain variable region. Such glycosylation sites may result in an increase in the immunogenicity of the antibody or a change in the pK of the antibody due to altered antigen binding (Marshall et al. (1972) Annu Rev Biochem 41:673-702; Gala and Morrison (2004) J Immunol 172:5489-94; Wallick et al. (1988) J Exp Med 168:1099-109; Spiro (2002) Glycobiology 12:43R-56R; Parekh et al. (1985) Nature 316:452-7; and Mimura et al. (2000) Mol Immunol 37:697-706). Glycosylation is known to occur at motifs comprising N-X-S/T sequences. In some cases, an anti-PD-L1 antibody comprising no variable region glycosylation is preferred. This can be achieved by selecting an antibody in which the variable regions do not comprise the glycosylation motif or by mutating residues within the glycosylation region.

In one preferred embodiment, the antibody does not comprise an asparagine isomerization site. Deamidation of asparagine may occur on the N-G or D-G sequence and lead to the production of isoaspartic acid residues, which introduce kinks into the polypeptide chain and reduce its stability (isoaspartic acid effect).

Each antibody will have a unique isoelectric point (pI), which generally falls within a pH range of 6 to 9.5. The pI values of IgG1 antibodies generally fall within a pH range of 7-9.5, and the pI values of IgG4 antibodies generally fall within a pH range of 6-8. It was speculated that antibodies having pI values outside the normal range may have some unfolding and instability under in vivo conditions. Thus, an anti-4-1BB antibody having a pI value that falls within the normal range is preferred. This can be achieved by selecting an antibody having a pI value that falls within the normal range or by mutating a charged surface residue.

## III. Pharmaceutical Composition

In another aspect, the present disclosure provides a pharmaceutical composition that comprises the bispecific antibody of the present disclosure, formulated together with a pharmaceutically acceptable excipient, and may optionally comprise one or more chemotherapeutic agents.

The pharmaceutical composition may comprise any number of excipients. Excipients that can be used include carriers, surfactants, thickeners or emulsifiers, solid binders, dispersing or suspending aids, solubilizers, colorants, flavoring agents, coating agents, disintegrants, lubricants, sweeteners, preservatives, isotonic agents, and combinations thereof. Selection and use of suitable excipients are taught in Gennaro, Ed., Remington: The Science and Practice of Pharmacy, 20th Edition (Lippincott Williams & Wilkins 2003), the disclosure of which is incorporated herein by reference.

In some embodiments, the pharmaceutical composition is suitable for intravenous, intramuscular, subcutaneous, parenteral, spinal, or epidermal administration (e.g., by injection or infusion). Depending on the route of administration, the active compound may be coated in a material to protect it from the action of acids and other natural conditions that may inactivate it. The phrase "parenteral administration" as used herein means modes of administration other than enteral and topical administration, usually by injection, including but not limited to intravenous, intramuscular, intraarterial, intrathecal, intracapsular, intraorbital, intracardiac, intradermal, intraperitoneal, transtracheal, subcutaneous, subcuticular, intraarticular, subcapsular, subarachnoid, intraspinal, epidural, and intrasternal injection and infusion. Optionally, the antibody of the present disclosure may be administered via a non-parenteral route, such as a topical, epidermal, or mucosal route of administration, for example, intranasally, orally, vaginally, rectally, sublingually, or topically. Preferably, the antibody is formulated as a formulation for intravenous infusion or subcutaneous injection.

The pharmaceutical composition may take the form of a sterile aqueous solution or a dispersion. They may also be formulated as microemulsions, liposomes, or other ordered structures suitable for high drug concentrations.

The amount of the active ingredient that can be combined with a carrier material to produce a single dosage form will vary depending upon the subject being treated and the particular mode of administration, and will generally be an amount of the composition that produces a therapeutic effect. Generally, out of one hundred per cent, combined with a pharmaceutically acceptable carrier, this amount will range from about 0.01% to about 99% of the active ingredient, preferably from about 0.1% to about 70%, and most preferably from about 1% to about 30% of the active ingredient.

The pharmaceutical composition may be a controlled-release formulation, including implants, transdermal patches, and microencapsulated delivery systems. Biodegradable, biocompatible polymers may be used, such as ethylene-vinyl acetate, polyanhydrides, polyglycolic acid, collagen, polyorthoesters, and polylactic acid. See, e.g., Sustained and Controlled Release Drug Delivery Systems, J. R. Robinson Ed., Marcel Dekker, Inc., New York, 1978.

Therapeutic agents may be administered via medical devices, such as (1) needleless subcutaneous injection devices (e.g., U.S. Patent Nos. 5,399,163; 5,383,851; 5,312,335; 5,064,413; 4,941,880; 4,790,824; and 4,596,556); (2) micro-infusion pumps (U.S. Patent No. 4,487,603); (3) transdermal devices (U.S. Patent No. 4,486,194); (4) infusion devices (U.S. Patent Nos. 4,447,233 and 4,447,224); and (5) osmotic devices (U.S. Patent Nos. 4,439,196 and 4,475,196); the disclosures of these documents are incorporated herein by reference.

In some embodiments, the pharmaceutical composition comprises: (i) the bispecific antibody of the present disclosure; (ii) a podophyllotoxin derivative, such as etoposide, etoposide glucuronide, or etoposide phosphate; and (iii) a platinum-based agent, such as carboplatin or cisplatin.

## IV. Combination Therapy and Pharmaceutical Combination

In another aspect, the present disclosure provides a combination therapy, wherein the bispecific molecule of the present disclosure is co-administered with one or more additional therapeutic agents (i.e., a pharmaceutical combination) and/or modalities of treatment, e.g., additional antibodies or chemotherapeutic agents, effective in palliating cancer, an infection, or an autoimmune disease in a subject.

In some embodiments, the present disclosure provides a combination therapy for treating a cancer disease in a subject, and the therapy comprises administering to the subject the antibody of the present disclosure and one or more additional therapies (e.g., standard disease treatment, such as standard cancer treatment), e.g., modalities of treatment and/or additional therapeutic agents.

In some embodiments, modalities of treatment include surgery, radiation, cryosurgery, and/or thermal therapy.

In some embodiments, the additional therapeutic agents are, for example, additional antibodies, such as anti-PD-1 antibodies and/or anti-CTLA-4 antibodies.

In some embodiments, the therapeutic agents are selected from chemotherapeutic agents, additional antibodies, cytotoxic agents, vaccines, anti-infective agents, small molecule drugs, and immunomodulatory agents.

In one aspect, the present disclosure provides a pharmaceutical combination comprising the bispecific molecule of the present disclosure and one or more additional therapeutic agents.

For example, the antibody of the present disclosure may be combined with standard cancer treatment. For example, the antibody may be effectively combined with a chemotherapy regimen. In these cases, the dose of the chemotherapeutic agent administered may be reduced (Mokyr, M. et al., (1998) Cancer Research 58: 5301-5304). In certain embodiments, the methods and antibody described herein are administered in combination with one or more of the following: additional antibody molecules, chemotherapy, additional anti-cancer therapies (e.g., targeted anti-cancer therapies or oncolytic drugs), cytotoxic active agents, immune-based therapies (e.g., cytokines), surgery, and/or radiation.

In certain embodiments, the antibody of the present disclosure is used in combination with a chemotherapeutic agent for standard cancer treatment, and the chemotherapeutic agent for standard cancer treatment includes, but is not limited to, a platinum-based agent and/or a podophyllotoxin derivative; more preferably, the platinum-based agent is carboplatin or cisplatin; most preferably, the podophyllotoxin derivative is etoposide, etoposide glucuronide, or etoposide phosphate. In some embodiments, the pharmaceutical combination comprises the bispecific antibody of the present disclosure; a podophyllotoxin derivative, such as etoposide, etoposide glucuronide, or etoposide phosphate; and a platinum-based agent, such as carboplatin or cisplatin.

In some embodiments, the antibody of the present disclosure is combined with carboplatin and etoposide, or with cisplatin and etoposide.

In some embodiments, the bispecific molecule and the one or more additional therapeutic agents in the pharmaceutical combination of the present disclosure are administered as separate entities to a patient, either simultaneously without a specific time limitation or sequentially at identical or different time intervals, wherein such administration provides prophylactically or therapeutically effective levels of the two active agents in the patient. Illustratively, the bispecific molecule of the present disclosure and the one or more additional therapeutic agents are kits.

In some specific embodiments, the bispecific molecule and the one or more additional therapeutic agents in the pharmaceutical combination of the present disclosure are simultaneously administered in the form of a single entity to the patient. The dose and/or time intervals of the bispecific molecule of the present disclosure and the one or more additional therapeutic agents are preferably selected such that the combined use of the ingredients can result in a therapeutic effect on the disease or disorder greater than that achieved by the use of any one of the ingredients alone. Each ingredient may take a separate formulation form, and their formulation forms may be the same or different. The bispecific antibody of the present disclosure and/or the additional therapeutic agents in the pharmaceutical combination of the present disclosure may be a pharmaceutical dose unit(s), e.g., a single pharmaceutical dose unit(s).

In some other embodiments, the bispecific molecule and the one or more additional therapeutic agents in the pharmaceutical combination of the present disclosure are present in the same pharmaceutical composition.

In one aspect, the present disclosure further relates to a kit comprising the pharmaceutical combination of the present disclosure, wherein preferably, the kit takes the form of a pharmaceutical dose unit. A dose unit may thus be provided according to the dosage regimen or the interval of administration.

In one embodiment, the kit of parts of the present disclosure comprises, in the same package:

- a first container containing the antibody or the pharmaceutical composition of the present disclosure; and
- a second container containing one or more additional therapeutic agents, e.g., chemotherapeutic agents, such as chemotherapeutic agents for standard cancer treatment. In some embodiments, the bispecific antibody of the present disclosure and the additional therapeutic agents, e.g., chemotherapeutic agents, in the pharmaceutical combination of the present disclosure may each take a separate dosage form, e.g., any dosage form known to those skilled in the art, such as tablets, capsules, granules, syrups, powders, lozenges, sachets, cachets, elixirs, suspensions, emulsions, solutions, syrups, aerosols, ointments, creams, and injections, and may take identical or different dosage forms. In some embodiments, the bispecific antibody of the present disclosure is preferably formulated as a formulation for intravenous infusion or subcutaneous injection. In some embodiments, the chemotherapeutic agents of the present disclosure are formulated for intravenous infusion or oral formulation administration. In some embodiments, in the pharmaceutical combination of the present disclosure, the bispecific antibody is administered intravenously, and the chemotherapeutic agents are also administered intravenously.

### V. Use/Treatment Method

The bispecific antibody of the present disclosure has numerous in vitro and in vivo uses, including, for example, treatment of cancer, infections, and autoimmune diseases. The bispecific antibody of the present disclosure may be administered to human subjects, e.g., in vivo, to treat these diseases. In some embodiments, the treatment method of the present disclosure comprises administering to a subject the bispecific antibody of the present disclosure. In some other embodiments, the treatment method of the present disclosure comprises administering to a subject the bispecific antibody of the present disclosure and one or more additional therapies (e.g., standard disease treatment, such as standard cancer treatment), e.g., modalities of treatment and/or additional therapeutic agents.

In one aspect, the present disclosure provides a method for modulating an immune response in a subject.

In some embodiments, the present disclosure provides a method for activating T cells or inducing T cell-mediated anti-tumor activity. In one embodiment, the T cells are CD8$^+$ T cells. In another aspect, the present disclosure provides a method for reducing Treg cells, e.g., CD4$^+$ T cells.

In some embodiments, the activation of T cells comprises stimulating cytokine secretion from T cells, e.g., IL-12 secretion from T cells.

In some embodiments, the present disclosure provides a method for activating the 4-1BB signaling pathway.

In some embodiments, the methods described above comprise administering the bispecific antibody of the present disclosure or a pharmaceutical combination/combination therapy comprising same. Specifically, the methods described above comprise administering the bispecific antibody of the present disclosure, a pharmaceutical composition, formulation, or combination product comprising the bispecific antibody of the present disclosure, or a nucleic acid encoding the bispecific antibody of the present disclosure.

In another aspect, the present disclosure provides a method for treating a tumor in a subject, and the method comprises administering the bispecific antibody binding to 4-1BB and PD-L1 of the present disclosure or a pharmaceutical composition or pharmaceutical combination comprising same.

In some embodiments, the tumor is a malignant tumor, e.g., cancer.

In some embodiments, the cancer is tumor immune evasion.

In some embodiments, the cancer includes solid cancer and non-solid cancer, as well as metastatic lesions. In one embodiment, examples of solid cancers include malignant tumors. The cancer may be early, intermediate, or advanced or metastatic cancer. In some embodiments, the cancer is cancer that requires T cell activation, such as cancer having T cell dysfunction.

In some embodiments, the cancer is cancer having, for example, an increased level of PD-L1 protein expression or an increased level of the nucleic acid encoding PD-L1 as compared to the level in a normal subject or a normal cell.

In some embodiments, the malignant tumor is selected from bladder cancer, breast cancer, endometrioid carcinoma, cervical cancer, an advanced solid tumor, renal cell carcinoma, a neuroendocrine neoplasm, transitional cell carcinoma, urothelial carcinoma, Hodgkin lymphoma, non-Hodgkin lymphoma, cancer in the gastrointestinal tract (e.g., colorectal cancer, rectal cancer, or colon cancer), non-small cell lung cancer, and hepatocellular carcinoma; preferably, the neuroendocrine neoplasm is a neuroendocrine carcinoma or neuroendocrine tumor; relatively preferably, the neuroendocrine neoplasm is an extrapulmonary neuroendocrine neoplasm; more preferably, the neuroendocrine neoplasm is an advanced neuroendocrine neoplasm.

In some embodiments, the neuroendocrine carcinoma is an advanced neuroendocrine carcinoma. In some other embodiments, the neuroendocrine carcinoma is an extrapulmonary neuroendocrine carcinoma, e.g., an advanced extrapulmonary neuroendocrine carcinoma, or small cell lung cancer, e.g., extensive-stage small cell lung cancer. In an exemplary embodiment, the neuroendocrine carcinoma is an unresectable, locally advanced or metastatic neuroendocrine carcinoma.

In some embodiments, the treatment of cancer will benefit from:

(i) inhibition of PD-L1 nucleic acid or protein levels;
(ii) blocking the binding of PD-L1 to a receptor thereof, such as PD-1;
(iii) activation of the 4-1BB signaling pathway;
(iv) activation of T cells, e.g., increasing the proliferation of CD8+ T cells, or reducing Treg cells, e.g., CD4+ T cells; and
(v) any one of the above or a combination of more than one of the above.

In some embodiments, the cancer is cancer having positive expression of a biomarker or altered, e.g., elevated, expression thereof in a sample, e.g., tumor tissue or cells, or blood, as compared to a control. In some embodiments, the control is corresponding tissue or blood or cells or tissue of a healthy subject, or healthy tissue or healthy cells surrounding the tumor tissue.

In some embodiments, the biomarker is selected from PD-L1, d-MMR, MSI-H, TMB, and/or 4-1BB.

In some embodiments, the biomarker may be detected by an immunohistochemical method or an NGS method.

In some embodiments, the cancer is a tumor having one or more of high biomarker levels or expression and/or tumor-infiltrating lymphocytes (TILs)+. In certain embodiments, the cancer is a tumor having high PD-L1 levels or expression and TILs+. In some embodiments, the tumor having TILs+ is positive for CD8 and/or IFN$\gamma$. In some embodiments, the cancer or the subject having the cancer has a high percentage of cells that are positive for one or more of the biomarker, CD8, and/or IFN$\gamma$. In certain embodiments, the cancer or the subject having the cancer has or is identified as having a high percentage of cells that are positive for both the biomarker and CD8.

In some embodiments, the methods described herein further describe identifying the cancer or the subject having the cancer based on the presence of a high percentage of cells that are positive for one or more of the biomarker, CD8, and/or IFN$\gamma$. In certain embodiments, the methods described herein further describe identifying the cancer or the subject having the cancer based on the presence of a high percentage of cells that are positive for both the biomarker and CD8. In some embodiments, the cancer or the subject having the cancer has or is identified as having one or more of the biomarker and CD8.

In some embodiments, the treatment method using the bispecific antibody of the present disclosure may be used for first-line treatment, second-line treatment, or multi-line treatment of a malignant tumor or cancer.

In some specific embodiments, the subject having the malignant tumor or cancer has not received systemic therapy; for example, the subject has not received other cancer therapies such as immunotherapy or standard cancer treatment methods. In some other specific embodiments, the subject having the malignant tumor or cancer is a subject who has experienced progression after receiving at least a prior chemotherapy first-line treatment; for example, the subject includes, but is not limited to, a subject who has experienced progression after receiving prior second-line or higher-line chemotherapy, e.g., a subject who has experienced progression after receiving prior second-line chemotherapy, a subject who has experienced progression after receiving prior third-line chemotherapy, or a subject who has experienced progression after receiving prior fourth-line chemotherapy.

In some exemplary embodiments, the treatment method using the bispecific antibody of the present disclosure may be used for first-line treatment, second-line treatment, or multiple-line treatment of a neuroendocrine carcinoma (e.g., an extrapulmonary neuroendocrine carcinoma or small cell lung cancer), specifically for first-line treatment, second-line treatment, or multiple-line treatment of an advanced neuroendocrine carcinoma, and more specifically for first-line treatment, second-line treatment, or multiple-line treatment of an advanced extrapulmonary neuroendocrine carcinoma or extensive-stage small cell lung cancer.

In a specific embodiment, the subject may be a subject with small cell lung cancer who has not previously received systemic therapy, a subject with an advanced extrapulmonary neuroendocrine carcinoma who has experienced progression after receiving at least a prior chemotherapy first-line treatment, or a subject with an advanced extrapulmonary neuroendocrine carcinoma who has experienced progression after receiving at least a prior chemotherapy first-line treatment, including but not limited to a subject with an advanced extrapulmonary neuroendocrine carcinoma who has experienced progression after receiving prior second-line or higher-line chemotherapy, e.g., a subject with an advanced extrapulmonary neuroendocrine carcinoma who has experienced progression after receiving prior second-line chemotherapy, a subject with an advanced extrapulmonary neuroendocrine carcinoma who has experienced progression after receiving prior third-line chemotherapy, or a subject with an advanced extrapulmonary neuroendocrine carcinoma who has experienced progression after receiving prior fourth-line chemotherapy.

In a more specific embodiment, the chemotherapy treatment that the subject has received may be a platinum-containing chemotherapy treatment, e.g., a platinum-containing chemotherapy first-line treatment, a platinum-containing chemotherapy second-line treatment, or a platinum-containing chemotherapy third-line treatment.

**V.** Dosage Regimen

In the treatment method of the present disclosure, the bispecific antibody of the present disclosure is administered to a human subject at a therapeutically effective dose.

A "therapeutically effective dose" of the bispecific antibody of the present disclosure preferably causes a reduction in the severity of disease symptoms or an increase in the frequency and duration of disease symptom-free periods, or prevents disorders or disability caused by affliction with the disease. For example, a "therapeutically effective dose" for treating a subject bearing a tumor preferably inhibits tumor growth by at least about 20%, more preferably at least about 40%, even more preferably at least about 60%, and yet more preferably at least about 80%, relative to an untreated subject. A therapeutically effective amount of a therapeutic compound can reduce tumor size or otherwise ameliorate symptoms in a subject who is typically a human or may be another mammal.

Dosage regimens are adjusted to provide the optimum desired response (e.g., a therapeutic response). For example, a single bolus injection may be administered, several divided doses may be administered over time, or the dose may be proportionally reduced or increased, as indicated by the exigencies of the therapeutic situation. It is particularly advantageous to formulate parenteral compositions in dose unit form for ease of administration and to maintain dose uniformity. The term dose unit form as used herein refers to physically discrete units suitable for use as unit doses for a subject to be treated; each unit comprises a predetermined amount of an active compound calculated to produce the desired therapeutic effect in association with the required pharmaceutical carrier. Optionally, the antibody may be administered as a sustained-release formulation, in which case less frequent administration is required.

The dosage regimen of the bispecific antibody of the present disclosure described below applies to both treatment methods in which only the bispecific antibody of the present disclosure is administered and the pharmaceutical combination/combination therapy of the present disclosure.

In a dosage regimen of the present disclosure, the bispecific antibody of the present disclosure may be administered to an individual in need thereof at one or more doses.

In some embodiments, about 50 mg-5000 mg of the bispecific antibody, preferably 60 mg-4000 mg of the bispecific antibody, more preferably 80 mg-3750 mg of the bispecific antibody, and most preferably 100 mg-3500 mg of the bispecific antibody, e.g., 200 mg, 400 mg, 600 mg, 800 mg, 1000 mg, 1250 mg, 1500 mg, 1750 mg, 2000 mg, 2250 mg, 2500 mg, 2750 mg, 3000 mg, 3250 mg, or 3500 mg of the bispecific antibody, is administered to the subject in each administration cycle or each time.

In some embodiments, the bispecific antibody is administered to the subject at about 0.2 mg/kg-200 mg/kg, preferably at 0.8 mg/kg-25 mg/kg, more preferably at 3.2 mg/kg-20 mg/kg, yet more preferably at 6 mg/kg-15 mg/kg, and most preferably at 10 mg/kg-15 mg/kg, in each administration cycle or each time. In some embodiments, the bispecific antibody is administered to the subject at 0.8 mg/kg, 1 mg/kg, 1.5 mg/kg, 2 mg/kg, 2.5 mg/kg, 3.2 mg/kg, 4 mg/kg, 5 mg/kg, 6 mg/kg, 8 mg/kg, 10 mg/kg, 12 mg/kg, 14 mg/kg, 15 mg/kg, 16 mg/kg, 17 mg/kg, 18 mg/kg, 19 mg/kg, 20 mg/kg, 21 mg/kg, 22 mg/kg, 23 mg/kg, 24 mg/kg, or 25 mg/kg in each administration cycle or each time.

In some embodiments, the administration of the bispecific antibody may be completed within 30-120 min, e.g., more than 45 min, more than 60 min, more than 75 min, more than 90 min, or more than 105 min.

In some embodiments, in the case of administering multiple doses, the next dose is administered about 2 days-84 days after the previous dose; for example, each administration cycle of the bispecific antibody of the present disclosure is about 2 days to 84 days; for example, the administration cycle is as follows: the bispecific antibody is administered once every 12 weeks, once every 9 weeks, once every 6 weeks, once every 5 weeks, once every 4 weeks, once every 3 weeks, once every 2 weeks, once weekly, twice weekly, or three times weekly. In some embodiments, the bispecific antibody of the present disclosure is administered to the subject for, for example, 1, 2, 3, 4, 5, 6, 7, 8, 9, 10, 11, 12, 13, 14, 15, 20, 30, 40, 50, 60, 70, 80, 90, or 100 administration cycles; preferably, the bispecific antibody is administered for a period of no more than two years.

The additional therapeutic agents, e.g., chemotherapeutic agents, in the pharmaceutical combination of the present disclosure are administered about once daily, once every two days, once every three days, or once every four days. In some embodiments, the chemotherapeutic agents are administered for 1-5 days, e.g., 1 day or 3 days.

In some embodiments, the chemotherapeutic agents in the pharmaceutical combination/combination therapy of the present disclosure are podophyllotoxin derivatives, and are administered at a dose of less than or equal to about 100 mg/m$^2$ or less, e.g., less than or equal to about 90, 80, 75, 70, 65, 60, 55, or 50 mg/m$^2$, or between the ranges, in each administration cycle or each time.

In some embodiments, the chemotherapeutic agents in the pharmaceutical combination/combination therapy of the present disclosure are platinum-based agents, and are administered at a dose of less than or equal to about 75 mg/m$^2$ or less, e.g., less than or equal to about 70, 65, 60, 55, 50, 45, 42.5, 40, 37.5, or 35 mg/m$^2$, or between the range; or AUC $\leq$ about 5 mg/mL/min or less, or less than or equal to about 4, 3, 2, or 1 mg/mL/min, or between the ranges, in each administration cycle or each time, wherein AUC $\leq$ about 5 mg/mL/min means that the administration amount can be calculated according to the following formula:

$$\text{dose (mg)} = \text{set AUC (mg/mL/min)} \times [\text{creatinine clearance rate (mL/min)} + 25],$$

wherein AUC $\leq$ about 5 mg/mL/min. The administration doses expressed in terms of AUC (mg/mL/min) herein are all calculated according to this formula.

The bispecific antibody of the present disclosure or the bispecific antibody in the pharmaceutical combination may be administered before, simultaneously with, or after the administration of the additional therapeutic agents. When the bispecific antibody of the present disclosure is administered "before" the administration of the additional therapeutic agents, the interval between the administration of the bispecific antibody of the present disclosure and the administration of the additional therapeutic agents may be greater than 150 h, about 150 h, about 100 h, about 72 h, about 60 h, about 48 h, about 36 h, about 24 h, about 12 h, about 10 h, about 8 h, about 6 h, about 4 h, about 2 h, about 1 h, about 30 min, about 15 min, about 10 min, or zero. When the bispecific antibody is administered after the administration of the additional therapeutic agents, the interval between the administration of the bispecific antibody of the present disclosure and the administration of the additional therapeutic agents may be zero, about 10 min, about 15 min, about 30 min, about 1 h, about 2 h, about 4 h, about 6 h, about 8 h, about 10 h, about 12 h, about 24 h, about 36 h, about 48 h, about 60 h, about 72 h, or more than 72 h. Being administered "simultaneously" with the administration of the additional therapeutic agents means that the bispecific antibody of the present disclosure is administered to the individual less than 10 min before, less than 10 min after, or simultaneously with the administration of the additional therapeutic agents. Preferably, the bispecific antibody of the present disclosure or the bispecific antibody in the pharmaceutical combination is administered before the administration of the additional therapeutic agents; for example, the interval between the administration of the bispecific antibody of the present disclosure and the administration of the additional therapeutic agents may be zero, about 1 h, about 3 h, about 6 h, about 12 h, about 15 h, or about 24 h.

In some embodiments, the pharmaceutical combination/combination therapy is administered according to cycles; for example, each administration cycle is at least 14-35 days, e.g., 2, 3, 4, or 5 weeks, preferably 3 weeks.

The pharmaceutical combination/combination therapy of the present disclosure may be administered for at least one cycle, e.g., 2-6 or more treatment cycles. The bispecific antibody and the additional therapeutic agents in the pharmaceutical combination/combination therapy may differ in the number of administration cycles; for example, the number of administration cycles of the bispecific antibody is not greater than two years, and the number of administration cycles of the additional therapeutic agents (e.g., chemotherapeutic agents) is 2-6 cycles, preferably 4-6 cycles.

Preferably, the bispecific antibody of the present disclosure is administered once or twice in each cycle, or the bispecific antibody of the present disclosure is administered in each cycle; and/or
the additional therapeutic agents are administered at least on days 1 to 5 of each cycle, e.g., on day 1 or days 1 to 3, followed by a break lasting until the end of the cycle.

The pharmaceutical combination/combination therapy of the present disclosure may be administered every 5 weeks, every 4 weeks, every 3 weeks, every 2 weeks, or weekly.

In some embodiments, the pharmaceutical combination/combination therapy comprises

(i) the bispecific antibody of the present disclosure;
(ii) a podophyllotoxin derivative, e.g., etoposide, etoposide glucuronide, or etoposide phosphate; and
(iii) a platinum-based agent, e.g., carboplatin or cisplatin.

In some embodiments of the pharmaceutical combination/combination therapy,

(i) is administered at a single dose of about 0.2 mg/kg-200 mg/kg, preferably 0.8 mg/kg-25 mg/kg, more preferably 3.2 mg/kg-20 mg/kg, yet more preferably 6 mg/kg-15 mg/kg, and most preferably 10 mg/kg-15 mg/kg, e.g., 0.8 mg/kg, 1 mg/kg, 1.5 mg/kg, 2 mg/kg, 2.5 mg/kg, 3.2 mg/kg, 4 mg/kg, 5 mg/kg, 6 mg/kg, 8 mg/kg, 10 mg/kg, 12 mg/kg, 14 mg/kg, 15 mg/kg, 16 mg/kg, 17 mg/kg, 18 mg/kg, 19 mg/kg, 20 mg/kg, 21 mg/kg, 22 mg/kg, 23 mg/kg, 24 mg/kg, or 25 mg/kg; or about 50 mg-5000 mg, preferably 60 mg-4000 mg, more preferably 80 mg-3750 mg, and most preferably 100 mg-3500 mg, e.g., 200 mg, 400 mg, 600 mg, 800 mg, 1000 mg, 1250 mg, 1500 mg, 1750 mg, 2000 mg, 2250 mg, 2500 mg, 2750 mg, 3000 mg, 3250 mg, or 3500 mg;
(ii) is administered at a single dose of less than or equal to about 100 $mg/m^2$, e.g., less than or equal to about 90, 80, 75, 70, 65, 60, 55, or 50 $mg/m^2$, or between the ranges; and/or
(iii) is administered at a single dose of less than or equal to about 75 $mg/m^2$, e.g., less than or equal to about 70, 65, 60, 55, 50, 45, 42.5, 40, 37.5, or 35 $mg/m^2$, or between the ranges; or AUC ≤ about 5 mg/mL/min or less, or less than or equal to about 4, 3, 2, or 1 mg/mL/min, or between the ranges.

The additional therapeutic agents, e.g., chemotherapeutic agents, in the pharmaceutical combination/combination therapy of the present disclosure are administered about once daily, once every two days, once every three days, or once every four days. In some embodiments, the chemotherapeutic agents are administered for 1-5 days, e.g., 1 day or 3 days.

In some embodiments of the pharmaceutical combination, the pharmaceutical combination is administered with every three weeks as a cycle, wherein in each cycle, (i) is administered once, (ii) is administered once daily for 1-5 days, preferably once daily for 3 days, and (iii) is administered once; preferably, after the administration of (i) (e.g., immediately or after about 15 min, 30 min, 60 min, 1 h, 2 h, or 3 h), the first dose of (ii) is administered, and (iii) is administered, wherein the first dose of (ii) and (iii) may be administered simultaneously or sequentially (e.g., at an interval of about 0 min, 15 min, 30 min, 60 min, 1 h, 2 h, or 3 h). In some embodiments, (i) is administered at a single dose of 5-20 mg/kg, e.g., about 6, 7, 8, 9, 10, 11, 12, 13, 14, or 15 mg/kg; (ii) is administered at a single dose of 100 $mg/m^2$ (body surface area), preferably (ii) is etoposide and is administered at a single dose of 100 $mg/m^2$, and preferably (ii) is administered once daily for 3 days; (iii) is administered at a single dose of 100 $mg/m^2$ or AUC = 5 mg/mL/min, and preferably (iii) is cisplatin and is administered at a single dose of 75 $mg/m^2$, or is carboplatin and is administered at a single dose of AUC = 5 mg/mL/min.

In one preferred embodiment, the pharmaceutical combination is administered for 4-6 cycles.

In one embodiment, after the administration cycles of the pharmaceutical combination are all completed, the bispecific antibody of the present disclosure continues to be administered continuously, for example, once every three weeks, once every four weeks, once every five weeks, or once every six weeks, at a single dose of 0.2-25 mg/kg, e.g., about 0.8 mg/kg, 1 mg/kg, 1.5 mg/kg, 2 mg/kg, 2.5 mg/kg, 3.2 mg/kg, 4 mg/kg, 5 mg/kg, 6 mg/kg, 8 mg/kg, 10 mg/kg, 12 mg/kg, 14 mg/kg, 15 mg/kg, 16 mg/kg, 17 mg/kg, 18 mg/kg, 19 mg/kg, 20 mg/kg, 21 mg/kg, 22 mg/kg, 23 mg/kg, 24 mg/kg, or 25 mg/kg, each time.

In one specific embodiment, after the administration cycles of the pharmaceutical combination are all completed, the bispecific antibody of the present disclosure continues to be administered continuously, for example, once every three weeks, once every four weeks, once every five weeks, or once every six weeks, for example, for 1, 3, 5, 7, 10, 15, 20, 30, 40, 50, 60, 70, 80, 90, or 100 administration cycles. Preferably, the sum of the duration of pharmaceutical combination treatment and the duration of bispecific antibody monotherapy is not greater than two years.

In one preferred embodiment, the bispecific antibody of the present disclosure (e.g., P4B-3) is used in combination with etoposide and cisplatin, with every 3 weeks as an administration cycle, wherein on day 1 of each administration cycle, the bispecific antibody is intravenously infused at 6 mg/kg, 10 mg/kg, or 15 mg/kg; etoposide is intravenously infused at a dose of 100 mg/m$^2$ or less; cisplatin is intravenously infused at a dose of 75 mg/m$^2$ or less; the bispecific antibody is infused first; after the end of the infusion of the bispecific antibody, etoposide is administered; and after the end of the administration of etoposide, cisplatin is administered; on days 2 and 3, etoposide alone is intravenously infused at a daily dose of 100 mg/m$^2$ or less; the administration is performed for a maximum of 6 cycles.

In another preferred embodiment, the bispecific antibody of the present disclosure (e.g., P4B-3) is used in combination with etoposide and carboplatin, with every 3 weeks as an administration cycle, wherein on day 1 of each administration cycle, the bispecific antibody is intravenously infused at 6 mg/kg, 10 mg/kg, or 15 mg/kg; etoposide is intravenously infused at a dose of 100 mg/m$^2$ or less; carboplatin is intravenously infused at a dose of AUC = 5 mg/mL/min or less; the bispecific antibody is infused first; after the end of the infusion of the bispecific antibody, etoposide is administered; and after the end of the administration of etoposide, carboplatin is administered; on days 2 and 3, etoposide alone is intravenously infused at a daily dose of 100 mg/m$^2$ or less; the administration is performed for a maximum of 6 cycles.

In some embodiments, administering the bispecific antibody of the present disclosure as monotherapy or in combination with the additional therapeutic agents increases, preferably synergistically increases, the progression-free survival (PFS) or overall survival (OS) of a patient. In some embodiments, administering the bispecific antibody or the pharmaceutical combination of the present disclosure for at least one cycle increases the PFS of a patient by at least about 1 month, about 2 months, about 3 months, about 4 months, about 5 months, about 6 months, about 7 months, about 8 months, about 9 months, about 10 months, about 11 months, about 1 year, about 2 years, or longer. In some embodiments, administering the bispecific antibody or the pharmaceutical combination of the present disclosure for at least one cycle increases the OS of a patient by at least about 1 month, about 2 months, about 3 months, about 4 months, about 5 months, about 6 months, about 7 months, about 8 months, about 9 months, about 10 months, about 11 months, about 1 year, about 2 years, or longer.

The bispecific antibody or the pharmaceutical combination of the present disclosure increases, preferably synergistically increases, tumor growth inhibition. In some embodiments, the bispecific antibody pharmaceutical combination of the present disclosure inhibits tumor growth by at least about 10%, about 20%, about 30%, about 40%, about 50%, about 60%, about 70%, or about 80%. In some embodiments, administering the pharmaceutical combination of the present disclosure increases tumor regression, tumor shrinkage, and/or tumor disappearance.

In some embodiments, the bispecific antibody pharmaceutical combination of the present disclosure prevents tumor recurrence in an individual and/or increases the duration of survival; for example, the bispecific antibody pharmaceutical combination increases the duration of survival by more than 15 days, more than 1 month, more than 3 months, more than 6 months, more than 12 months, more than 18 months, more than 24 months, more than 36 months, or more than 48 months. In some embodiments, the pharmaceutical combination of the present disclosure may increase progression-free survival or overall survival.

In some embodiments, administering the bispecific antibody or the pharmaceutical combination of the present disclosure to an individual with cancer results in the complete disappearance of a tumor ("complete response"). In some embodiments, administering the bispecific antibody or the pharmaceutical combination of the present disclosure to an individual with cancer results in a reduction of at least 30% or more in tumor cells or tumor size ("partial response"). Tumor reductions can be measured by any method known in the art, such as X-ray, positron emission tomography (PET), computed tomography (CT), magnetic resonance imaging (MRI), cytology, histology, or molecular genetic analysis.

In some embodiments, the bispecific antibody or the pharmaceutical combination of the present disclosure can reduce adverse events caused by the administration of known therapies, e.g., hematological toxic reactions, non-hematological toxic reactions, or other toxic reactions, such as pneumonia, diarrhea, enterocolitis, renal insufficiency, rash, hepatitis, endocrine diseases, peripheral or central neuritis, and abnormalities of liver function.

**Examples**

Example 1:

Bispecific antibody P4B-3 (FIG. 1) was constructed and expressed as described in CN 114555638 A.

Bispecific antibody P4B-3 was constructed as follows: The two scFvs of a 4-1BB antibody were linked to the C-termini of the two heavy chains of an intact PD-L1 antibody, respectively, to construct a bispecific antibody comprising a heavy chain consisting of, from N-terminus to C-terminus:

a VH from the PD-L1 antibody (the VH of the anti-PD-L1 antibody)-a heavy chain constant region-a linker-a VH from the 4-1BB antibody (the VH of the anti-4-1BB antibody)-a linker-a VL from the 4-1BB antibody (the VL of the anti-4-1BB antibody); and

a light chain from the PD-L1 antibody (the light chain of the anti-PD-L1 antibody, i.e., the VL of the light chain constant region of the anti-PD-L1 antibody).

Nucleotides encoding the bispecific antibody and controls were generated by gene synthesis (Genscript) and cloned into pcDNA3.1 expression vectors (Invitrogen). A nucleic acid encoding the light chain variable region was inserted into a pcDNA3.1 expression vector (Invitrogen) containing a nucleic acid encoding the light chain constant region to construct a vector expressing the light chain of the antibody, and a nucleic acid encoding the heavy chain variable region was inserted into a pcDNA3.1 expression vector (Invitrogen) containing a nucleic acid encoding the heavy chain constant region to construct a vector expressing the heavy chain of the antibody. According to the manufacturer's instructions, the obtained vectors were co-transfected at a 1:1 molar ratio into CHO-S cells using an ExpiCHO™ expression system (Thermo Fisher, Cat. No. A29133). According to the manufacturer's instructions, the transfected cells were cultured in an ExpiCHO™ expression culture medium for 12 days, and the culture supernatant was then collected and purified by protein A affinity chromatography (GE Healthcare).

The amino acid sequences and nucleic acid sequences of different regions are shown in Table 1.

Table 1. The SEQ ID NOs of regions/domains of the bispecific antibody

| | P4B-3 (amino acid sequence ID/nucleic acid sequence ID) |
|---|---|
| VH-CDR1 of PDL1 antibody | 11/35 |
| VH-CDR2 of PDL1 antibody | 12/36 |
| VH-CDR3 of PDL1 antibody | 13/37 |
| VL-CDR1 of PDL1 antibody | 14/38 |
| VL-CDR2 of PDL1 antibody | 15/39 |
| VL-CDR3 of PDL1 antibody | 16/40 |
| VH of anti-PD-L1 antibody | 17/41 |
| VL of anti-PD-L1 antibody | 18/42 |
| VH-CDR1 of anti-4-1BB antibody | 1/27 |
| VH-CDR2 of anti-4-1BB antibody | 2/28 |
| VH-CDR3 of anti-4-1BB antibody | 3/29 |
| VL-CDR1 of anti-4-1BB antibody | 4/30 |
| VL-CDR2 of anti-4-1BB antibody | 5/31 |
| VL-CDR3 of anti-4-1BB antibody | 6/32 |
| VH[1] of anti-4-1BB antibody | 9/33 |
| VL[2] of anti-4-1BB antibody | 10/34 |
| Linker between scFv of anti-4-1BB antibody and anti-PD-L1 antibody | 23,n=3 |
| Linker between VH of anti-41BB antibody and VL of anti-4-1BB antibody | 24 |
| Heavy chain | 25/45 |
| Light chain | 26/46 |
| Heavy chain constant region of anti-PD-L1 antibody | 20/43 |
| Light chain constant region of anti-PD-L1 antibody ($\kappa$) | 22/44 |

[1] 4-1BB VH was derived from the VH of monoclonal antibody 41BB-2; forming a disulfide bond with the VL through G44C (EU numbering) improved the stability of the scFv.
[2] 4-1BB VL was derived from the VL of monoclonal antibody 41BB-2; forming a disulfide bond with the VH through T104C (EU numbering) improved the stability of the scFv.

Example 2: Preclinical Toxicology Studies

2.1. 29-day repeated intermittent dosing and 28-day recovery period GLP toxicity study of P4B-3, administered to rats by intravenous infusion

Male and female SD rats (Beijing Vital River Laboratory Animal Technology Co., Ltd.) were each treated with 5 consecutive doses of the study drug P4B-3 (one dose per week, 30, 100, or 300 mg/kg), and the results showed that: during the 29 consecutive days of treatment with P4B-3 (intravenous infusion, once weekly, 30, 100, or 300 mg/kg/dose) and a 28-day recovery period, the drug was well tolerated by the SD rats, and no trial drug-related clinical symptoms, injection site irritations, ophthalmological changes, clinical pathological changes (hematology, blood biochemistry, coagulation, and urinalysis), organ weight changes, macroscopic lesions, or histopathological changes were observed. The no-observed-adverse-effect level (NOAEL) in this trial was 300 mg/kg.

2.2. 29-day repeated dosing and 28-day recovery period GLP toxicology study of P4B-3, administered to cynomolgus monkeys by intravenous infusion

Cynomolgus monkeys (Guangdong Blooming Spring Biotechnology Development Co., Ltd.) were each treated with 5 consecutive doses of the study drug P4B-3 (one dose per week, 20, 60, or 200 mg/kg), and the results showed that no trial drug-related clinical symptoms, body weight changes, food intake changes, ophthalmological changes, body temperature changes, safety pharmacology parameter changes (quantitative and qualitative electrocardiogram, cardiovascular, respiratory, and neurological changes), coagulation changes, urinalysis result changes, or organ weight changes were observed. The highest non-severely toxic dose (HNSTD) in this trial was 200 mg/kg.

Example 3: Clinical Pharmacology Study

A preliminary pharmacokinetic (PK) analysis of P4B-3 was performed. The drug was administered once every 3 weeks (Q3W), with every 3 weeks counted as one administration cycle.

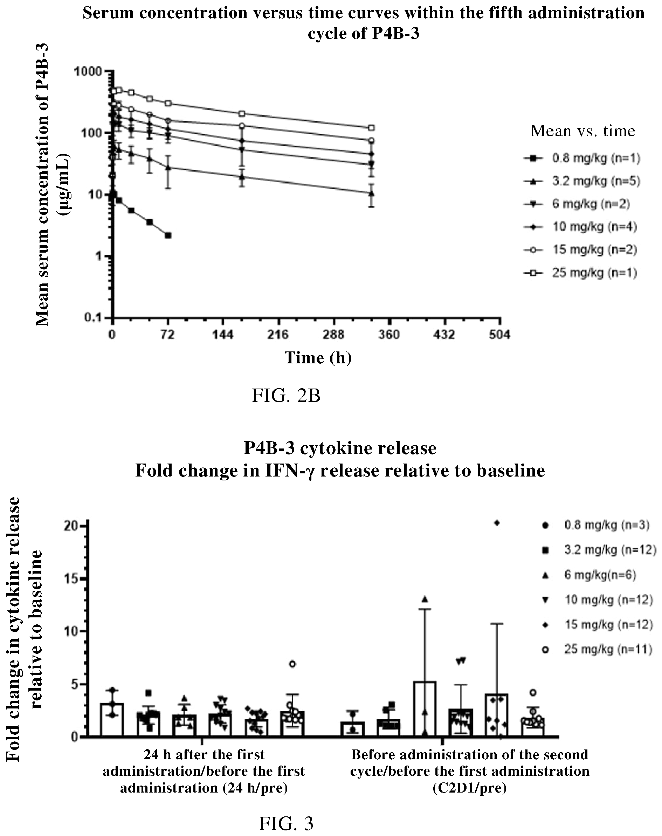

As of September 4, 2023, PK data from 5 cycles had been collected. These data showed that after a single administration and multiple administrations of P4B-3 at 0.2-25 mg/kg, the serum concentration of P4B-3 increased as the dose increased, and the serum concentration-time curves for these doses showed similar change trends. The serum concentrations after multiple administrations were similar to those after the first administration (see FIGs. 2A and B).

After a single administration, within a dose range of 0.2-25 mg/kg, Cmax and AUC increased as the dose increased, and the mean half-lives after a single administration in the dose groups of 0.2-25 mg/kg were about 1.886-6.732 days. After a single administration, the Cmax and AUC0-inf distributions after dose correction in the dose groups of 0.2-25 mg/kg were similar, suggesting an approximately proportional dose-response relationship between the exposure level and the dose.

After multiple administrations, within a dose range of 0.8-25 mg/kg, Cmax and AUC increased as the dose increased. The Rac on C5D1 after multiple administrations was 1.02-1.30, suggesting that some drug accumulation was observed in high-dose groups. In addition, the trough concentrations before administration on C2D1, C3D1, and C5D1 in the dose groups were approximately similar, suggesting that a steady state had been reached.

In the dose-escalation stage of the clinical study of P4B-3, the doses were 0.2 mg/kg, 0.8 mg/kg, 3.2 mg/kg, 6 mg/kg, 10 mg/kg, 15 mg/kg, and 25 mg/kg. At low doses from 0.2 mg/kg to 3.2 mg/kg, the serum exposure levels of P4B-3 exhibited TMDD, a nonlinear pharmacokinetic characteristic. At high doses from 6 mg/kg to 25 mg/kg, the serum exposure levels of P4B-3 exhibited linear dose proportional pharmacokinetic characteristics, with Ctrough values of 9.9, 16.3, 31.8, and 45.5 $\mu$g/mL, respectively; at 6 mg/kg to 15 mg/kg, the values reached and were maintained within a PDL1/P4B-3/4-1BB trimer EC90 range. In NEC patients, from 6 mg/kg to 15 mg/kg, the clinical efficacy ORRs of P4B-3 were 0%, 20%, and 41%, respectively; the ORR tended to increase as the dose increased, and exhibited a certain dose-response correlation; the ORR reached 41% at 15 mg/kg. P4B-3 was clinically well tolerated. The MTD was not reached even at 25 mg/kg, and no upward trends in its liver function and heart function safety and hematotoxicity were observed as the dose increased.

Example 4: PD Marker Test

4.1. Cytokine release in serum

As of August 14, 2023, a total of 58 subjects in phase I had provided valid test data. According to the test results, IFN-$\gamma$ release was most significant at two time points: 24 h after administration on C1D1 and before administration on C2D1. Among 54 subjects with baseline data before administration, 49 subjects (90.7%) showed concentration increases 24 h after administration on C1D1 compared to the concentrations before administration, and 34 subjects (63.0%) showed concentration increases before administration on C2D1 compared to the concentrations before administration. Based on the drug mechanism of P4B-3, it can be presumed that after intravenous injection of P4B-3, the drug played a role in activating T lymphocytes and promoting the release of IFN-γ in most subjects. See **FIG. 3**.

The test results for the other four cytokines (IL-2, IL-6, IL-10, and TNF-α) show that the number of subjects who had provided valid test data was relatively small, and the release level of each cytokine was not significantly different from the concentration level before administration.

4.2. PD-L1 immunohistochemical staining of tumor tissue

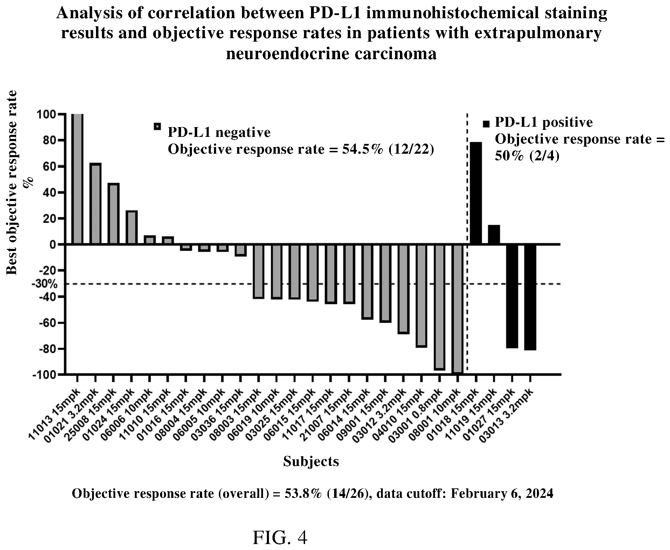

As of January 10, 2024, PD-L1 immunohistochemical staining had been performed on tumor tissue samples from a total of 73 subjects. The results were interpreted using CPS, with a cut-off of ≥1 preliminarily defined as positivity. Among them, 26 subjects had extrapulmonary neuroendocrine carcinomas (with measurable lesions), only 4 subjects were positive for PD-L1, and 2 subjects achieved PR; 22 subjects were negative for PD-L1, and 12 subjects achieved PR. It can be seen that both PD-L1-negative subjects and PD-L1-positive subjects with extrapulmonary neuroendocrine carcinomas benefited from P4B-3 monotherapy, and the ORRs in the two groups were almost the same (54.5% vs. 50%). See **FIG. 4**.

Example 5: Clinical Study of Safety, Tolerability, and Preliminary Effectiveness of P4B-3 in Subjects with Advanced Malignant Tumors

Drug name and strength: P4B-3, 100 mg/vial, prepared according to PCT/CN2020/119388; name: P4B-3 for injection or P4B-3.

Strength: 100 mg/vial, each vial containing 100 mg of the P4B-3 protein. Formulation: Freeze-dried formulation.

Auxiliary materials: Histidine, histidine hydrochloride, trehalose dihydrate, mannitol, and polysorbate 80.

Safety

Safety overview: A total of 175 subjects were enrolled, including 20 subjects in the phase I dose-escalation stage. No DLT events were observed, and the MTD was not reached. Common TRAEs (incidence ≥ 10%) were: increased aspartate transaminase, anemia, increased alanine transaminase, decreased white blood cell count, decreased neutrophil count, and decreased platelet count. Most events were grade 1-2 in severity, and the safety was controllable.

Effectiveness

As of February 19, 2024, efficacy was assessable in 154 subjects: 1 achieved CR, 20 achieved PR, and 49 had SD (ORR: 13.6%, DCR: 45.4%).

**Pharmacokinetic (PK) evaluation**

PK parameters mainly included peak concentration ($C_{max}$), time to peak ($T_{max}$), elimination half-life ($T_{1/2}$), clearance (CL), volume of distribution (Vd), the area under the serum concentration-time curve from time 0 to time t ($AUC_{0-t}$), the area under the serum concentration-time curve from time 0 to infinity ($AUC_{0-inf}$), the area under the serum concentration-time curve at steady state ($AUCss$), peak serum concentration at steady state (Cmax, ss), trough serum concentration at steady state ($C_{min, ss}$), time to peak serum concentration at steady state ($T_{max, ss}$), accumulation factor, etc.

**Immunogenicity evaluation**

The immunogenicity evaluation indicators were the incidence of anti-drug antibodies (ADAs) and the incidence of neutralizing antibodies (if applicable) in subjects. If applicable, the influence of ADAs on the effectiveness, PK characteristics, and safety of the trial drug will be further assessed.

**Biomarker evaluation**

The biomarker evaluation indicator was PD-L1. The expression level of PD-L1 in tumor tissue will be evaluated.

**Statistical analysis**

(1) 1. Sample size estimation

With a one-sided $\alpha$ of 0.025, the sample size calculated based on the exact test for a single-sample binomial distribution was 98 subjects.

(2) Statistical analysis

1. General method

All statistical analyses will be completed using SAS 9.4 or later versions.

2. Subject distribution

The subject distribution includes a summary of the screening, grouping, dropping out, or withdrawal of the subjects in each group, and data set division, and was further presented using a subject distribution flow chart.

3. Baseline statistical analysis

A descriptive analysis of the baseline characteristics of each group, including demographics, medical history, treatment history, medication history, disease characteristics, etc., will be performed using descriptive statistics.

4. Safety analysis

Adverse events will be coded using the Medical Dictionary for Regulatory Activities (MedDRA) 24.1 or later versions.

5. Effectiveness analysis

The number and proportion of subjects achieving best overall responses of complete response (CR), partial response (PR), stable disease (SD), and progressive disease (PD) will be summarized by cancer type using descriptive statistics, and objective response rates and their 95% confidence intervals will be calculated using the Clopper-Pearson method.
Survival curves for progression-free survival (PFS) of patients after treatment with the trial drug will be plotted for different dose groups and cancer types using the Kaplan-Meier method, and their medians, first quartiles, and third quartiles will be calculated. For time-to-event indicators, their medians and quartiles, along with their 95% CIs, will be calculated using the Brookmeyer & Crowley method, and the variance will be calculated using the Greenwood formula.

Primary efficacy endpoint: IRC-assessed ORR

The primary efficacy endpoint for this study stage is IRC-assessed ORR. The primary analysis of IRC-assessed ORR will be performed based on the FAS analysis set, and an ORR estimate and its 95% confidence interval will be provided using the Clopper-Pearson method.
Secondary efficacy endpoints: The secondary efficacy endpoints for this study stage include investigator-assessed ORR, investigator and IRC-assessed DOR, DCR, PFS, and OS.

ORR and DCR

The number of subjects achieving the best objective response will be summarized in different categories of CR, PR, SD, and PD. In addition, the ORR and DCR, along with their 95% CIs, will be calculated using the Clopper-Pearson method.

PFS, OS, and DOR

PFS, OS, and DOR will be analyzed using the Kaplan-Meier method, and their medians and 95% confidence intervals will be provided.
Expected timing for primary efficacy endpoint analysis: When mature ORR data are observed, the primary efficacy analysis will be performed. The analysis is expected no later than 6 months after the first dose is administered to the last subject.

6. Pharmacokinetic analysis

Individual PK data will be listed, and mean PK data within groups will be summarized descriptively. In addition, plasma concentration-time curves for P4B-3 will be plotted. PK parameters will be summarized using descriptive statistics. For multiple-administration PK studies, PK parameters, drug accumulation, and the time to reach a steady state will be discussed.

7. Immunogenicity analysis

For all subjects who received at least one dose of the trial drug, the incidence of anti-P4B-3 antibodies and neutralizing antibodies (if applicable) will be summarized. If applicable, the influence of ADAs on the PK, effectiveness, and safety of P4B-3 will be assessed.

10. Biomarker analysis

The expression level of PD-L1 in tumor tissue samples will be assessed.

Example 6: Clinical Study of Safety, Tolerability, Pharmacokinetic Characteristics, and Effectiveness of P4B-3 plus Etoposide and Platinum-Based Agent (Cisplatin or Carboplatin) in Subjects with Advanced Neuroendocrine Carcinomas

An open, multicenter, phase Ib/II clinical study for evaluating antibody P4B-3 plus etoposide and a platinum-based agent (cisplatin or carboplatin) as a first-line treatment in subjects with advanced neuroendocrine carcinomas.

## Objectives

The primary objective of this study is to evaluate the safety, tolerability, and effectiveness of antibody P4B-3 plus etoposide and a platinum-based agent (cisplatin or carboplatin) as a first-line treatment in subjects with advanced neuroendocrine carcinomas.
Other objectives include evaluating the pharmacokinetic (PK) characteristics of P4B-3 plus etoposide and a platinum-based agent as a first-line treatment in subjects with advanced neuroendocrine carcinomas, evaluating the immunogenicity of P4B-3 plus etoposide and a platinum-based agent as a first-line treatment in subjects with advanced neuroendocrine carcinomas, evaluating the correlation of biomarkers in tumor tissue with efficacy, etc.

## Study endpoints

Phase Ib clinical study

The primary endpoints include: the incidence and severity of dose-limiting toxicities (DLTs), adverse events (AEs), and serious adverse events (SAEs), as well as abnormal laboratory findings.
The secondary endpoints include: objective response rate (ORR), disease control rate (DCR), duration of response (DOR), progression-free survival (PFS), overall survival (OS), etc. (based on the Response Evaluation Criteria in Solid Tumors version 1.1 [RECIST 1.1] and the immune Response Evaluation Criteria in Solid Tumors [iRECIST]); PK, with indicators including Ctrough, etc.; immunogenicity evaluations, with the indicators being the incidence of anti-drug antibodies (ADAs) and the incidence of neutralizing antibodies (if applicable) in subjects; and the correlation of the expression levels of programmed death-ligand 1 (PD-L1), tumor mutational burden (TMB), deficient mismatch repair (dMMR), microsatellite instability-high (MSI-H), and 4-1BB and the level of CD8+ T cells in tumor tissue with efficacy.

Phase II clinical study

The primary endpoint includes: ORR assessed by the investigator per RECIST 1.1.
The secondary endpoints include: the incidence and severity of adverse events (AEs) and serious adverse events (SAEs), as well as abnormal laboratory findings; DCR, DOR, PFS, OS, etc. (based on RECIST 1.1 and iRECIST), and ORR (based on iRECIST); immunogenicity evaluations, with the indicators being the incidence of anti-drug antibodies (ADAs) and the incidence of neutralizing antibodies (if applicable) in subjects; and the correlation of the expression levels of PD-L1, TMB, dMMR, MSI-H, and 4-1BB and the level of CD8+ T cells in tumor tissue with efficacy.

**Study design and duration**

This trial includes a combination therapy dose-escalation stage (phase Ib) and an expansion stage (phase II).

It is planned that subjects with advanced neuroendocrine carcinomas who have not received systemic therapy will be enrolled in phase Ib (safety lead-in phase), and the safety and tolerability of 6 mg/kg, 10 mg/kg, or 15 mg/kg P4B-3 plus etoposide and a platinum-based agent (cisplatin or carboplatin) will be evaluated using a "Rolling six design" dose-escalation method, with 3-6 subjects enrolled in each dose group. In this study stage, subjects with extrapulmonary NECs will receive P4B-3 plus EP/EC. Etoposide will be administered at 100 mg/m$^2$ on consecutive days 1-3 of each cycle, Q3W; cisplatin will be administered at 75 mg/m$^2$ on day 1 of each cycle, Q3W; and carboplatin will be administered at AUC = 5 mg/mL/min on day 1 of each cycle, Q3W. On D1 of each cycle of combination therapy, P4B-3 will be infused first; after the end of the infusion of P4B-3, etoposide will be administered; and after the end of the administration of etoposide, cisplatin or carboplatin will be administered. On D2 and D3 of each cycle of combination therapy, subjects will receive etoposide alone. Chemotherapy will be administered for 6 cycles. After the end of chemotherapy, subjects may continue to receive P4B-3 treatment (Q3W). Subjects with SCLC will receive P4B-3 plus EC. Etoposide will be administered at 100 mg/m$^2$ on consecutive days 1-3 of each cycle, Q3W; and carboplatin will be administered at AUC = 5 mg/mL/min on day 1 of each cycle, Q3W. On D1 of each cycle of combination therapy, P4B-3 will be infused first; after the end of the infusion of P4B-3, carboplatin will be administered; and after the end of the administration of carboplatin, etoposide will be administered. On D2 and D3 of each cycle of combination therapy, subjects will receive etoposide alone. Chemotherapy will be administered for 4 cycles. After the end of chemotherapy, subjects may continue to receive P4B-3 treatment (Q3W) until the occurrence of intolerable toxicity, disease progression or death, voluntary withdrawal, loss to follow-up, completion of 2 years of continuous treatment, or study termination (whichever occurs first). If the starting dosage level of P4B-3 cannot be tolerated by a subject, the SMC will assess the safety and tolerability of a reduced combination therapy dosage level of 3.2 mg/kg.

It is planned that about 50 subjects with advanced extrapulmonary neuroendocrine carcinomas who have not received systemic therapy and about 50 subjects with small cell lung cancer will be enrolled in phase II (combination therapy expansion stage). The subjects with extrapulmonary NECs will receive treatment with P4B-3 plus EP/EC in the same manner as in phase Ib. The subjects with SCLC will receive treatment with P4B-3 plus EC in the same manner as in phase Ib.

**Key inclusion criteria**

(1) Phase Ib: Histologically and/or cytologically confirmed unresectable, locally advanced or metastatic neuroendocrine carcinomas (NECs), including extrapulmonary and intrapulmonary NECs.

Phase II: Histologically and/or cytologically confirmed unresectable, locally advanced or metastatic extrapulmonary NECs; histologically and/or cytologically confirmed extensive-stage small cell lung cancer (ES-SCLC) (according to the Veterans Administration Lung Study Group (VALG) staging system).

(2) No prior systemic therapy for advanced NECs. Subjects who have previously received neoadjuvant or adjuvant therapy are eligible for enrollment if recurrence or metastasis occurred more than 6 months after the last dose (except for subjects who have previously received treatment with anti-PD(L)-1 antibodies). No prior systemic therapy for ES-SCLC. Patients who have received prior chemoradiotherapy for limited-stage SCLC must have been treated with intent to cure the disease, and there has been a treatment-free interval of at least 6 months from the last course of chemotherapy, radiotherapy, or chemoradiotherapy to the diagnosis of extensive-stage SCLC.

(3) Eastern Cooperative Oncology Group (ECOG) performance status score of 0-1.

(4) Expected survival time of at least 12 weeks.

(5) The subject is required to have at least 1 measurable target lesion, as evaluated per the RECIST 1.1 criteria.

**Dose adjustments**

Dose adjustments for P4B-3

P4B-3 dose increases or decreases are not permitted in this trial.

If the use of one drug in the combination therapy regimen needs to be discontinued, treatment with the other drug(s) in the combination therapy regimen may continue. When a chemotherapeutic drug dose is delayed, if the investigator deems that P4B-3 administration can continue, P4B-3 will be administered per the established cycle. The maximum of 6 administration cycles for the EP/EC regimen refers to a maximum of 6 administrations of a combination of P4B-3 and EP/EC. The reasons for dose adjustments, treatment delays, and supportive measures should be documented in the medical records and CRFs.

## Duration of drug treatment

Subjects will receive treatment with the trial drug long-term until the occurrence of intolerable toxicity, disease progression, death, subject's voluntary withdrawal, completion of 2 years of continuous treatment, or study termination (including completion or early termination of the trial) (whichever occurs first).

## Safety evaluation

During the trial, subjects will undergo scheduled safety assessments, including laboratory tests (complete blood count, urinalysis, blood biochemistry, coagulation function, thyroid function, etc.), vital signs, physical examinations, 12-lead electrocardiograms (12-ECG), etc., the clinical manifestations and characteristics, severity, onset time, end time, duration, treatment measures, and outcome of any adverse event will be recorded, and the correlation of it with P4B-3 will be assessed. Adverse events will be graded using the U.S. National Cancer Institute's Common Terminology Criteria for Adverse Events (NCI CTCAE) V 5.0. The investigator will assess the correlation of adverse events with P4B-3 per the attribution assessment criteria specified in the protocol.
This trial will also evaluate drug exposure levels, including duration of drug exposure, actual administration intensity, relative administration intensity, actual total administered amount, etc.

## Evaluation of effectiveness

Anti-tumor efficacy will be evaluated per the revised Response Evaluation Criteria in Solid Tumors (RECIST version 1.1) and the immune Response Evaluation Criteria in Solid Tumors (iRECIST). The efficacy evaluation indicators are objective response rate (ORR), duration of response (DOR), disease control rate (DCR), progression-free survival (PFS), 6-month overall survival rate (6M-OS), overall survival (OS), etc.
Objective response rate (ORR): including partial response (PR) and complete response (CR), defined as the proportion of subjects achieving complete response and partial response. For subjects with solid tumors, anti-tumor efficacy will be evaluated per RECIST 1.1. For subjects with a first evaluation result of PR or CR, the efficacy should be confirmed at least 4 weeks later.
Duration of response (DOR) is defined as the time from the first confirmed objective response to disease progression or death from any cause (whichever occurs first). Disease control rate (DCR) is defined as the proportion of subjects achieving responses (PR + CR) and stable disease (SD) after treatment.
Progression-free survival (PFS) is defined as the time from the day of the subject's first dose of medication to the first assessment of the tumor as confirmed disease progression or death from any cause.
Overall survival (OS) is defined as the time from the enrollment of the subject to death from any cause.

## Pharmacokinetic (PK) evaluation

PK parameters mainly include Ctrough, etc. PK blood samples will be sent for analysis.

## Immunogenicity evaluation

The immunogenicity evaluation indicators were the incidence of anti-drug antibodies (ADAs) and the incidence of neutralizing antibodies (if applicable) in subjects. If applicable, the influence of ADAs on the effectiveness, PK characteristics, and safety of the trial drug will be further assessed. Immunogenicity blood samples will be sent for analysis.

## Biomarker evaluation

The biomarker evaluation indicators are the expression of PD-L1, d-MMR, MSI-H, TMB, and 4-1BB and the level of CD8+ T cells in tumor tissue. The expression of PD-L1, dMMR, and B-1BB and the level of CD8+ T cells in tumor tissue will be immunohistochemically assessed, and the levels of MSI-H and TMB in tumor tissue will be determined by NGS. Tumor tissue samples and paired whole blood required for TMB/MSI-H detection will be used for biomarker detection.

## Statistical analysis

(1) 1. Sample size estimation

No formal statistical test will be performed in Phase Ib of this study. The sample size is not determined by statistical calculation. In phase Ib, a "rolling six" dose-escalation design with a total of 3 dose groups will be employed. Thus, it is planned that 9-12 subjects will be enrolled.

The primary endpoint for the phase II study stage is ORR. It is planned that about 50 subjects will be enrolled in phase II to preliminarily observe the efficacy of P4B-3 plus EP/EC.

Thus, a total of about 62 subjects will be enrolled in phase Ib and phase II.

(2) Statistical analysis

1. General method

All statistical analyses will be completed using SAS 9.4 or later versions.

2. Subject distribution

The subject distribution includes a summary of the screening, grouping, dropping out, or withdrawal of the subjects in each group, and data set division, and was further presented using a subject distribution flow chart.

3. Baseline statistical analysis

A descriptive analysis of the baseline characteristics of each group, including demographics, medical history, treatment history, medication history, disease characteristics, etc., will be performed using descriptive statistics.

4. Drug exposure data analysis

To reflect the extent of study drug exposure, the following data will be summarized: the number of treatment cycles, the number of treatment days, the total amount of the study drug received, and the number of subjects requiring dose reduction, treatment interruption, and treatment discontinuation due to adverse events.

5. Compliance and concomitant medication analysis

A descriptive statistical analysis of planned drug usage and actual drug usage will be performed. The formula is as follows:

$$\text{compliance} = \text{actual drug usage}/\text{planned drug usage} \times 100\%.$$

The frequency and percentage of concomitant medications administered during the study treatment period will be summarized. Detailed lists of concomitant medications for subjects will be provided.

6. Safety analysis

Adverse events will be coded using the Medical Dictionary for Regulatory Activities (MedDRA) 26.0 or later versions.

7. Effectiveness analysis

Effectiveness analysis for solid tumors will primarily follow RECIST 1.1, with iRECIST used for exploratory analysis.

The number of subjects achieving best objective responses will be summarized in different categories of complete response (CR), partial response (PR), stable disease (SD), progressive disease (PD), and not evaluable (NE). In addition, the ORR and DCR, along with their 95% CIs, will be calculated using the Clopper-pearson method.

Survival curves (KM curves) for PFS and OS will be plotted using the Kaplan-Meier method, their medians, first quartiles, and third quartiles will be calculated, and the two-sided 95% confidence intervals of these statistics will be calculated using the Greenwood formula. Other survival endpoints (DOR, etc.) will be analyzed and summarized using similar statistical methods.

8. Pharmacokinetic analysis

Individual PK data will be listed, and mean PK data within groups will be summarized descriptively. In addition, serum concentration-time curves for P4B-3 will be plotted. PK parameters and dose-PK proportional relationships will be summarized using descriptive statistics. For multiple-administration PK studies, PK parameters, drug accumulation, and the time to reach a steady state will be discussed.

Population PK will be analyzed by establishing a relevant model (if applicable).

9. Immunogenicity analysis

For all subjects who received at least one dose of the trial drug, the incidence of anti-P4B-3 antibodies and neutralizing antibodies (if applicable) will be summarized. If applicable, the influence of ADAs on the PK, effectiveness, and safety of P4B-3 will be assessed.

10. Biomarker analysis

The correlation of the expression of PD-L1, dMMR, MSI-H, TMB, and 4-1BB and the levels of CD8+ T cells in tumor tissue samples and the determined TMB/MSI-H levels in paired whole blood with efficacy will be assessed. The expression of PD-L1, dMMR, and B-1BB and the level of C8+ T cells in tumor tissue will be immunohistochemically assessed, and the levels of MSI-H and TMB in tumor tissue will be determined by NGS.

Example 7: Phase IIb Clinical Study of Effectiveness and Safety of P4B-3 in Patients with Advanced Extrapulmonary Neuroendocrine Carcinomas Who Have Experienced Progression After Receiving Prior Second-Line or Higher-Line Chemotherapy

**Objectives**

The primary objective of this study is to evaluate the efficacy of P4B-3 based on ORR assessed per RECIST 1.1.

Other objectives include assessing the efficacy of P4B-3 based on other efficacy indicators assessed by the investigator (based on RECIST 1.1 and iRECIST) or the IRC (based on RECIST 1.1), evaluating the safety of P4B-3 in subjects with advanced extrapulmonary neuroendocrine carcinomas, evaluating the pharmacokinetic (PK) characteristics of P4B-3 in subjects with advanced extrapulmonary neuroendocrine carcinomas, evaluating the immunogenicity of P4B-3 in the treatment of subjects with advanced extrapulmonary neuroendocrine carcinomas, and evaluating the correlation of the biomarker (PD-L1) in tumor tissue with efficacy.

**Study endpoints**

The primary endpoint includes: ORR assessed by the IRC.

The secondary endpoints include: 1) DCR, PFS, and DOR assessed by the IRC, ORR, DCR, DOR, and PFS assessed by the investigator, 6-month OS rate, OS, etc.; 2) adverse events (laboratory tests, physical examinations, vital signs, electrocardiograms, etc.); 3) PK parameters, including but not limited to Cmax, Tmax, T1/2, CL, Vd, AUC, etc.; 4) immunogenicity: the immunogenicity evaluation indicators are the incidence of ADAs and the incidence of neutralizing antibodies (if applicable) in subjects; and 5) the expression of PD-L1 in tumor tissue.

**Study design and duration**

The assessment of the effectiveness and safety of P4B-3 in patients with advanced extrapulmonary neuroendocrine carcinomas who have experienced progression after receiving prior second-line or higher-line chemotherapy will continue.

Analysis will be performed based on the previously obtained safety, efficacy, PK, and PD data. The dosage level of P4B-3 used in this stage of the study is 15 mg/kg Q3W. All subjects must provide tumor tissue for pathological type review. Only subjects whose pathological type is diagnosed as an extrapulmonary neuroendocrine carcinoma by the central laboratory are eligible for enrollment in this study. Tumor tissue samples should be collected from all subjects whenever possible to evaluate expression levels of PD-L1. All subjects will undergo a sparse PK study, and blood samples should be collected from all subjects to evaluate the immunogenicity of P4B-3.

**Dose group design**

In the phase IIb study, subjects will receive a dosage level of 15 mg/kg Q3W.

The body weight of subjects will be measured before each administration, and the drug amount will be calculated based on the body weight. If a subject's body weight change is within $\pm 10\%$ (including 10%) relative to the baseline, the dose will still be calculated based on the baseline body weight. If a body weight change is >10%, the dose should be calculated based on the new body weight, and the new body weight will be used as the baseline for subsequent body weight measurements.

**Key inclusion criteria**

(1) Patients with histologically or cytologically confirmed locally advanced or metastatic extrapulmonary neuroendocrine carcinomas who have experienced progression after receiving prior first-line platinum-containing chemotherapy and second-line chemotherapy.

Note: It means that disease progression occurred during or after the chemotherapy treatment. For subjects who have previously received neoadjuvant chemotherapy, concurrent chemoradiotherapy, or adjuvant chemotherapy, if recurrence/metastasis occurred within 6 months of completing the previous treatment, the original treatment regimen is defined as the first-line treatment regimen for these subjects.

(2) Eastern Cooperative Oncology Group (ECOG) performance status score of 0-1 (see appendix 1);

(3) Expected survival time of at least 12 weeks.

(4) Subjects who have at least one measurable tumor lesion.

**Dose adjustments**

Dose adjustments for P4B-3

Trial drug dose increases or decreases are not permitted in this trial. If a subject experiences a grade $\geq 3$ trial drug-related adverse event (AE), a recurrent grade 2 trial drug-related AE, a grade $\geq 3$ infusion-related adverse reaction, or a life-threatening serious adverse event, the investigator will assess whether to suspend or permanently discontinue the use of the trial drug. For a subject who has not received the trial drug for 3 consecutive administration cycles, the investigator will assess whether to withdraw the subject from the study.

**Duration of drug treatment**

Subjects will receive treatment with the trial drug long-term until the occurrence of intolerable toxicity, disease progression, death, subject's voluntary withdrawal, more than 24 months of continuous treatment, or study termination (including completion or early termination of the trial) (whichever occurs first).

**Safety evaluation**

During the trial, subjects will undergo scheduled safety assessments, including laboratory tests (complete blood count, urinalysis, blood biochemistry, coagulation function, thyroid function, etc.), vital signs, physical examinations, 12-lead electrocardiograms (12-ECG), etc., the clinical manifestations and characteristics, severity, onset time, end time, duration, treatment measures, and outcome of any adverse event will be recorded, and the correlation of it with P4B-3 will be assessed. Adverse events will be graded using the U.S. National Cancer Institute's Common Terminology Criteria for Adverse Events (NCI CTCAE) V 5.0. The investigator will assess the correlation of adverse events with P4B-3 per the attribution assessment criteria specified in the protocol.

**Evaluation of effectiveness**

Anti-tumor efficacy will be evaluated per the revised Response Evaluation Criteria in Solid Tumors (RECIST version 1.1) and the immune Response Evaluation Criteria in Solid Tumors (iRECIST). The efficacy evaluation indicators are objective response rate (ORR), duration of response (DOR), disease control rate (DCR), progression-free survival (PFS), 6-month overall survival rate (6M-OS), overall survival (OS), etc.

Objective response rate (ORR): including partial response (PR) and complete response (CR), defined as the proportion of subjects achieving complete response and partial response. For subjects with solid tumors, anti-tumor efficacy will be evaluated per RECIST 1.1. For subjects with a first evaluation result of PR or CR, the efficacy should be confirmed at least 4 weeks later.

Duration of response (DOR) is defined as the time from the first confirmed objective response to disease progression or death from any cause (whichever occurs first). Disease control rate (DCR) is defined as the proportion of subjects achieving responses (PR + CR) and stable disease (SD) after treatment.

Progression-free survival (PFS) is defined as the time from the day of the subject's first dose of medication to the first assessment of the tumor as confirmed disease progression or death from any cause.

Overall survival (OS) is defined as the time from the enrollment of the subject to death from any cause.

As of February 19, 2024, a total of 47 subjects with EP-NECs (all with measurable lesions) who had experienced progression after undergoing at least a prior platinum-containing chemotherapy first-line treatment had received P4B-3 treatment. Among them, 45 subjects underwent at least one efficacy assessment, 15 subjects achieved PR, and 9 subjects achieved SD, resulting in an ORR of 33.3% and a DCR of 53.3%. Among 25 subjects with EP-NECs who had experienced progression after undergoing prior second-line or higher-line systemic therapy, 24 subjects were evaluable for efficacy, 7 subjects achieved PR, and 5 subjects achieved SD, resulting in an ORR of 29.2% and a DCR of 50.0%. As of September 19, 2024, for the 45 patients who underwent at least one efficacy assessment, the median follow-up time was 12.3 months, and the median OS was 15 months (95% CI: 10.4, NE).

The study results show that P4B-3 monotherapy achieved good efficacy in patients with EP-NECs who had experienced progression after undergoing at least a prior platinum-containing chemotherapy first-line treatment. In particular, P4B-3 monotherapy is expected to meet the unmet clinical needs of the third-line or higher-line population, for whom there are no recommended treatment regimens in the guidelines.

**Claims**

1. Use of a bispecific antibody in the preparation of a drug for treating a malignant tumor, wherein the bispecific antibody comprises a first antigen-binding region specifically binding to 4-1BB and a second antigen-binding region specifically binding to PD-L1.

2. The use according to claim 1, wherein the first antigen-binding region comprises a first heavy chain variable region and/or a first light chain variable region, wherein:

   the first heavy chain variable region:

   (i) comprises an HCDR1, an HCDR2, and an HCDR3 derived from a heavy chain variable region consisting of the sequence set forth in SEQ ID NO: 7 or SEQ ID NO: 9;
   (ii) comprises an HCDR1, an HCDR2, and an HCDR3 comprising or consisting of the amino acid sequences of SEQ ID NO: 1, SEQ ID NO: 2, and SEQ ID NO: 3, respectively, or comprising or consisting of amino acid sequences having one, two, or three modifications (preferably amino acid substitutions, and preferably conservative substitutions) compared to the amino acid sequences of SEQ ID NO: 1, SEQ ID NO: 2, and SEQ ID NO: 3, respectively; or
   (iii) comprises or consists of the amino acid sequence of SEQ ID NO: 7 or SEQ ID NO: 9, or comprises or consists of an amino acid sequence having at least 80%, 81%, 82%, 83%, 84%, 85%, 86%, 87%, 88%, 89%, 90%, 91%, 92%, 93%, 94%, 95%, 96%, 97%, 98%, or 99% identity to the amino acid sequence of SEQ ID NO: 7 or SEQ ID NO: 9, or comprises or consists of an amino acid sequence having one or more (preferably no more than 10, and more preferably no more than 5) amino acid modifications (preferably amino acid substitutions, and more preferably conservative substitutions) compared to the amino acid sequence of SEQ ID NO: 7 or SEQ ID NO: 9, wherein preferably, the amino acid modifications do not occur in a CDR; more preferably, the amino acid modifications occur in an FR, for example, in an FR1, an FR2, an FR3, or an FR4;

   the first light chain variable region:

   (i) comprises an LCDR1, an LCDR2, and an LCDR3 derived from a light chain variable region consisting of the sequence set forth in SEQ ID NO: 8 or SEQ ID NO: 10;
   (ii) comprises an LCDR1, an LCDR2, and an LCDR3 comprising or consisting of the sequences set forth in SEQ ID NO: 4, SEQ ID NO: 5, and SEQ ID NO: 6, respectively, or comprising or consisting of amino acid sequences having one, two, or three modifications (preferably amino acid substitutions, and preferably conservative substitutions) compared to the amino acid sequences of SEQ ID NO: 4, SEQ ID NO: 5, and SEQ ID NO: 6, respectively; or

(iii) comprises or consists of the amino acid sequence of SEQ ID NO: 8 or SEQ ID NO: 10, or comprises or consists of an amino acid sequence having at least 80%, 81%, 82%, 83%, 84%, 85%, 86%, 87%, 88%, 89%, 90%, 91%, 92%, 93%, 94%, 95%, 96%, 97%, 98%, or 99% identity to the amino acid sequence of SEQ ID NO: 8 or SEQ ID NO: 10, or comprises or consists of an amino acid sequence having one or more (preferably no more than 10, and more preferably no more than 5) amino acid modifications (preferably amino acid substitutions, and more preferably conservative substitutions) compared to the amino acid sequence of SEQ ID NO: 8 or SEQ ID NO: 10, wherein preferably, the amino acid modifications do not occur in a CDR; more preferably, the amino acid modifications occur in an FR, for example, in an FR1, an FR2, an FR3, or an FR4;

in SEQ ID NO: 8, X = S or G.

3. The use according to claim 1 or 2, wherein the first antigen-binding region further comprises a first heavy chain constant region and/or a first light chain constant region; the first heavy chain constant region comprises or consists of the amino acid sequence set forth in SEQ ID NO: 19 or 20, and the first light chain constant region comprises or consists of the amino acid sequence set forth in SEQ ID NO: 21 or 22.

4. The use according to any one of claims 1-3, wherein the first antigen-binding region activates 4-1BB signaling and is a monoclonal antibody.

5. The use according to any one of claims 1-4, wherein the second antigen-binding region specifically binding to PD-L1 comprises a second heavy chain variable region and a second light chain variable region, wherein the second heavy chain variable region comprises an HCDR1, an HCDR2, and an HCDR3 consisting of the sequences set forth in SEQ ID NO: 11, SEQ ID NO: 12, and SEQ ID NO: 13, respectively, and the second light chain variable region comprises an LCDR1, an LCDR2, and an LCDR3 consisting of the sequences set forth in SEQ ID NO: 14, SEQ ID NO: 15, and SEQ ID NO: 16, respectively.

6. The use according to claim 5, wherein the second antigen-binding region further comprises a second heavy chain constant region and/or a second light chain constant region; the second heavy chain constant region comprises or consists of the amino acid sequence set forth in SEQ ID NO: 19 or 20, and the second light chain constant region comprises or consists of the amino acid sequence set forth in SEQ ID NO: 21 or 22.

7. The use according to any one of claims 1-6, wherein the bispecific antibody comprises: (1) a chain 1: the heavy chain of the second antigen-binding region that is linked to an antigen-binding portion in the first antigen-binding region at the C-terminus by a linker or by no linker; and
(2) a chain 2: the light chain of the second antigen-binding region.

8. The use according to claim 7, wherein the linker comprises an amino acid sequence of (Gly4Ser)n or (GlySer4)n, wherein n is a positive integer from 1 to 7.

9. The use according to claim 7 or 8, wherein the bispecific antibody consists of two chains 1 and two chains 2.

10. The use according to any one of claims 7-9, wherein the antigen-binding portion is selected from: (i) a Fab fragment; (ii) a F(ab')2 fragment; (iii) an Fd fragment; (iv) an Fv fragment; (v) a dAb fragment; (vi) a nanobody; and (vii) a single-chain Fv (scFv); the scFv comprises the first heavy chain variable region and the first light chain variable region.

11. The use according to any one of claims 7-9, wherein the first antigen-binding region and/or the second antigen-binding region is a mouse antibody, a human antibody, a chimeric antibody, or a humanized antibody.

12. The use according to any one of claims 7-9, wherein the first antigen-binding region and/or the second antigen-binding region is of the IgG1, IgG2, or IgG4 isotype.

13. The use according to claim 7, wherein the chain 1 of the bispecific antibody comprises or consists of an amino acid sequence having at least 90%, 91%, 92%, 93%, 94%, 95%, 96%, 97%, 98%, or 99% identity to the amino acid sequence set forth in SEQ ID NO: 25, and the chain 2 of the bispecific antibody comprises or consists of an amino acid sequence having at least 90%, 91%, 92%, 93%, 94%, 95%, 96%, 97%, 98%, or 99% identity to the amino acid sequence set forth in SEQ ID NO: 26.

14. The use according to any one of claims 1-13, wherein the malignant tumor is bladder cancer, breast cancer, endometrioid carcinoma, cervical cancer, an advanced solid tumor, renal cell carcinoma, a neuroendocrine neoplasm, gastric cancer, transitional cell carcinoma, urothelial carcinoma, Hodgkin lymphoma, non-Hodgkin lymphoma, colorectal cancer, non-small cell lung cancer, or hepatocellular carcinoma; preferably, the neuroendocrine neoplasm is a neuroendocrine carcinoma or neuroendocrine tumor; relatively preferably, the neuroendocrine neoplasm is an extrapulmonary neuroendocrine neoplasm; more preferably, the neuroendocrine neoplasm is an advanced neuroendocrine neoplasm.

15. The use according to claim 14, wherein the neuroendocrine carcinoma is an advanced neuroendocrine carcinoma; preferably, the neuroendocrine carcinoma is an extrapulmonary neuroendocrine carcinoma or small cell lung cancer; more preferably, the neuroendocrine carcinoma is an unresectable, locally advanced or metastatic neuroendocrine carcinoma.

16. The use according to claim 14 or 15, wherein the drug is administered to a subject having the malignant tumor; the subject is a subject who has not received systemic therapy or a subject who has experienced progression after receiving at least a prior chemotherapy first-line treatment; for example, the subject is a subject who has experienced progression after receiving at least a prior platinum-containing chemotherapy first-line treatment; further, the subject includes a subject who has experienced progression after receiving prior second-line or higher-line chemotherapy.

17. The use according to any one of claims 1-16, comprising administering to the subject the bispecific antibody at 0.2 mg/kg-200 mg/kg, preferably at 0.8 mg/kg-25 mg/kg, more preferably at 3.2 mg/kg-20 mg/kg, yet more preferably at 6 mg/kg-15 mg/kg, and most preferably at 10 mg/kg-15 mg/kg, in each administration cycle or each time.

18. The use according to any one of claims 1-16, comprising administering to the subject 50 mg-5000 mg of the bispecific antibody, preferably 60 mg-4000 mg of the bispecific antibody, more preferably 80 mg-3750 mg of the bispecific antibody, and most preferably 100 mg-3500 mg of the bispecific antibody, e.g., 200 mg, 400 mg, 600 mg, 800 mg, 1000 mg, 1250 mg, 1500 mg, 1750 mg, 2000 mg, 2250 mg, 2500 mg, 2750 mg, 3000 mg, 3250 mg, or 3500 mg of the bispecific antibody, in each administration cycle or each time.

19. The use according to claim 17 or 18, wherein the administration cycle of the bispecific antibody in the use is as follows: the bispecific antibody is administered once every 12 weeks, once every 9 weeks, once every 6 weeks, once every 5 weeks, once every 4 weeks, once every 3 weeks, once every 2 weeks, once weekly, twice weekly, or three times weekly.

20. The use according to any one of claims 1-19, wherein the bispecific antibody is formulated as a formulation for intravenous infusion or subcutaneous injection.

21. The use according to any one of claims 1-20, wherein the bispecific antibody is further administered in combination with one or more chemotherapeutic agents; preferably, the chemotherapeutic agents are platinum-based agents and/or podophyllotoxin derivatives; more preferably, the platinum-based agents are carboplatin or cisplatin; most preferably, the podophyllotoxin derivatives are etoposide, etoposide glucuronide, or etoposide phosphate.

22. The use according to claim 21, wherein an administration cycle of the chemotherapeutic agents is as follows: the chemotherapeutic agents are administered once every 5 weeks, once every 4 weeks, once every 3 weeks, once every 2 weeks, once weekly, twice weekly, or three times weekly.

23. The use according to claim 21 or 22, wherein the chemotherapeutic agents are formulated for intravenous infusion or oral formulation administration.

24. The use according to any one of claims 21-23, comprising, after administering the bispecific antibody to the subject in each administration cycle or each time after administering the bispecific antibody to the subject, administering the chemotherapeutic agents.

25. The use according to claim 24, comprising, after administering the bispecific antibody in each administration cycle or each time after administering the bispecific antibody, administering the podophyllotoxin derivatives once daily for 1-5 days (e.g., 3 days), starting from the day the bispecific antibody is administered, wherein preferably, the podophyllotoxin derivatives are administered at 100 mg/m$^2$ or less each time.

26. The use according to claim 24, comprising, after administering the bispecific antibody in each administration cycle or each time after administering the bispecific antibody, administering the platinum-based agents for 1-5 days (e.g., 3 days), starting from the day the bispecific antibody is administered, wherein preferably, the platinum-based agents are administered at 75 mg/m$^2$ or less, or at AUC = 5 mg/mL/min or less, daily.

27. A drug for treating a malignant tumor, comprising the bispecific antibody defined in claims 1-13.

28. A pharmaceutical combination, comprising the bispecific antibody defined in any one of claims 1-13 and one or more chemotherapeutic agents, wherein preferably, the chemotherapeutic agents are platinum-based agents and/or podophyllotoxin derivatives; more preferably, the platinum-based agents are carboplatin or cisplatin; most preferably, the podophyllotoxin derivatives are etoposide, etoposide glucuronide, or etoposide phosphate; preferably, the pharmaceutical combination is used for treating a malignant tumor.

29. The drug according to claim 27 or the pharmaceutical combination according to claim 28, wherein the malignant tumor is bladder cancer, breast cancer, endometrioid carcinoma, cervical cancer, an advanced solid tumor, renal cell carcinoma, a neuroendocrine neoplasm, gastric cancer, transitional cell carcinoma, urothelial carcinoma, Hodgkin lymphoma, non-Hodgkin lymphoma, colorectal cancer, colorectal cancer, non-small cell lung cancer, or hepatocellular carcinoma; preferably, the neuroendocrine neoplasm is a neuroendocrine carcinoma or neuroendocrine tumor; relatively preferably, the neuroendocrine neoplasm is an extrapulmonary neuroendocrine neoplasm; more preferably, the neuroendocrine neoplasm is an advanced neuroendocrine neoplasm.

30. The drug or pharmaceutical combination according to claim 29, wherein the neuroendocrine carcinoma is an advanced neuroendocrine carcinoma; preferably, the neuroendocrine carcinoma is an extrapulmonary neuroendocrine carcinoma or small cell lung cancer; more preferably, the neuroendocrine carcinoma is an unresectable, locally advanced or metastatic neuroendocrine carcinoma.

31. The drug or pharmaceutical combination according to claim 29 or 30, wherein the drug is administered to a subject having the malignant tumor; the subject is a subject who has not received systemic therapy or a subject who has experienced progression after receiving at least a prior chemotherapy first-line treatment; for example, the subject is a subject who has experienced progression after receiving at least a prior platinum-containing chemotherapy first-line treatment; further, the subject includes a subject who has experienced progression after receiving prior second-line or higher-line chemotherapy.

FIG. 1

**Serum concentration versus time curves within the first administration cycle of P4B-3**

FIG. 2A

Serum concentration versus time curves within the fifth administration
cycle of P4B-3

FIG. 2B

P4B-3 cytokine release
Fold change in IFN-γ release relative to baseline

FIG. 3

**Analysis of correlation between PD-L1 immunohistochemical staining results and objective response rates in patients with extrapulmonary neuroendocrine carcinoma**

Objective response rate (overall) = 53.8% (14/26), data cutoff: February 6, 2024

FIG. 4

## INTERNATIONAL SEARCH REPORT

| International application No. |
| --- |
| **PCT/CN2024/121415** |

| A. | CLASSIFICATION OF SUBJECT MATTER |
| --- | --- |

C07K16/46(2006.01)i; C07K16/28(2006.01)i; C12N15/13(2006.01)i; C07K19/00(2006.01)i; A61K39/395(2006.01)i; A61P35/00(2006.01)i

According to International Patent Classification (IPC) or to both national classification and IPC

| B. | FIELDS SEARCHED |
| --- | --- |

Minimum documentation searched (classification system followed by classification symbols)

IPC: C07K C12N A61K A61P

Documentation searched other than minimum documentation to the extent that such documents are included in the fields searched

Electronic data base consulted during the international search (name of data base and, where practicable, search terms used)

CNTXT, ENTXT, ENTXTC, CNKI, 万方数据库, WANFANG DATABASE, NCBI, EBI, STN: PD-L1, 4-1BB, CD137, TNFRSF9, 双特异性抗体, 双抗, p4B-3, 神经内分泌, NEC, 铂类, 卡铂, 顺铂, 神经内分泌, 依托泊苷, 鬼臼毒素, 癌症, 肿瘤, cancer, tumor, neuroendocrine, 南京维立志博生物科技股份有限公司, SEQ ID NOs: 1-48

| C. | DOCUMENTS CONSIDERED TO BE RELEVANT |
| --- | --- |

| Category* | Citation of document, with indication, where appropriate, of the relevant passages | Relevant to claim No. |
| --- | --- | --- |
| X | CN 114555638 A (NANJING LEADS BIOLABS CO., LTD.) 27 May 2022 (2022-05-27) claims 1-26, and description, paragraphs 11-29, 120-122, 127-136, 140-141, 184-246, 311-315, and 331-333 | 1-14, 16, 17-31 |
| Y | CN 114555638 A (NANJING LEADS BIOLABS CO., LTD.) 27 May 2022 (2022-05-27) claims 1-26, and description, paragraphs 11-29, 120-122, 127-136, 140-141, 184-246, 311-315, and 331-333 | 14-26 |
| X | CN 114729053 A (HARBOUR BIOMED (SHANGHAI) CO., LTD.) 08 July 2022 (2022-07-08) description, paragraphs 35-76 | 1, 4, 14-31 |
| Y | CN 114729053 A (HARBOUR BIOMED (SHANGHAI) CO., LTD.) 08 July 2022 (2022-07-08) description, paragraphs 35-76 | 14-26 |
| Y | CN 115052629 A (SHANGHAI JUNSHI BIOSCIENCES CO., LTD.) 13 September 2022 (2022-09-13) description, paragraphs 5-41 | 14-26 |

☑ Further documents are listed in the continuation of Box C. ☑ See patent family annex.

| * | Special categories of cited documents: |
| --- | --- |
| "A" | document defining the general state of the art which is not considered to be of particular relevance |
| "D" | document cited by the applicant in the international application |
| "E" | earlier application or patent but published on or after the international filing date |
| "L" | document which may throw doubts on priority claim(s) or which is cited to establish the publication date of another citation or other special reason (as specified) |
| "O" | document referring to an oral disclosure, use, exhibition or other means |
| "P" | document published prior to the international filing date but later than the priority date claimed |

"T" later document published after the international filing date or priority date and not in conflict with the application but cited to understand the principle or theory underlying the invention

"X" document of particular relevance; the claimed invention cannot be considered novel or cannot be considered to involve an inventive step when the document is taken alone

"Y" document of particular relevance; the claimed invention cannot be considered to involve an inventive step when the document is combined with one or more other such documents, such combination being obvious to a person skilled in the art

"&" document member of the same patent family

| Date of the actual completion of the international search | Date of mailing of the international search report |
| --- | --- |
| **10 December 2024** | **25 December 2024** |

| Name and mailing address of the ISA/CN | Authorized officer |
| --- | --- |
| **China National Intellectual Property Administration (ISA/CN)** **China No. 6, Xitucheng Road, Jimenqiao, Haidian District, Beijing 100088** | |
| | Telephone No. |

Form PCT/ISA/210 (second sheet) (July 2022)

## INTERNATIONAL SEARCH REPORT

| International application No. |
|---|
| **PCT/CN2024/121415** |

### C. DOCUMENTS CONSIDERED TO BE RELEVANT

| Category* | Citation of document, with indication, where appropriate, of the relevant passages | Relevant to claim No. |
|---|---|---|
| X | CN 113166250 A (ABL BIO INC.) 23 July 2021 (2021-07-23)<br>description, paragraphs 149-157, and figure 7 | 1, 4, 7-12, 14, 16-31 |
| Y | CN 113166250 A (ABL BIO INC.) 23 July 2021 (2021-07-23)<br>description, paragraphs 149-157, and figure 7 | 14-26 |
| X | WO 2021013142 A1 (JIANGSU HENGRUI MEDICINE CO., LTD.) 28 January 2021<br>(2021-01-28)<br>claims 1-37 | 1, 4, 7-12, 14, 16-31 |
| Y | WO 2021013142 A1 (JIANGSU HENGRUI MEDICINE CO., LTD.) 28 January 2021<br>(2021-01-28)<br>claims 1-37 | 14-26 |
| X | CN 113286825 A (ABL BIO INC. et al.) 20 August 2021 (2021-08-20)<br>description, paragraphs 4-58 | 1, 4, 7-12, 17-31 |
| Y | CN 113286825 A (ABL BIO INC. et al.) 20 August 2021 (2021-08-20)<br>description, paragraphs 4-58 | 14-26 |
| X | WO 2023000675 A1 (ANHUI ANKE BIOTECHNOLOGY (GROUP) CO., LTD. et al.) 26<br>January 2023 (2023-01-26)<br>description, paragraphs 8-53, and figure 1 | 1, 4, 7-12, 14, 16-31 |
| Y | WO 2023000675 A1 (ANHUI ANKE BIOTECHNOLOGY (GROUP) CO., LTD. et al.) 26<br>January 2023 (2023-01-26)<br>description, paragraphs 8-53, and figure 1 | 14-26 |
| X | JEONG, S. et al. "Novel Anti-4-1BB×PD-L1 Bispecific Antibody Augments Anti-tumor<br>Immunity Through Tumor-Directed T-cell Activation and Checkpoint Blockade"<br>*Journal for ImmunoTherapy of Cancer*, Vol. 9, 31 December 2021 (2021-12-31),<br>e002428, pages 1-12 | 1, 4, 7-12, 14, 16-31 |
| Y | JEONG, S. et al. "Novel Anti-4-1BB×PD-L1 Bispecific Antibody Augments Anti-tumor<br>Immunity Through Tumor-Directed T-cell Activation and Checkpoint Blockade"<br>*Journal for ImmunoTherapy of Cancer*, Vol. 9, 31 December 2021 (2021-12-31),<br>e002428, pages 1-12 | 14-26 |
| A | 张思汗 等 (ZHANG, Sihan et al.). "肿瘤免疫治疗药物的开发现状及展望 (Current status<br>and prospects of tumor immunotherapy drugs development)"<br>临床与病理杂志 (*Journal of Clinical and Pathological Research*),<br>Vol. 41, No. 10, 31 December 2021 (2021-12-31),<br>pages 2447-2460 | 1-31 |
| A | KR 20220139658 A (EUTILEX CO., LTD.) 17 October 2022 (2022-10-17)<br>claims 1-6 | 1-31 |

Form PCT/ISA/210 (second sheet) (July 2022)

| **INTERNATIONAL SEARCH REPORT** | International application No. |
|---|---|
| | **PCT/CN2024/121415** |

| **Box No. I**      **Nucleotide and/or amino acid sequence(s) (Continuation of item 1.c of the first sheet)** |
|---|

1. With regard to any nucleotide and/or amino acid sequence disclosed in the international application, the international search was carried out on the basis of a sequence listing:

     a.   ☑   forming part of the international application as filed.

     b.   ☐   furnished subsequent to the international filing date for the purposes of international search (Rule 13*ter*.1(a)),

           ☐   accompanied by a statement to the effect that the sequence listing does not go beyond the disclosure in the international application as filed.

2.   ☐   With regard to any nucleotide and/or amino acid sequence disclosed in the international application, this report has been established to the extent that a meaningful search could be carried out without a WIPO Standard ST.26 compliant sequence listing.

3. Additional comments:

Form PCT/ISA/210 (continuation of first sheet) (July 2022)

**INTERNATIONAL SEARCH REPORT**
Information on patent family members

International application No.

**PCT/CN2024/121415**

| Patent document cited in search report | | | Publication date (day/month/year) | Patent family member(s) | | | Publication date (day/month/year) |
|---|---|---|---|---|---|---|---|
| CN | 114555638 | A | 27 May 2022 | US | 2021107982 | A1 | 15 April 2021 |
| | | | | US | 11466086 | B2 | 11 October 2022 |
| | | | | EP | 4041772 | A1 | 17 August 2022 |
| | | | | EP | 4041772 | A4 | 24 April 2024 |
| | | | | US | 2023192862 | A1 | 22 June 2023 |
| | | | | JP | 2022553922 | A | 27 December 2022 |
| | | | | WO | 2021068841 | A1 | 15 April 2021 |
| CN | 114729053 | A | 08 July 2022 | CA | 3183462 | A1 | 06 January 2022 |
| | | | | EP | 4155319 | A1 | 29 March 2023 |
| | | | | EP | 4155319 | A4 | 12 June 2024 |
| | | | | WO | 2022002063 | A1 | 06 January 2022 |
| | | | | JP | 2023531042 | A | 20 July 2023 |
| | | | | KR | 20230013125 | A | 26 January 2023 |
| | | | | TW | 202202528 | A | 16 January 2022 |
| | | | | TWI | 792371 | B | 11 February 2023 |
| | | | | AU | 2021301927 | A1 | 09 February 2023 |
| | | | | US | 2023242658 | A1 | 03 August 2023 |
| CN | 115052629 | A | 13 September 2022 | EP | 4104855 | A1 | 21 December 2022 |
| | | | | EP | 4104855 | A4 | 19 June 2024 |
| | | | | US | 2023212292 | A1 | 06 July 2023 |
| | | | | WO | 2021160152 | A1 | 19 August 2021 |
| CN | 113166250 | A | 23 July 2021 | KR | 20210099052 | A | 11 August 2021 |
| | | | | BR | 112021010402 | A2 | 24 August 2021 |
| | | | | EP | 3887403 | A1 | 06 October 2021 |
| | | | | EP | 3887403 | A4 | 31 August 2022 |
| | | | | CA | 3121218 | A1 | 04 June 2020 |
| | | | | US | 2022242961 | A1 | 04 August 2022 |
| | | | | PH | 12021551171 | A1 | 29 November 2021 |
| | | | | AU | 2019390274 | A1 | 22 July 2021 |
| | | | | PE | 20211778 | A1 | 08 September 2021 |
| | | | | US | 2022056136 | A1 | 24 February 2022 |
| | | | | CA | 3121562 | A1 | 04 June 2020 |
| | | | | WO | 2020111913 | A1 | 04 June 2020 |
| | | | | AU | 2019386549 | A1 | 24 June 2021 |
| | | | | AU | 2019386549 | B2 | 25 July 2024 |
| | | | | EP | 3891187 | A1 | 13 October 2021 |
| | | | | EP | 3891187 | A4 | 05 October 2022 |
| | | | | JP | 2022513694 | A | 09 February 2022 |
| | | | | JP | 7328658 | B2 | 17 August 2023 |
| | | | | JP | 2022510253 | A | 26 January 2022 |
| | | | | JP | 7378474 | B2 | 13 November 2023 |
| | | | | MX | 2021006379 | A | 13 October 2021 |
| | | | | KR | 20210087094 | A | 09 July 2021 |
| | | | | EA | 202191457 | A1 | 06 September 2021 |
| | | | | ZA | 202102870 | B | 31 May 2023 |
| | | | | CL | 2021001368 | A1 | 04 March 2022 |
| | | | | SG | 11202105152 | YA | 29 June 2021 |
| | | | | CO | 2021006911 | A2 | 09 August 2021 |
| | | | | IL | 283530 | A | 29 July 2021 |
| | | | | WO | 2020107715 | A1 | 04 June 2020 |

Form PCT/ISA/210 (patent family annex) (July 2022)

## INTERNATIONAL SEARCH REPORT
### Information on patent family members

| International application No. |
|---|
| **PCT/CN2024/121415** |

| Patent document cited in search report | | | Publication date (day/month/year) | Patent family member(s) | | | Publication date (day/month/year) |
|---|---|---|---|---|---|---|---|
| | | | | BR | 112021010394 | A2 | 24 August 2021 |
| WO | 2021013142 | A1 | 28 January 2021 | TW | 202118787 | A | 16 May 2021 |
| CN | 113286825 | A | 20 August 2021 | KR | 20210099052 | A | 11 August 2021 |
| | | | | BR | 112021010402 | A2 | 24 August 2021 |
| | | | | EP | 3887403 | A1 | 06 October 2021 |
| | | | | EP | 3887403 | A4 | 31 August 2022 |
| | | | | CA | 3121218 | A1 | 04 June 2020 |
| | | | | US | 2022242961 | A1 | 04 August 2022 |
| | | | | PH | 12021551171 | A1 | 29 November 2021 |
| | | | | AU | 2019390274 | A1 | 22 July 2021 |
| | | | | PE | 20211778 | A1 | 08 September 2021 |
| | | | | US | 2022056136 | A1 | 24 February 2022 |
| | | | | CA | 3121562 | A1 | 04 June 2020 |
| | | | | WO | 2020111913 | A1 | 04 June 2020 |
| | | | | AU | 2019386549 | A1 | 24 June 2021 |
| | | | | AU | 2019386549 | B2 | 25 July 2024 |
| | | | | EP | 3891187 | A1 | 13 October 2021 |
| | | | | EP | 3891187 | A4 | 05 October 2022 |
| | | | | JP | 2022513694 | A | 09 February 2022 |
| | | | | JP | 7328658 | B2 | 17 August 2023 |
| | | | | JP | 2022510253 | A | 26 January 2022 |
| | | | | JP | 7378474 | B2 | 13 November 2023 |
| | | | | MX | 2021006379 | A | 13 October 2021 |
| | | | | KR | 20210087094 | A | 09 July 2021 |
| | | | | EA | 202191457 | A1 | 06 September 2021 |
| | | | | ZA | 202102870 | B | 31 May 2023 |
| | | | | CL | 2021001368 | A1 | 04 March 2022 |
| | | | | SG | 11202105152 | YA | 29 June 2021 |
| | | | | CO | 2021006911 | A2 | 09 August 2021 |
| | | | | IL | 283530 | A | 29 July 2021 |
| | | | | WO | 2020107715 | A1 | 04 June 2020 |
| | | | | BR | 112021010394 | A2 | 24 August 2021 |
| WO | 2023000675 | A1 | 26 January 2023 | None | | | |
| KR | 20220139658 | A | 17 October 2022 | None | | | |

Form PCT/ISA/210 (patent family annex) (July 2022)

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

**Patent documents cited in the description**

- CN 2020119388 W
- CN 109021107 A
- US 5225539 A
- US 5530101 A
- US 5585089 A
- US 5693762 A
- US 6180370 B
- US 20030153043 A
- US 5677425 A
- US 6165745 A
- US 5714350 A
- US 6350861 B
- US 20040110704 A
- EP 1176195 A
- WO 03035835 A
- WO 06089231 A
- WO 9954342 A
- EP 154316 A
- EP 0401384 A
- US 5399163 A
- US 5383851 A
- US 5312335 A
- US 5064413 A
- US 4941880 A
- US 4790824 A
- US 4596556 A
- US 4487603 A
- US 4486194 A
- US 4447233 A
- US 4447224 A
- US 4439196 A
- US 4475196 A
- CN 114555638 A

**Non-patent literature cited in the description**

- **WARD et al.** *Nature*, 1989, vol. 341, 544-546
- **BIRD et al.** *Science*, 1988, vol. 242, 423-426
- **HUSTON et al.** *Proc. Natl. Acad. Sci. USA*, 1988, vol. 85, 5879-5883
- **LIEBERMAN**. *Pharmaceutical Dosage Forms*, 1992, vol. 1-3
- **LLOYD**. *The Art, Science and Technology of Pharmaceutical Compounding*, 1999
- **PICKAR**. *Dosage Calculations*, 1999
- Remington: The Science and Practice of Pharmacy. Lippincott, Williams & Wilkins, 2003
- **ARAN F. LABRIJN et al.** Bispecific antibodies: a mechanistic review of the pipeline. *Nature Reviews Drug Discovery*, 2019, vol. 18, 585-608
- **BRUMMELL et al.** *Biochem*, 1993, 1180-8
- **DE WILDT et al.** *Prot. Eng.*, 1997, vol. 10, 835-41
- **KOMISSAROV et al.** *J. Biol. Chem.*, 1997, vol. 272, 26864-26870
- **HALL et al.** *J. Immunol.*, 1992, vol. 149, 1605-12
- **KELLEY** ; **O'CONNELL**. *Biochem.*, 1993, vol. 32, 6862-35
- **ADIB-CONQUY et al.** *Int. Immunol.*, 1998, vol. 10, 341-6
- **BEERS et al.** *Clin. Can. Res.*, 2000, vol. 6, 2835-43
- **RIECHMANN et al.** *Nature*, 1998, vol. 332, 323-327
- **JONES et al.** *Nature*, 1986, vol. 321, 522-525
- **QUEEN et al.** *Proc. Natl. Acad. See. U.S.A.*, 1989, vol. 86, 10029-10033
- **TOMLINSON et al.** *J. Mol. Biol.*, 1992, vol. 227, 776-798
- **COX et al.** *Eur. J. Immunol.*, 1994, vol. 24, 827-836
- **YAMANE-OHNUKI et al.** *Biotechnol Bioeng*, 2004, vol. 87, 614-22
- **SHIELDS et al.** *J. Biol. Chem.*, 2002, vol. 277, 26733-26740
- **UMANA et al.** *Nat. Biotech.*, 1999, vol. 17, 176-180
- **TARENTINO et al.** *Biochem.*, 1975, vol. 14, 5516-23
- **MARSHALL et al.** *Annu Rev Biochem*, 1972, vol. 41, 673-702
- **GALA** ; **MORRISON**. *J Immunol*, 2004, vol. 172, 5489-94
- **WALLICK et al.** *J Exp Med*, 1988, vol. 168, 1099-109
- **SPIRO**. *Glycobiology*, 2002, vol. 12, 43R-56R
- **PAREKH et al.** *Nature*, 1985, vol. 316, 452-7
- **MIMURA et al.** *Mol Immunol*, 2000, vol. 37, 697-706

- Remington: The Science and Practice of Pharmacy. Lippincott Williams & Wilkins, 2003
- Sustained and Controlled Release Drug Delivery Systems. Marcel Dekker, Inc., 1978
- **MOKYR, M. et al.** *Cancer Research*, 1998, vol. 58, 5301-5304